(19) Europäisches Patentamt / European Patent Office / Office européen des brevets

(11) EP 2 975 125 B1

(12) EUROPEAN PATENT SPECIFICATION

(45) Date of publication and mention of the grant of the patent: **29.04.2020 Bulletin 2020/18**

(21) Application number: **14764391.0**

(22) Date of filing: **13.03.2014**

(51) Int Cl.: ***C12N 15/86*** *(2006.01)*

(86) International application number: **PCT/JP2014/056651**

(87) International publication number: **WO 2014/142235 (18.09.2014 Gazette 2014/38)**

(54) **MICROVESICLE, AND MANUFACTURING METHOD FOR SAME**

MIKROVESIKEL UND HERSTELLUNGSVERFAHREN DAFÜR

MICROVÉSICULE ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2013 US 201361779556 P**
**23.10.2013 US 201361894563 P**

(43) Date of publication of application: **20.01.2016 Bulletin 2016/03**

(73) Proprietors:
- **Li, Zhong**
  **Tokyo 104-0054 (JP)**
- **Katsura, Misako**
  **Tokyo 104-0054 (JP)**

(72) Inventor: **FENG, Luo**
**San Gabriel, California 91776 (US)**

(74) Representative: **Altmann Stößel Dick Patentanwälte PartG mbB**
**Dudenstrasse 46**
**68167 Mannheim (DE)**

(56) References cited:
**WO-A2-2011/106376 WO-A2-2012/135844**

- **LYDIA ALVAREZ-ERVITI ET AL: "Delivery of siRNA to the mouse brain by systemic injection of targeted exosomes", NATURE BIOTECHNOLOGY, vol. 29, no. 4, 20 March 2011 (2011-03-20) , pages 341-345, XP055227686, US ISSN: 1087-0156, DOI: 10.1038/nbt.1807**
- **KOPPERS-LALIC, D. ET AL.: 'Virus-modified exosomes for targeted RNA delivery; A new approach in nanomedicine' ADVANCED DRUG DELIVERY REVIEWS vol. 65, 2013, pages 348 - 356, XP055082389**
- **PAINTER, R. G. ET AL.: 'Conditional expression of a suicide gene by the telomere reverse transcriptase promoter for potential post-therapeutic deletion of tumorigenesis' CANCER SCI. vol. 96, no. 9, 2005, pages 607 - 613, XP002405578**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 975 125 B1

## Description

Technical Field

The present invention relates to a microvesicle and a method for producing the same.

Background Art

Various cells are known to secrete or release microvesicles (small membrane vesicles), for example, exosomes *in vivo.* It has been thought that one of the roles of the microvesicles is to extracellularly release unnecessary intracellular components. In recent years, however, the possibility has been indicated that the microvesicles serve as signaling vehicles for transmitting substances such as proteins or lipids between secreting cells and their target cells and function in cell-cell interaction.

Diverse clinical applications of microvesicles, particularly, exosomes, have been proposed so far. For example, Patent Literature 1 discloses use of an exosome isolated from reticulocytes comprising a *Plasmodium sp.* antigen in defense against malaria. Patent Literature 2 discloses the treatment of cancer using an exosome from a B cell. Patent Literature 3 discloses use of a stem-cell derived microvesicle in endothelial or epithelial regeneration.

Lentivirus, for example, human immunodeficiency virus (HIV) infects cells and has the property of being integrated into the genomes of both dividing and non-dividing cells. Therefore, lentiviral vectors based on a lentiviral genome sequence are widely used as a tool for gene transduction.

Citation List

Patent Literature

Patent Literature 1: International Publication WO2011/080271
Patent Literature 2: International Publication WO2011/000551
Patent Literature 3: International Publication WO2009/057165

Summary of Invention

Technical Problem

An object of the present invention is to provide a microvesicle and a method for producing the same.

Solution to Problem

The present inventors have conducted diligent studies to attain the object and consequently found that the introduction of a transgene into cells using a lentiviral vector comprising the transgene under control of a telomerase reverse transcriptase (TERT) gene promoter can activate the integration of the transgene into host genome and its expression and can enhance the extracellular release of microvesicles having transgene products, etc., produced by the cells. On the basis of these findings, the present invention has been completed.

Specifically, the present invention encompasses the followings:

[1] A method for producing microvesicles comprising a transgene product and/or a lentiviral RNA comprising a transgene, comprising the steps of:

culturing a cell into which the transgene has been introduced using a lentiviral vector *in vitro* to extracellularly release microvesicles comprising the transgene product and/or the lentiviral RNA comprising the transgene, wherein said lentiviral vector is deficient in at least one structural protein gene, wherein said at least one structural protein gene is selected from the group consisting of gag, pol and env genes, and comprises the transgene under control of a telomerase reverse transcriptase (TERT) gene promoter in a lentiviral genome sequence, and collecting the microvesicles released.

[2] The method according to [1], wherein said lentiviral vector is deficient in env gene.

[3] The method according to any of [1] or [2], wherein said telomerase reverse transcriptase (TERT) gene promoter is a human TERT gene promoter.
[4] The method according to [3], wherein said human TERT gene promoter comprises the nucleotide sequence of SEQ ID NO: 1 or a nucleotide sequence having 90% or more sequence identity to the nucleotide sequence of SEQ ID NO: 1.
[5] The method according to [4], wherein said human TERT gene promoter comprises a nucleotide sequence having 95% or more sequence identity to the nucleotide sequence of SEQ ID NO: 1.
[6] The method according to any of [1] to [5], wherein said lentiviral RNA comprises a TERT gene promoter sequence upstream of the transgene.
[7] The method according to any of [1] to [6], wherein said lentiviral vector is:

(i) an RNA vector comprising the lentiviral genome sequence,
(ii) a DNA vector encoding an RNA comprising the lentiviral genome sequence, or
(iii) a viral particle carrying an RNA comprising the lentiviral genome sequence.

[8] The method according to any of [1] to [7], wherein said lentiviral genome sequence comprises at least a portion of TERT transcribed region between the TERT gene promoter and the transgene.
[9] The method according to [8], wherein said at least a portion of TERT transcribed region comprises the nucleotide sequence of SEQ ID NO: 2 or a nucleotide sequence having 90% or more sequence identity to the nucleotide sequence of SEQ ID NO: 2.
[10] The method according to any of [1] to [9], wherein said lentiviral genome sequence is an HIV genome sequence.
[11] The method according to [10], wherein said HIV genome sequence is an HIV-1 genome sequence.
[12] The method according to [11], wherein said HIV-1 genome sequence comprises 5' LTR; packaging signal $\psi$; gag gene; pol gene; vif gene; vpr gene; tat gene; rev gene; vpu gene; and 3' LTR.
[13] The method according to any of [1] to [12], wherein said transgene encodes a protein or RNA.
[14] The method according to any of [1] to [13], wherein said lentiviral vector comprises said transgene being a tumor-suppressor gene.
[15] The method according to [14], wherein said tumor-suppressor gene is PTEN or p16 gene.
[16] The method according to any of [1] to [15], wherein said lentiviral vector comprises said transgene that encodes a shRNA.
[17] The method according to [16], wherein said shRNA targets a gene encoding a cell proliferation regulator.
[18] The method according to [17], wherein said cell proliferation regulator is CDC6.
[19] The method according to any of [1] to [18], wherein said cell is a human cell.
[20] The method according to any of [1] to [19], wherein said cell is a kidney-derived cell.
[21] The method according to any of [1] to [20], wherein said cell is human embryonic kidney 293T cell.
[22] A microvesicle comprising a transgene product and/or a lentiviral RNA comprising a transgene, wherein said microvesicle is produced by the method according to any of [1] to [21].
[23] A method of gene transduction comprising, contacting a target cell with the microvesicle comprising the transgene product and/or the lentiviral RNA comprising the transgene according to [22] to fuse them, thereby introducing the transgene into the cell.
[24] The method according to [23], wherein said target cell is contacted with the microvesicle *in vitro.*
[25] A composition comprising the microvesicle according to [22].
[26] A pharmaceutical composition comprising the microvesicle according to [22].
[27] The pharmaceutical composition according to [26], which is for use in treatment of cancer.
[28] The pharmaceutical composition according to [27], wherein said cancer is selected from the group consisting of colon cancer, pancreatic cancer, kidney cancer, lung cancer, neuroblastoma, breast cancer, ovarian cancer, gastric cancer, prostate cancer, thyroid cancer and malignant lymphoma.
[29] The pharmaceutical composition according to [27] or [28], wherein said cancer involves an elevated expression of CDC6.
[30] The pharmaceutical composition according to any of [26] to [29], further comprising a pharmaceutically acceptable carrier.
[31] A method for treating a patie 3 to 4nt, comprising administering the microvesicle according to [22] to said patient in need of introduction of said transgene or said transgene product.
[32] The method according to [31], wherein said patient suffers from cancer.
[33] The method according to [32], wherein said cancer is selected from the group consisting of colon cancer, pancreatic cancer, kidney cancer, lung cancer, neuroblastoma, breast cancer, ovarian cancer, gastric cancer, prostate cancer, thyroid cancer and malignant lymphoma.
[34] The method according to [32] or [33], wherein said cancer involves an elevated expression of CDC6.

Effects of Invention

The present invention provides a microvesicle and a method for producing the same. Brief Description of Drawings

Figure 1 is a schematic diagram of the HIV-1 genomic region of plasmids having an HIV-1 backbone. Figure 1A is a schematic diagram of the HIV-1 genomic region of pNL4-3. Figure 1B is a schematic diagram of the HIV-1 genomic region of pTHTK.

Figure 2 shows recombinant lentiviral plasmid vectors pRBL0213T and pTHTN. Figure 2A is an agarose gel electrophoretogram showing the plasmid DNA mapping of pRBL0213T and pTHTN. Lane 1: pRBL0213T, *Eco*R I; Lane 2: pRBL0213T, *Mlu* I + *Xho* I; Lane 3: pRBL0213T, Nhe I; Lane 4: pTHTN, *Eco*R I; Lane 5: pTHTN, *Mlu* I + *Xho* I; Lane M: 1 kb ladder. Figure 2B is a schematic diagram of the HIV-1 genomic region of pRBL0213T. Figure 2C is a schematic diagram of the HIV-1 genomic region of pTHTN.

Figure 3 shows recombinant lentiviral plasmid vector pRBL001. Figure 3A is an agarose gel electrophoretogram showing the plasmid DNA mapping of pRBL001. Lane 1: pRBL001, *Eco*R I; Lane 2: pRBL001, *Eco*R I + *Nhe* I; Lane 3: pRBL001, *Mlu* I + *Xho* I; Lane 4: pRBL001, *Sal* I; Lane 5: pRBL0213T, *Nhe* I; Lane M: 1 kb ladder. Figure 3B is a schematic diagram of the HIV-1 genomic region of pRBL001. The restriction enzyme sites represent RI: *Eco*R I, Sal: *Sal* I, Mlu: *Mlu* I, Nhe: *Nhe* I and Xho: *Xho* I.

Figure 4 is a schematic diagram of the HIV-1 genomic region of recombinant lentiviral plasmid vectors. Figure 4A is a schematic diagram of the HIV-1 genomic region of pNL4-3 (wild-type HIV-1). Figure 4B is a schematic diagram of the HIV-1 genomic region of pD64V that has a mutation in HIV-1 integrase and is deficient in the integration of HIV-1 virus genomic DNA into host genome. Figure 4C is a schematic diagram of the HIV-1 genomic region of pTHTK. Figure 4D is a schematic diagram of the HIV-1 genomic region of pTHTN. Figure 4E is a schematic diagram of the HIV-1 genomic region of pTHTH that lacks the 5' portion of hTERT promoter. Figure 4F is a schematic diagram of the HIV-1 genomic region of pTHTC having a promoter sequence from human cytomegalovirus (CMV).

Figure 5 is a schematic diagram showing the preparation of *Bpm* I site for detecting LTR-Tag. Figure 5A is a schematic diagram showing the novel *Bpm* I site prepared in 5' LTR of the HIV-1 genomic region of pTHTK. Figure 5B is a schematic diagram showing that *Bpm* I restriction enzyme cleaves host chromosomal DNA at an integration site (14 nucleotides) adjacent to the *Bpm* I site copied to HIV-1 3' LTR. Figure 5C is a schematic diagram of the terminal structure of lentiviral genome.

Figure 6 is a schematic diagram showing the formation of LTR-Tag via ligation-mediated PCR (LM-PCR).

Figure 7 is a gel electrophoretogram of LM-PCR for detecting LTR-Tag.

Figure 8 shows results of examining the splicing of viral RNA. Figures 8A and 8B are photographs showing results of electrophoresing RT-PCR reaction products using 2.2% agarose gel in 1 x TAE. Figure 8A is a gel electrophoretogram showing PCR products obtained using primers 5' LTRU5 and 3' NL8960. Figure 8B is a gel electrophoretogram showing PCR products obtained using primers 5' LTRU5 and 3' NL5850. The positions of bands of spliced RNA fragments a (E1+A1/3'NL5850), b (E1+A2/3'NL5850), c1 (E1+E2+E3+A3/3'NL5850), c2 (E1+E2+A3/3'NL5850) and c3 (E1+E3+A3/3'NL5850) are shown on the right side of Figure 8B. Figure 8C is a schematic diagram showing an unspliced transcript. Figure 8D is a schematic diagram showing spliced RNA. In Figure 8D, A1 to A3 denote 3' splice acceptors and E1 to E3 denote exons.

Figure 9 is a graph showing results of p24 assay.

Figure 10 is a schematic diagram showing the constitution of plasmid vectors used in PTEN assay. Figure 10A shows pGL3-1375 (Empt.; plasmid size: approximately 7 kb) as a negative control in PTEN gene transduction. Figure 10B shows pcDNA3.1/CMV-hPTEN (Regul.; plasmid size: approximately 7.5 kb) as a positive control in PTEN gene transduction. Figure 10C shows retroviral vector pRBL016Bn (Retro.; plasmid size: approximately 7.9 kb) for PTEN gene transduction. Figure 10D shows recombinant lentiviral vector pRBL0213T (Lenti.; plasmid size: approximately 15 kb) for PTEN gene transduction. The restriction enzyme sites in the vectors represent BamH: *BamH* I; Bgl: *Bgl* II; RI: *EcoR* I; RV: *EcoR* V; Hd3: *Hin*d III; Mlu: *Mlu* I; Nco: *Nco* I; Nde: *Nde* I; Sal: *Sal* I; Stu: *Stu* I; and Xho: *Xho* I.

Figure 11 is a graph showing results of PTEN assay on transient transfection with a plasmid vector.

Figure 12 is a graph showing the ratio of PTEN activity in the mv lysates to PTEN activity in the cell lysates shown in Figure 11.

Figure 13 is a graph showing results of PTEN assay on recombinant lentiviral particle RBL0213T infection.

Figure 14 is a graph showing the ratio of PTEN activity in mv lysates to PTEN activity in cell lysates of cells transfected with recombinant lentiviral plasmid vector pRBL0213T or cells infected by recombinant lentiviral particle RBL0213T.

Figure 15 is a photograph showing results of Western blot on viral proteins or endogenous or foreign proteins in mv or in cells incubated with mv. Figure 15A shows results obtained using anti-Vpu antibody. Figure 15B shows results obtained using anti-RT antibody. Figure 15C shows results obtained using anti-FEN-1 antibody.

Figure 16 is a set of microscope photographs showing the delivery of contents to cells by mv in which c-myc-FEN-1 was encapsulated. Figure 16A shows an anti-c-myc antibody stained image. Figure 16B shows a DAPI stained image. Figure 16C shows an overlaid image of Figures 16A and 16B. Figure 16D shows an image prepared from the overlaid image by the color curve program in the image "adjustment" method of Photoshop(R) (Adobe Systems Inc.).

Figure 17 is a graph showing the cancer cell proliferation suppressive effect of Lenti-mv2010/CDC6 shRNA.

Figure 18 shows a photograph indicating results of Western blotting analysis in cellular protein extracts and mv lysates.

Figure 19 shows photographs showing typical morphologies observed for tumors from respective test groups by pathological examination. A, negative control group; B, interferon (IFN) $\alpha$-2b injection group; C, Cytomox PTEN injection group and D, Cytomox p53 injection group.

Figure 20 shows photographs showing typical morphologies observed for tumors from respective test groups by pathological test. A, low dose Cytomox EX injection group; B, high dose Cytomox EX injection group; C, low dose Cytomox HD injection group and D, high dose Cytomox HD injection group.

## Description of Embodiments

Hereinafter, the present invention will be described in detail.

### 1. Method for producing microvesicles

The present invention relates to a method for producing microvesicles. The method of the present invention comprises the steps of:

culturing a cell into which the transgene has been introduced using a lentiviral vector *in vitro* to extracellularly release microvesicles comprising the transgene product and/or the lentiviral RNA comprising the transgene, wherein said lentiviral vector is deficient in at least one structural protein gene as specified in the claims and comprises the transgene under control of a telomerase reverse transcriptase (TERT) gene promoter in a lentiviral genome sequence, and

collecting the microvesicles released.

The microvesicles produced by the method of the present invention comprise a transgene product and/or a lentiviral RNA comprising a transgene. The method of the present invention can efficiently produce microvesicles comprising a transgene product and/or a lentiviral RNA comprising a transgene.

The lentiviral vector according to the present invention refers to a vector for gene transduction having a lentiviral genome sequence as a basic backbone. Lentivirus is an RNA virus having reverse transcriptase. The lentivirus can integrate viral genomic DNA (proviral DNA) into the host chromosomes of not only dividing cells but non-dividing cells so that virus-derived genes can be expressed by the host cells. The lentiviral vector is based on such properties of the lentivirus.

The lentiviral vector according to the present invention can be:

(i) an RNA vector comprising the lentiviral genome sequence,
(ii) a DNA vector encoding an RNA comprising the lentiviral genome sequence, or
(iii) a viral particle carrying an RNA comprising the lentiviral genome sequence.

The RNA vector of (i) can be prepared, for example, by *in vitro* transcription from an expression vector or an expression cassette comprising the lentiviral genome sequence. The DNA vector of (ii) can include a plasmid vector. The plasmid vector usually comprises a DNA sequence encoding the RNA comprising the lentiviral genome sequence as well as a promoter and a terminator for bringing about the transcription of the DNA sequence, a replication origin, and a marker gene for screening for recombinants, etc. Such a DNA vector can be prepared by a method known in the art using a gene recombination technique or the like. The viral particle of (iii) may be a viral particle pseudotyped by an envelope protein of a different virus, for example, envelope glycoprotein G (VSV-G) of vesicular stomatitis virus (VSV). The pseudotyped viral particle may be prepared, for example, by: cotransfecting a cultured cell with a DNA vector encoding an RNA comprising the lentiviral genome sequence and a plasmid encoding the envelope protein (e.g., VSV-G) of the different virus; collecting a viral particle released into a medium; and purifying the particle.

The lentiviral vector used in the present invention is deficient in at least one viral structural protein gene in its lentiviral genome sequence. Such a lentiviral genome sequence may be one in which at least one viral structural protein gene has been disrupted (e.g., by the deletion of a partial or whole region or by the insertion of a nucleic acid molecule) in a full-length lentiviral genome sequence. In the present invention, the term "deficient" in a gene means that the whole gene is deleted or the gene is disrupted or mutated so that a functional protein cannot be expressed. The viral structural protein gene in which the lentiviral vector is deficient is at least one selected from the group consisting of gag, pol and env genes. The gag gene encodes a protein involved in viral particle formation. The pol gene encodes an enzyme such as reverse transcriptase (RT). The env gene encodes a coat (envelope) protein involved in adsorption on and penetration to host cells. For example, the lentiviral vector may be deficient in env gene. For example, a region corresponding to position 6344 to position 7611 of SEQ ID NO: 4 may be deleted from the lentiviral vector to result in the deficiency of HIV-1 env gene. In one embodiment, the cell does not have, in the genome or outside the chromosome, the at least one viral structural protein gene in which the lentiviral vector is deficient. Such a cell into which the transgene has been introduced using the lentiviral vector does not produce infectious lentiviral particles. Therefore, produced microvesicles are highly safe.

Examples of the lentiviral genome sequence used as a basic backbone in the lentiviral vector according to the present invention include, but not limited to, sequences from the genomes of human immunodeficiency virus (HIV), simian immunodeficiency virus (SIV), feline immunodeficiency virus (FIV) and canine immunodeficiency virus (CIV). The lentiviral genome sequence according to the present invention is preferably an HIV genome sequence. More specifically, the HIV genome sequence may be HIV-1 or HIV-2 genome sequence. Preferably, the HIV genome sequence may be HIV-1 genome sequence. The HIV may be a strain belonging to HIV-1 group M, N, O or P. More specifically, the HIV may be any of HIV strains including HIV-1 IIIb, HIV-1 SF2, HIV-1 SF162, HIV-1 BRU, HIV-1 NY5, HIV-1L AI, HIV-1 NL4-3, etc. An exemplary HIV genome sequence is available from Genbank accession Nos. EU541617, K03455 and K02013, etc. The lentiviral genome sequence may be RNA or may be DNA.

The lentiviral genome sequence in the lentiviral vector may comprise 5' LTR and 3' LTR, and optionally at least one selected from the group consisting of packaging signal ψ, gag gene, pol gene, vif gene, vpr gene, tat gene, rev gene, vpu gene or vpx gene, nef gene and env gene. In a preferred embodiment, the lentiviral genome sequence of HIV-1 in the lentiviral vector may comprise 5' LTR, packaging signal ψ, gag gene, pol gene, vif gene, vpr gene, tat gene, rev gene, vpu gene and 3' LTR. In another preferred embodiment, the lentiviral genome sequence of HIV-2 in the lentiviral vector may comprise 5' LTR, packaging signal ψ, gag gene, pol gene, vif gene, vpr gene, tat gene, rev gene, vpx gene and 3' LTR. Such a lentiviral genome sequence typically comprises at least one splice donor (SD) and splice acceptor (SA). In one embodiment, the lentiviral genome sequence in the lentiviral vector may comprise vpu gene, tat gene and rev gene. The lentiviral genome sequence in the lentiviral vector may be deficient in nef gene.

The lentiviral vector according to the present invention comprises the transgene under control of a telomerase reverse transcriptase (TERT) gene promoter in a lentiviral genome sequence. TERT is an enzyme that synthesizes telomeric repeat during DNA replication. The telomerase reverse transcriptase (TERT) gene promoter used in the present invention may be, but not limited to, a human TERT gene promoter. Preferably, the human TERT gene promoter may comprise the nucleotide sequence of SEQ ID NO: 1 or a nucleotide sequence having 90% or more sequence identity to the nucleotide sequence of SEQ ID NO: 1. More preferably, the human TERT gene promoter may comprise a nucleotide sequence having 95%, 97%, 99%, 99.5% or 99.9% or more sequence identity to the nucleotide sequence of SEQ ID NO: 1.

In one embodiment, also preferably, the lentiviral vector according to the present invention comprises the nucleotide sequence of SEQ ID NO: 5 or a sequence having 90% or more, preferably 95% or more, more preferably 99% or more, for example, 99.8% or more sequence identity thereto as the lentiviral genome sequence; and a sequence comprising the TERT gene promoter and the transgene under control thereof, which has been further inserted into the lentiviral genome sequence (preferably in nef gene).

In the present invention, the phrase "transgene under control of a TERT gene promoter" means that the transcription of the transgene is initiated by the activity of the TERT gene promoter. Preferably, the transgene is located downstream of the TERT gene promoter.

At least a portion of TERT transcribed region may exist between the TERT gene promoter and the transgene inserted in the lentiviral genome sequence. The at least a portion of TERT transcribed region may comprise at least the first exon of TERT gene. The at least a portion of TERT transcribed region may comprise the nucleotide sequence of SEQ ID NO: 2 or a nucleotide sequence having 90%, 95%, 97%, 99% or 99.5% or more sequence identity to the nucleotide sequence of SEQ ID NO: 2.

The transgene used in the present invention may encode any protein or RNA such as microRNA (miRNA), small interfering RNA (siRNA) or short hairpin RNA (shRNA). In one embodiment, the transgene may be a tumor-suppressor gene. Examples of the tumor-suppressor gene include, but not limited to, tumor-suppressor genes known to those skilled in the art, such as p53, BRCA1, Rb, PTEN and p16 genes. Preferably, the tumor-suppressor gene may be PTEN or p16 gene. The PTEN protein is a phosphatase which catalyzes the dephosphorylation of phosphatidylinositol 3,4,5-triphosphate (PtdIns(3,4,5)P3). The PTEN gene may comprise the nucleotide sequence of SEQ ID NO: 6 or a nucleotide sequence having 90% or more, preferably 95% or more, more preferably 99% or more, for example, 99.5% or 99.9% or more sequence identity thereto. The PTEN gene also may be a nucleic acid encoding a PTEN protein that consists of the amino acid sequence of SEQ ID NO: 22 (GenBank accession Nos. AAD13528 and NP_000305) or an amino acid sequence having 90% or more, preferably 95% or more, more preferably 99% or more, for example, 99.5% or 99.7% or more sequence identity thereto. p16 protein (also referred to as $p16^{INK4a}$) is known as a cyclin-dependent kinase (CDK) inhibitor. For example, p16 gene may comprise the nucleotide sequence of SEQ ID NO: 23 (GenBank accession No. L27211) or a nucleotide sequence having 80% or more, preferably 90% or more, more preferably 95% or more, further preferably 99% or more, for example, 99.5% or 99.9% or more sequence identity thereto. p16 gene also may be a nucleic acid encoding a protein consisting of the amino acid sequence of SEQ ID NO: 24 or an amino acid sequence having 90% or more, preferably 95% or more, more preferably 99% or more sequence identity thereto. p16 gene preferably encodes a protein having CDK inhibitory activity. Examples of p16 gene include a nucleic acid comprising the nucleotide sequence of positions 434 to 480 of SEQ ID NO: 25. Further, p53 gene may comprise the nucleotide sequence of SEQ ID NO: 26 (GenBank accession No. BC003596) or a nucleotide sequence having 80% or more, preferably 90% or more, more preferably 95% or more, further preferably 99% or more, for example, 99.5% or 99.9% or more sequence identity thereto. p53 gene also may be a nucleic acid encoding a protein consisting of the amino acid sequence of SEQ ID NO: 27 or an amino acid sequence having 90% or more, preferably 95% or more, more preferably 99% or more sequence identity thereto. p53 gene preferably encodes a protein having transcription factor activity. In the context of the present application, a nucleic acid may be DNA or RNA and may comprise a modified base.

In another embodiment, the transgene may encode a shRNA. The shRNA is a single-stranded RNA in which an antisense sequence complementary to a target sequence and a sense sequence (typically having a poly U overhang at the 3' end) complementary to the antisense sequence are linked via a linker, and forms a hairpin structure via intramolecular double strand formation. Such a shRNA is intracellularly cleaved at its double-stranded portion into siRNAs, which can in turn cause RNAi to suppress the expression of a target gene comprising the target sequence. A shRNA precursor may be transcribed from the transgene and then subjected to editing and processing to form a shRNA. Even in that case, it is defined herein that such a transgene encodes a shRNA. A target of the shRNA may include, but not limited to, a gene encoding a cell proliferation regulator. Examples of the cell proliferation regulator include proteins involved in DNA replication or the regulation of cell cycle, such as CDC6, cyclin E, CDK2, CDT1, ORC2 and MCM7. Preferably, the cell proliferation regulator may be CDC6. The CDC6 is a protein that plays a central role in the initiation of DNA replication. CDC6 knockdown has been found to result in the apoptosis of human cancer cells (Feng, et al., Cancer Res., 2003, Vol. 63, p. 7356-7364; Lau et al., EMBO Rep., 2006, Vol. 7, p. 425-430; and Feng et al., Mol. Cell. Biochem., 2008, Vol. 311, p. 189-197). CDC6 gene may comprise the nucleotide sequence of SEQ ID NO: 7 or a nucleotide sequence having 90% or more, preferably 95% or more, more preferably 99% or more, for example 99.5% or 99.9% or more sequence identity thereto. The transgene encoding CDC6 shRNA may comprise a DNA sequence consisting of the nucleotide sequence of SEQ ID NO: 10 or a nucleotide sequence having 90% or more, preferably 95% or more, more preferably 98% or more sequence identity thereto. Typically, the CDC6 shRNA comprises: an antisense sequence of CDC6 shRNA consisting of the nucleotide sequence of SEQ ID NO: 19 or a nucleotide sequence having 90% or more sequence identity thereto; a linker consisting of the nucleotide sequence of SEQ ID NO: 20 or a nucleotide sequence having 80% or more sequence identity thereto; and a sense sequence consisting of a sequence complementary to the antisense sequence and a 3' poly U overhang of two or more bases. The transgene product may comprise an RNA from the transgene as described above or a protein translated from the RNA from the transgene.

Each lentiviral vector may comprise one or more transgenes. Two or more transgenes may be introduced into a cell using one lentiviral vector or using two or more lentiviral vectors. For example, the lentivirus vector may contain a transgene that encodes shRNA and a transgene that is a tumor-suppressor gene. In a preferred embodiment, the lentiviral vector comprises a transgene encoding CDC6 shRNA and/or a transgene encoding p16 protein.

The transgene according to the present invention is introduced into a cell using the lentiviral vector *in vitro.* The organism species of the cell used in the present invention is preferably the same species as a recipient of the microvesicles to be produced. The cell may be, but not limited to, a mammalian cell, for example, a cell of dog, cat, cattle, sheep, mouse, rat or primate such as monkey or human. A human cell is preferred. Also preferably, the cell may be a kidney-derived cell, a uterus-derived cell, a lymphocyte-derived cell or a fibroblast cell. The cell may include human embryonic kidney 293T cells, human uterine cervix cancer HeLa cells, human lymphocyte CEM cells, N144 fibroblast cells and other human cell lines. Preferably, the cell may be human embryonic kidney 293T cell or human uterine cervix cancer HeLa cell.

The introduction of the transgene into the cell using the lentiviral vector can be performed by a method known in the art. The introduction of the transgene into the cell using the RNA vector or the DNA vector as the lentiviral vector may be performed by calcium phosphate method, lipofection method, DEAE dextran method or electroporation method or the like known in the art and may be performed using commercially available transfection reagents such as Lipofectamine(R) 2000 (Invitrogen) and FuGene(R) 6 (Roche). The introduction of the transgene into the cell using the viral particle as the lentiviral vector can be performed by adding the viral particle into a culture medium of the cell to infect the cell by the virus.

In addition to the lentiviral vector, an additional expression vector may also be introduced into the cell. Such an expression vector may comprise a transgene encoding a protein or RNA to be further encapsulated in the microvesicles, under control of a promoter. The cell into which the transgene has been thus introduced is then cultured. The culture may be performed by an appropriate method according to the cell. For example, the culture may be performed for 1 to 5 days, for example, 2 to 4 days, specifically 36 hours to 96 hours or 36 hours to 72 hours, in DMEM/high-glucose complete medium supplemented with 10% fetal bovine serum and an appropriate antibiotic. In the cell into which the transgene has been introduced using the lentiviral vector, lentiviral DNA (e.g., produced from the lentiviral RNA by the action of the reverse transcriptase) is integrated into intracellular genome by the action of integrase and the like to form proviral DNA. From this proviral DNA, the lentiviral RNA is produced by transcription mediated by intracellular RNA polymerase II. From the lentiviral RNA thus produced, the transgene product is usually produced through RNA splicing and/or protein translation, etc. The transgene product and/or the lentiviral RNA thus produced in the cell are incorporated into the microvesicles. The cell extracellularly releases such microvesicles during the culture so that the microvesicles accumulate in the medium. Preferably, the lentiviral vector used in the present invention is deficient in the structural protein gene, thereby forming no virus-like particle and allowing no virus-like particle to accumulate in the medium.

The method for producing microvesicles according to the present invention comprises the step of collecting the microvesicles released. The collection of the microvesicles can be performed by collecting the cell culture medium. The microvesicles thus collected may be further purified. For example, larger vesicles can be precipitated and removed by centrifugation at 1,000 to 10,000 x g (in a preferred embodiment, 9,000 x g), thereby purifying the microvesicles collected. Such centrifugation may be performed one or more times (preferably with different centrifugal forces). Alternatively or additionally, for example, the microvesicles can be purified by ultrafiltration using a membrane with a molecular weight cutoff of 1,000 kDa. Further, the microvesicles can also be precipitated by mixing with a PEG/NaCl solution and centrifugation, thereby purifying the microvesicles. After such purification, the microvesicles may be PEGylated. The PEGylation of the microvesicles can be performed using various types of PEGylating reagents and can be performed using, for example, methoxy PEG succinimidyl carbonate NHS (mPEG-NHS) (Croyle et al., J. Virol., 2004, Vol. 78, p. 912-921). For example, methoxy PEG succinimidyl carbonate NHS (mPEG-NHS, m.w. 10K (NANOCS, USA)) is added into the solution containing microvesicles and the mixture can be incubated at room temperature for 60 minutes on a rotary platform, thereby PEGylating the microvesicles. The microvesicles PEGylated or unPEGylated may then be subjected to buffer replacement by dialysis (e.g., using 1 x PBS), concentration by ultrafiltration and filtration through a syringe filter (e.g., having 0.45-μm pore size), etc.

The microvesicles produced by the method of the present invention comprise the transgene product and/or the lentiviral RNA comprising the transgene. The lentiviral RNA may comprise a TERT gene promoter sequence upstream of the transgene. The transgene is introduced into a cell using the lentiviral vector comprising the TERT gene promoter sequence, thereby promoting the integration of the transgene into host genome and its expression and further enhancing the release of microvesicles from the cell. Therefore, the microvesicles can be efficiently produced.

In one embodiment, the present invention also relates to:
a method for producing microvesicles comprising a transgene product and/or a lentiviral RNA comprising a transgene, comprising the steps of:

introducing the transgene into a cell using a lentiviral vector *in vitro,* wherein said lentiviral vector is deficient in at least one structural protein gene and comprises the transgene under control of a telomerase reverse transcriptase (TERT) gene promoter in a lentiviral genome sequence,
culturing the cell to extracellularly release microvesicles comprising the transgene product and/or the lentiviral RNA comprising the transgene, and
collecting the microvesicles released.

2. Microvesicle produced by method of the present invention

The present invention also relates to a microvesicle comprising a transgene product and/or a lentiviral RNA comprising a transgene, wherein the microvesicle is produced by the method of the present invention.

The microvesicle (mv) according to the present invention refers to a membrane vesicle of 5 nm to 5 μm, preferably 10 nm to 1 μm, more preferably 20 nm to 500 nm in size that is produced by cells and extracellularly released or shed. The size of the microvesicle can be determined by an electron microscope method. Examples of the microvesicle generally include, but not limited to, exosomes, shedding microvesicles and apoptotic bodies. Typically, the microvesicle of the present invention is an exosome. The exosome is a membrane vesicle composed of a lipid bilayer. The exosome has a size of 150 nm or smaller, typically, 20 to 120 nm or 40 to 100 nm. The exosome is extracellularly secreted and produced by exocytosis resulting from the cell membrane fusion of multivesicular body (MVB) formed via the inward budding of an endosomal membrane.

The microvesicle according to the present invention may comprise a protein and/or RNA, such as the transgene product and/or the lentiviral RNA comprising the transgene, which has been transported from a host cell and encapsulated therein. Examples of the RNA that may be contained in the microvesicle include, but not limited to, mRNA, miRNA, shRNA, siRNA and lentiviral RNA (including various splicing variants of the lentiviral RNA). Examples of the protein that may be contained in the microvesicle include, but not limited to, viral proteins (e.g., HIV-1 Vpu protein and reverse transcriptase (RT) protein), cell-endogenous proteins (e.g., cytoskeletal proteins, signaling proteins and enzymes) and foreign transgene products. For example, the exosome may generally comprise a cytoskeletal protein (e.g., tubulin, actin and actin-binding protein), a membrane transport-related protein (e.g., annexin and Rab protein), a signaling protein (e.g., protein kinase and 14-3-3), a metabolic enzyme (e.g., GAPDH, ATPase and enolase), tetraspanin family (e.g., CD9, CD63, CD81 and CD82), a heat shock protein (e.g., HSP90 and HSP70), a MVB biosynthesis protein (e.g., Alix and TSG101), an immunomodulatory molecule (e.g., MHCI and MHCII), etc.

Lentiviral DNA (proviral DNA) comprising the lentiviral genome sequence comprising the transgene described above that has been introduced into a cell by the method of the present invention is integrated into host genome. The lentiviral RNA contained in the microvesicle of the present invention is transcribed from this proviral DNA. The lentiviral RNA may comprise an RNA sequence of the TERT gene promoter (e.g., hTERT promoter) at 5'upstream of the transgene.

In a particularly preferred embodiment, the microvesicle of the present invention may comprise a tumor-suppressor protein such as PTEN and p16 protein and/or shRNA such as CDC6 shRNA or its precursor RNA, as the transgene product.

The microvesicle of the present invention can be taken up by other cells to deliver, into the cells, the transgene product and/or the lentiviral RNA comprising the transgene contained in the microvesicle.

3. Method of gene transduction using microvesicle of the present invention

The present invention also relates to a method of gene transduction comprising, contacting a target cell with the microvesicle comprising the transgene product and/or the lentiviral RNA comprising the transgene to fuse them, thereby introducing the transgene into the cell, wherein the microvesicle is produced by the method of the present invention. In one embodiment, the target cell can be contacted with the microvesicle *in vitro* or *in vivo*.

The microvesicle, particularly, the exosome, can penetrate into its neighboring other cells to participate in cell-cell interaction. The microvesicle is thought to be able to reach the inside of the target cell via membrane fusion or through an endocytosis-like manner, though the present invention is not restricted to this theory. In the method of gene transduction of the present invention, the contact of the target cell with the microvesicle may be made by any method known to those skilled in the art. For example, the *in vitro* contact of the target cell with the microvesicle may be made by the addition of the microvesicle into a cell culture medium. The *in vivo* contact of the target cell with the microvesicle may be made, for example, by the oral administration of the microvesicle or by the parenteral administration such as direct application or injection of the microvesicle to a target site (e.g., intrahepatic, intraarticular, intraventricular and intranasal sites). Other *in vivo* administration methods and administration sites that may be used will be described later in relation to administration methods and administration sites for a pharmaceutical composition.

The method of gene transduction of the present invention can efficiently deliver, into the target cell, the transgene product and/or the lentiviral RNA comprising the transgene contained in the microvesicle.

4. Composition comprising microvesicle of the present invention

The present invention also relates to a composition comprising the microvesicle produced by the method of the present invention. The composition can comprise any ingredient other than the microvesicle according to the intended use thereof. For example, the composition may be for use in gene transduction. In that case, the composition may comprise a drug promoting gene transduction and/or a drug stabilizing nucleic acid, etc.

5. Pharmaceutical composition comprising microvesicle of the present invention and treatment method

The present invention also relates to a pharmaceutical composition comprising the microvesicle produced by the method of the present invention.

In one embodiment, the pharmaceutical composition may be for use in treatment of diseases such as cancer, diabetes, neurodegenerative disease, immune dysfunction, inflammation, liver cirrhosis, arteriosclerosis, thrombus and infection. Preferably, the pharmaceutical composition may be for use in treatment of cancer. More specifically, the cancer may be selected from the group consisting of, for example, colon cancer, pancreatic cancer, kidney cancer, lung cancer, neuroblastoma, breast cancer, ovarian cancer, gastric cancer, prostate cancer, thyroid cancer and malignant lymphoma. In one embodiment, the transgene product contained in the microvesicle may cause a reduced expression of CDC6. For example, the transgene product may be a shRNA targeting CDC6. In that case, the disease to be treated, for example, cancer, may involve an elevated expression of CDC6.

In the case of using the microvesicle in the pharmaceutical composition, the transgene or the transgene product contained in the microvesicle according to the present invention functions to prevent and/or treat the disease. For example, such a transgene may be a tumor-suppressor gene such as PTEN or p16 gene and/or may be a gene encoding a shRNA targeting a gene encoding a cell proliferation regulator or its precursor. In one embodiment, the transgene is PTEN gene and/or a gene encoding CDC6 shRNA. In this embodiment, the pharmaceutical composition may comprise, for example, the microvesicle produced by the method of the present invention in which the transgene is PTEN gene, and the microvesicle produced by the method of the present invention in which the transgene is a gene encoding CDC6 shRNA, in combination. In another embodiment, the transgene is p16 gene and/or a gene encoding CDC6 shRNA. In this embodiment, the pharmaceutical composition may comprise, for example, the microvesicle produced by the method of the present invention in which the transgene is p16 gene, and the microvesicle produced by the method of the present invention in which the transgene is a gene encoding CDC6 shRNA, in combination.

The pharmaceutical composition of the present invention comprises a liquid medium in addition to the microvesicle of the present invention. Examples of the liquid medium include water, physiologically acceptable buffer solutions (phosphate-buffered saline, etc.) and biocompatible aqueous mediums such as propylene glycol and polyoxyethylene sorbitan fatty acid ester. Such a medium is desirably sterilized and preferably adjusted to be isotonic to blood, if necessary.

The pharmaceutical composition may comprise a pharmaceutically acceptable carrier. The "pharmaceutically acceptable carrier" refers to an additive usually used in the field of pharmaceutical techniques. Examples of the pharmaceutically acceptable carrier include suspending agents, tonicity agents, buffers and preservatives. Such a carrier is used mainly for facilitating formulation and maintaining the dosage form and drug effects and may be appropriately used according to the need.

For example, glyceryl monostearate, aluminum monostearate, methylcellulose, carboxymethylcellulose, hydroxymethylcellulose and sodium lauryl sulfate can be used as the suspending agents. Examples of the tonicity agents include sodium chloride, glycerin and D-mannitol. Examples of the buffers include phosphate, acetate, carbonate and citrate buffer solutions. Examples of the preservatives include benzalkonium chloride, parahydroxybenzoic acid and chlorobutanol.

The pharmaceutical composition can also comprise, if necessary, a corrigent, a thickener, a solubilizing agent, a pH adjuster, a diluent, a surfactant, an expander, a stabilizer, an absorption promoter, a wetting agent, a humectant, an adsorbent, a coating agent, a colorant, an antioxidant, a flavoring agent, a sweetener, an excipient, a binder, a disintegrant, a disintegration inhibitor, a filler, an emulsifier, a flow control additive, a lubricant, or the like, in addition to those described above.

The pharmaceutical composition of the present invention can also contain an additional drug without losing pharmacological effects possessed by the microvesicle of the present invention. For example, the pharmaceutical composition may contain a predetermined amount of an antibiotic.

The dosage form of the pharmaceutical composition is not limited and can be any form that neither inactivates the microvesicle nor inactivates the transgene product and/or the lentiviral RNA contained in the microvesicle. The dosage form of the pharmaceutical composition may be, for example, a liquid, solid or semisolid form. Specific examples of the dosage form include: parenteral dosage forms such as injections, suspensions, emulsions, creams, eye drops, nasal drops, ointments, plasters, patches and suppositories; and oral dosage forms such as liquid formulations, capsules, sublingual formulations, troches, powders, tablets and granules. The dosage form of the pharmaceutical composition is preferably a liquid formulation such as an injection.

The pharmaceutical composition can be administered to an organism in a pharmaceutically effective amount for treatment of the target disease. The recipient organism may be a vertebrate, for example, a mammal, bird, amphibian or reptile and is preferably a mammal. Examples of the mammal include: nonprimates such as dog, cat, horse, pig, cattle, goat, sheep, mouse and rat; and primates such as human, chimpanzee and gorilla. The mammal is preferably a human.

The "pharmaceutically effective amount" in the present specification refers to a dose required for the microvesicle contained in the pharmaceutical composition of the present invention to prevent or treat the target disease or alleviate symptoms with few or no harmful adverse reactions against the recipient organism. A specific dose differs depending on the type of the disease to be prevented and/or treated, the mechanism of action underlying the occurrence of the disease, the dosage form used, information about a subject and an administration route, etc. The range of the pharmaceutically effective amount and a preferred administration route of the pharmaceutical composition that is administered to a human are generally set on the basis of data obtained from cell culture assay and animal experiments. The final dose can be determined and adjusted by the judgment of, for example, a physician, according to an individual subject. The information about the subject to be taken into consideration in that case includes the degree of progression or severity of the disease, general health conditions, age, body weight, sex, diet, drug sensitivity and resistance to treatment, etc. In one embodiment, when the transgene encodes a protein, the pharmaceutical composition of the present invention may be administered in one or more doses of $1 \times 10^4$ to $1 \times 10^8$ transfection unit (t.u.)/kg body weight, for example, $1 \times 10^5$ to $1 \times 10^7$ t.u./kg body weight or $2 \times 10^5$ to $5 \times 10^6$ t.u./kg body weight, per single dose, by direct injection into affected sites or intravenous injection. Here, the transfection unit can be determined by introducing a transgene into a cell (for example a human embryonic kidney 293T cell) using 10 $\mu$g of a lentiviral vector of the present invention (for example a DNA vector such as a plasmid vector, e.g., pRBL0213T); determining the amount of the transgene product (e.g., protein, such as PTEN protein) in a fraction of microvesicles (mv) released from the cell, e.g., on the basis of ELISA assay; and normalizing the determined amount (transfection efficiency) as one transfection unit being equivalent to 20 pg of the transgene product (e.g., protein) per 1000 cells. The pharmaceutical composition of the present invention may be administered twice or more at predetermined intervals of time, for example, every 1 hour, 3 hours, 6 hours or 12 hours, every day, every 2 days, 3 days or 7 days, or every 1 month, 2 months, 3 months, 6 months or 12 months. Any other parenteral administration or oral administration can be performed in an amount that follows those described above. In the case of particularly severe symptoms, the dose may be increased according to the symptoms.

The administration of the pharmaceutical composition may be systemic administration or local administration and can be appropriately selected according to the type of the disease, the site where the disease occurs, or the degree of progression, etc. If the disease occurs at a local site, the pharmaceutical composition is preferably administered locally by direct administration to the local site (e.g., tumor) and its neighborhood using injection or an indwelling catheter or the like. This is because the microvesicle of the present invention can be administered in a sufficient amount to the site (tissue or organ) to be treated and has no influence on other tissues. Meanwhile, as in metastatic cancer, the site to be treated may not be identified, or the disease may occur systemically. In that case, systemic administration through intravenous injection or the like is preferred. This is because the microvesicle of the present invention can be spread systemically via blood flow, thereby permitting administration to even a lesion that cannot be found by diagnosis.

The pharmaceutical composition can be administered by any appropriate method that does not inactivate the active ingredient contained therein. For example, the administration may be parenteral (e.g., injection, aerosol, application, eye drop, nasal drop or indwelling catheter) or oral. Injection is preferred.

In the case of administration by injection, the injection site may be a non-limiting site where the microvesicle of the present invention can exert its functions and attain the purpose of the pharmaceutical composition. Examples of the injection site include intravenous, intraarterial, intrahepatic, intramuscular, intraarticular, intramedullary, intraspinal, intraventricular, percutaneous, subcutaneous, intracutaneous, intraperitoneal, intranasal, intestinal and sublingual sites. In one embodiment, direct administration to tumor is also preferred.

The pharmaceutical composition of the present invention can be used to effectively achieve the prevention and/or treatment of the disease by the transgene product or the like contained in the microvesicle.

Thus, the present invention also provides a method for treating a patient, comprising administering the microvesicle produced by the method of the present invention to the patient in need of introduction of the transgene or the transgene product. The patient may suffer from cancer, diabetes, neurodegenerative disease, immune dysfunction, inflammation, liver cirrhosis, arteriosclerosis, thrombus or infection. Preferably, the patient suffers from cancer. More specifically, the cancer may be selected from the group consisting of, for example, colon cancer, pancreatic cancer, kidney cancer, lung cancer, neuroblastoma, breast cancer, ovarian cancer, gastric cancer, prostate cancer, thyroid cancer and malignant lymphoma. In one embodiment, the transgene product contained in the microvesicle may cause a reduced expression of CDC6. For example, the transgene product may be a shRNA targeting CDC6. In that case, the disease to be treated, for example, cancer, may involve an elevated expression of CDC6. Administration methods and administration sites for the microvesicle to the patient can be used as described above in relation to the administration methods and administration sites for the pharmaceutical composition.

The treatment method of the present invention can effectively treat the disease such as cancer in the patient. In one preferred embodiment, the method for treatment of cancer of the present invention can inhibit (reduce) the growth of tumors.

6. Description of sequence

SEQ ID NO: 1 shows the nucleotide sequence of the telomerase reverse transcriptase (TERT) gene promoter from *Homo sapiens,* which was used to produce plasmids pRBL0213T, pTHTN and pRBL001 (see, Figures 2A and 2B and Figure 3B). The genomic DNA sequence comprising human TERT gene is available under Genbank accession No. AF128893.

SEQ ID NO: 2 shows the nucleotide sequence of the 5' portion of TERT transcribed region from *Homo sapiens,* which was inserted to produce plasmids pRBL0213T, pTHTN and pRBL001.

SEQ ID NO: 3 shows the nucleotide sequence comprising the TERT gene promoter (SEQ ID NO: 1) and the 5' portion (SEQ ID NO: 2) of TERT transcribed region from *Homo sapiens,* which was used to produce plasmids pRBL0213T, pTHTN and pRBL001. SEQ ID NO: 3 comprises upstream sequence, the whole first exon and a part of the second exon of the TERT gene. The first exon starts at position 1390 and ends at position 1670 of SEQ ID NO: 3. The second exon starts at position 1775 of SEQ ID NO: 3.

SEQ ID NO: 4 shows the sequence of recombinant plasmid pNL4-3 clone comprising the nucleotide sequence of full-length genomic DNA of HIV-1 NL4-3 strain (Genbank accession No. M19921). The nucleotide sequence from position 1 to position 9709 of SEQ ID NO: 4 corresponds to HIV-1 genome (5' LTR to 3' LTR). The nucleotide sequence from position 6221 to position 8785 of SEQ ID NO: 4 encodes env protein. For the structure of HIV-1 genome, see Figure 1A.

SEQ ID NO: 5 shows the nucleotide sequence of HIV-1 genomic region in plasmid pTHTK, i.e., the nucleotide sequence from 5' LTR to 3' LTR in pTHTK (see Figure 1B). The nucleotide sequence from position 6344 to position 7611 of SEQ ID NO: 4 was deleted from pNL4-3. Further, pNL4-3 was cleaved between positions 8650 and 8651 of SEQ ID NO: 4 with restriction enzyme Hpa I and the nucleotide sequence from position 7383 to position 7674 of SEQ ID NO: 5 was inserted thereinto to produce pTHTK.

SEQ ID NO: 6 shows the nucleotide sequence of PTEN CDS (from start codon to stop codon) from *Homo sapiens,* which was used to produce plasmid pRBL0213T (see Figure 2B). Human PTEN mRNA sequence is available under Genbank accession No. NM_000314.

SEQ ID NO: 7 shows the nucleotide sequence of CDC6 CDS from *Homo sapiens.* Human CDC6 mRNA sequence is available under Genbank accession No. NM_001254.

SEQ ID NOs: 8 and 9 show the oligonucleotide sequences Cdc6-5-A and Cdc6-3-A, respectively, which were used to prepare double-stranded oligonucleotide inserted into plasmid pRBL001 (see Figure 3B).

SEQ ID NO: 10 shows the DNA sequence encoding CDC6 shRNA.

SEQ ID NO: 11 shows the nucleotide sequence of primer B-NLR8950.

SEQ ID NO: 12 shows the nucleotide sequence of oligonucleotide HD-A.

SEQ ID NO: 13 shows the nucleotide sequence of oligonucleotide HD-S.

SEQ ID NO: 14 shows the nucleotide sequence of forward primer BHU5-S2.

SEQ ID NO: 15 shows the nucleotide sequence of reverse primer HDA/SBOT.

SEQ ID NO: 16 shows the nucleotide sequence of primer 5' LTRU5.

SEQ ID NO: 17 shows the nucleotide sequence of primer 3' NL5850.

SEQ ID NO: 18 shows the nucleotide sequence of primer 3' NL8960.

SEQ ID NO: 19 shows the nucleotide sequence of the antisense sequence of CDC6 shRNA.

SEQ ID NO: 20 shows the nucleotide sequence of the linker in CDC6 shRNA.

SEQ ID NO: 21 shows the nucleotide sequence of the partial fragment of SV40 large T antigen gene.

SEQ ID NO: 22 shows the amino acid sequence of the human PTEN protein encoded by the PTEN gene of SEQ ID NO: 6.

SEQ ID NO: 23 shows the nucleotide sequence (CDS) of human $p16^{INK4a}$ gene (GenBank accession No. L27211).

SEQ ID NO: 24 shows the amino acid sequence of the human $p16^{INK4a}$ protein encoded by the $p16^{INK4a}$ gene of SEQ ID NO: 23.

SEQ ID NO: 25 shows the nucleotide sequence of plasmid pCMV p16INK4a.

SEQ ID NO: 26 shows the nucleotide sequence (CDS) of human p53 gene (GenBank accession No.BC003596).

SEQ ID NO: 27 shows the amino acid sequence of the human p53 protein encoded by the p53 gene of SEQ ID NO: 26.

Examples

Hereinafter, the present invention will be described more specifically with reference to Examples. However, the technical scope of the present invention is not limited to these Examples.

Example 1 Recombinant lentiviral plasmid vector

Recombinant lentiviral plasmid vectors used in Examples described later are derived from HIV-1 genomic DNA vector pNL4-3 (Figures 1A and 4A) (Adachi A et al., J. Virol., 1986, p. 284-291). Recombinant pNL4-3 clone sequence comprising the full-length genomic DNA sequence of HIV-1 NL4-3 strain is available under Genbank accession No. M19921 (SEQ ID NO: 4). A recombinant lentiviral plasmid vector pD64V (Figure 4B) has a mutation that results in the substitution of aspartic acid at position 64 of HIV-1 integrase with valine in pNL4-3. The pD64V was kindly provided by Dr. Sam Chow, the Department of Molecular and Medical Pharmacology of University of California Los Angeles (UCLA).

Other recombinant lentiviral plasmid vectors used were prepared from construct pTHTK (Figure 1B). The pTHTK is derived from the pNL4-3 described above (Figures 1A and 1B). The pTHTK lacks nucleotides 6344 to 7611 (based on SEQ ID NO: 4; the same holds true for the description below unless otherwise specified) in the HIV-1 genomic DNA of pNL4-3 as a result of treatment with restriction enzymes *Kpn* I and *Bgl* II. Specifically, two sites, *Kpn* I (positions 6343 to 6348) and *Bgl* II (positions 7611 to 7616), in HIV-1 backbone were blunt-ended and re-ligated to prepare pTHTK deficient in HIV-1 env gene encoding envelope p120 glycoprotein. Further, the pTHTK was cleaved at the only one Hpa I site (positions 8648 to 8653) and modified by the addition of *Mlu* I linker (nucleotide sequence from positions 7383 to 7674 of SEQ ID NO: 5). The nucleotide sequence of the HIV-1 genomic region (i.e., 5' LTR to 3' LTR) of pTHTK is shown in SEQ ID NO: 5.

For ligation with a DNA fragment (transgene expression cassette) to be cloned, the vector pTHTK was cleaved 5'-terminally at *Mlu* I site and 3'-terminally at *Xho* I site (positions 8887 to 8892). The insertion of the transgene expression cassette to the *Mlu* I-*Xho* I site of this vector disrupts HIV-1 nef gene open reading frame. Nef protein encoded by the nef gene plays a role in viral infection and spreading and extracellular release of virion. Therefore, the insert-containing (i.e., nef-defective) lentiviral plasmid vector will produce viral particles less infectious for $CD4^+$ cells, unlike wild-type HIV-1 NL4-3 strain. In contrast, for VSV/G pseudotyped RBL0213T, RBL001, THTN and so on, whether Nef defective or not, more viral particles were budding out than that of wild-type NL4-3, as described below (see, e.g., Fig. 9).

The pTHTK backbone thus obtained was used to construct recombinant lentiviral plasmid vectors as described below.

(a) pRBL0213T

The pRBL0213T is a plasmid in which DNA encoding human PTEN gene was inserted under control of hTERT gene promoter (hTERT promoter) (Figure 2B).

Plasmid pGL-1375 was used as a source of the hTERT promoter (Takakura et al., Cancer Res., 1999, p. 551-557). The pGL-1375 was kindly provided by Dr. Satoru Kyo (Kanazawa University of Japan). The pGL-1375 has hTERT promoter DNA fragment inserted between *Mlu* I and *Bgl* II sites. hTERT 5' upstream sequence is available under Genbank accession No. AF128893. The hTERT promoter DNA fragment was excised from pGL-1375 and inserted to the pTHTK backbone.

Human PTEN mRNA sequence is available under Genbank accession No. NM-000314 (SEQ ID NO: 6). The PTEN gene-encoding DNA used to construct pRBL0213T was excised from vector pcDNA3.1/CMV-hPTEN (kindly provided by Dr. Hong Wu, Department of Molecular Medicine of University of California Los Angeles (UCLA)). The PTEN gene-encoding DNA was further inserted to the pTHTK backbone to prepare pRBL0213T.

The pRBL0213T comprises: the nucleotide sequence of SEQ ID NO: 3 consisting of the hTERT promoter sequence (SEQ ID NO: 1) and the 5' portion (SEQ ID NO: 2) of hTERT transcribed region containing the first exon and a part of the second exon of the hTERT gene; and the PTEN coding sequence.

(b) pTHTN

The pTHTN is a plasmid in which a partial fragment of SV40 large T antigen gene was inserted under control of hTERT promoter (Figure 2C).

The partial fragment (SEQ ID NO: 21) of the SV40 large T antigen gene was inserted downstream of the hTERT promoter inserted in the pTHTK backbone to prepare pTHTN. Simian virus 40 genome sequence comprising the SV40 large T antigen gene is available under Genbank accession No. NC-001669. The pTHTN comprises: the nucleotide sequence of SEQ ID NO: 3 consisting of the hTERT promoter sequence (SEQ ID NO: 1) and the 5' portion (SEQ ID NO: 2) of hTERT transcribed region containing the first exon and a part of the second exon of the hTERT gene; and the partial fragment (SEQ ID NO: 21; from nucleotide 5175 (*Hind* III) to nucleotide 4863 (*Hae* III) of DNA sequence of GenBank accession number: NC_001669) of the SV40 large T antigen gene.

(c) pRBL001

The pRBL001 is a plasmid (plasmid for producing recombinant lentiviral particle THTD) in which DNA encoding CDC6 shRNA was inserted under control of hTERT promoter (Figure 3B).

The sequences of synthetic oligonucleotides used to construct the DNA encoding CDC6 shRNA are shown below.

Cdc6-5-A: 5'-GATCCCCAGGCACTTGCTACCAGCAATTCAAGAGATTGCTGGTAGCAAGTGCCTT TTTTGGAAA-3' (SEQ ID NO: 8)

Cdc6-3-A: 5'-AGCTTTTCCAAAAAAGGCACTTGCTACCAGCAATCTCTTGAATTGCTGGTAGCAA GTGCCTGGG-3' (SEQ ID NO: 9)

For construction of pRBL001, the synthetic oligonucleotides Cdc6-5-A and Cdc6-3-A were mixed in equimolar amounts, denatured, and re-annealed to prepare a double-stranded oligonucleotide. This double-stranded oligonucleotide was blunt-ended and then inserted to the *Eco*R V site of subcloning vector pBlueScript. DNA sequencing was performed to confirm that the double-stranded CDC6 oligonucleotide was inserted in the correct orientation to have the correct sequence. The resulting subclone was linearized at *Xba* I site, blunt-ended, and modified by the ligation of the *Mlu* I linker described above. A DNA fragment comprising the hTERT promoter sequence was obtained by the digestion of the pGL3-1375 described above with *Mlu* I and *Bgl* II and cloned into the *Mlu* I and *Bam*H I sites of the subcloning vector carrying the double-stranded CDC6 oligonucleotide. A DNA fragment containing the hTERT promoter and the double-stranded CDC6 oligonucleotide was excised from the resulting subclone by *Mlu* I and *Xho* I double digestion. The resulting fragment was purified and inserted to the pTHTK backbone that was cleaved with *Mlu* I and *Xho* I and gel-purified, to prepare pRBL001.

The pRBL001 comprises: the nucleotide sequence of SEQ ID NO: 3 consisting of the hTERT promoter sequence (SEQ ID NO: 1) and the 5' portion (SEQ ID NO: 2) of hTERT transcribed region containing the first exon and a part of the second exon of the hTERT gene; and the CDC6 shRNA-encoding sequence (SEQ ID NO: 10). The CDC6 shRNA comprises: a CDC6 shRNA antisense sequence (SEQ ID NO: 19); a linker (SEQ ID NO: 20); and a sense sequence consisting of a sequence complementary to the antisense sequence and a 3' poly U overhang of 5 bases.

Human CDC6 mRNA-targeting shRNA (CDC6 shRNA) produced via the transcription of pRBL001 can specifically cause CDC6 mRNA degradation via RNA interference (RNAi) to result in the knockdown of DNA replication initiator CDC6 protein. Human CDC6 mRNA sequence is available under Genbank accession No. NM_001254. The nucleotide sequence of human CDC6 CDS is shown in SEQ ID NO: 7. A guide strand from the CDC6 shRNA antisense sequence of SEQ ID NO: 19 binds to CDC6 mRNA to cause RNAi.

(d) pTHTH

pTHTH is a plasmid in which a partial fragment of SV40 large T antigen gene was inserted under control of hTERT promoter that lacks 5' portion (1 kb) (Figure 4E).

To construct pTHTH, the plasmid pGL-378 (kindly provided by Dr. Satoru Kyo, Kanazawa University, Japan) carrying the shortened hTERT promoter sequence with the 5' deletion (1 kb) and luciferase gene was cleaved with *Mlu* I plus *Hind* III to obtain a DNA fragment comprising the shortened hTERT promoter sequence that lacks the 5' portion (1 kb). The DNA fragment was purified and incorporated into the plasmid pBluescript at the *Mlu* I and *Hind* III sites to generate plasmid pEND-HTPs. The partial fragment (SEQ ID NO: 21) of SV40 large T antigen gene was excised from a plasmid carrying the SV40 genome DNA with *Hind* III and *Hae* III and inserted into pEND-HTPs at the *Hind* III and *Hinc* II sites. The resulting plasmid subclone was then cleaved with *Mlu* I and *Xho* I, and the excised DNA fragment comprising the shortened hTERT promoter sequence that lacks the 5' portion (1 kb) and the partial fragment of SV40 large T antigen gene was purified and inserted into pTHTK at *Mlu* I and *Xho* I sites to generate pTHTH.

The pTHTH comprises: a nucleotide sequence consisting of the hTERT promoter sequence lacking the 5' portion (1 kb) and the 5' portion (SEQ ID NO: 2) of hTERT transcribed region containing the first exon and a part of the second exon of the hTERT gene; and the partial fragment (SEQ ID NO: 21) of the SV40 large T antigen gene.

(e) pTHTC

The pTHTC is a plasmid in which a partial fragment of SV40 large T antigen gene was inserted under control of a human cytomegalovirus (CMV) promoter (Figure 4F).

The CMV promoter was inserted to the pTHTK backbone. The partial fragment (SEQ ID NO: 21) of the SV40 large T antigen gene was inserted downstream of the CMV promoter in the pTHTK to prepare pTHTC.

Example 2 Preparation of pseudotyped recombinant lentiviral particle

The recombinant lentiviral plasmid vectors deficient in HIV-1 env gene described in Example 1 cannot produce infectious viral particles in themselves. Thus, in this Example, recombinant lentiviral particles (pseudotyped) were prepared via the cotransfection of human embryonic kidney 293T cells with each recombinant lentiviral plasmid vector described in Example 1 and plasmid pCMV-VSV/G expressing the envelope glycoprotein G of vesicular stomatitis virus (VSV). The pCMV-VSV/G was kindly provided by Dr. Sam Chow, the Department of Molecular and Medical Pharmacology of University of California Los Angeles (UCLA). Specific experimental procedures are as described below.

1. Plasmid DNA transfection

15 ml of DMEM/high-glucose (Hyclone, Utah, USA) complete medium (supplemented with 10% fetal bovine serum, 100 units/ml penicillin and 100 μg/ml streptomycin (Hyclone, Utah, USA)) was added to T75 flask, to which human embryonic kidney 293T cells were then inoculated. The 293T cells were proliferated in 5% $CO_2$ incubator at 37°C and increased to cultures in ten T75 flasks while subcultured.

2 x HEPBS (100 ml) was prepared as follows: 1 g of Hepes (acidic salt), 1.6 g of NaCl, 0.75 ml of $Na_2HPO_4$ (0.25 M) and 1 ml of KCl (1 M) were dissolved in an appropriate amount of $ddH_2O$. The pH of the solution was adjusted to 6.9 using NaOH (5 M) and then finely adjusted to 7.12 to 7.14 using NaOH (1 M). $ddH_2O$ was added to this solution up to 100 ml in total and the resulting solution was passed through a syringe filter with 0.22 μm pore size to prepare 2 x HEPBS.

The followings were added to 50-ml tube:

(a) 500 μl of plasmid DNA solution containing 170 μg of each recombinant lentiviral plasmid vector deficient in HIV-1 env gene described in Example 1 and 30 μg of pCMV-VSV/G,
(b) 1,650 μl of $ddH_2O$, and
(c) 350 μl of 2 M $CaCl_2$.

The solution was gently mixed and then 2,500 μl of 2 x HEPBS was added dropwise thereto with gentle stirring to circumvent the formation of large precipitates, thereby preparing a transfection mixture.

The tube containing the transfection mixture was left at room temperature for 20 minutes. The medium was discarded from the ten T75 flasks in which the 293T cells were proliferated. Then, 12 ml of fresh DMEM/high-glucose complete medium was added to each flask. 500 μl of the transfection mixture was gradually added to each flask. The cells were incubated in 5% $CO_2$ incubator at 37°C for 8 hours.

2. Cell culture and collection of medium

The medium was discarded and 15 ml of fresh DMEM/high-glucose complete medium was added to each flask. The cells were incubated in 5% $CO_2$ incubator at 37°C for 36 hours and the medium (medium after 36 hours) was collected. 15 ml of fresh DMEM/high-glucose complete medium was added to each flask. The cells were incubated in 5% $CO_2$ incubator at 37°C for additional 36 hours and the medium (medium after 72 hours) was collected. 15 ml of fresh DMEM/high-glucose complete medium was added to each flask. The cells were further incubated in 5% $CO_2$ incubator at 37°C for 24 hours and the medium (medium after 96 hours) was collected. Each 50-ml tube containing the medium collected was centrifuged at 3,000 rpm and 4°C for 5 minutes.

3. Titeration

200 μl of the medium supernatant was transferred to another tube and diluted 50- to 200-fold by the addition of 1 x PBS. This medium supernatant diluted was assayed for the amount of virus-derived protein p24 released into the medium from the cells infected by recombinant lentiviral particles using HIV-1 p24 antigen ELISA assay kit (Coulter Inc., Miami, FL, USA), thereby determine the titers of the viral particles.

4. Purification

The medium supernatant collected by centrifugation in "2. Cell culture and collection of medium" described above was transferred to 250-ml high-speed centrifuge tube and centrifuged at 9,000 x g and 4°C for 60 minutes. The resulting supernatant was concentrated 5-fold by ultrafiltration using Vivaspin$^{(R)}$ 20 (1,000 kDa molecular weight cutoff (mw. co.)) (Sartorius, NY, USA). MMP solution was added to the resulting solution and viral particles were precipitated overnight at 4°C. The MMP solution (300 ml) was prepared by: dissolving 90 g of PEG 8,000 (molecular biological grade) and 30 ml of NaCl (UltraPure) (5 M) in an appropriate amount of $ddH_2O$; and adding $ddH_2O$ to the solution up to 300 ml in total.

The solution in which viral particles were precipitated was centrifuged at 9,000 x g and 4°C for 30 minutes to form pellet containing the viral particles. The supernatant was discarded and the pellet was resuspended in 10 ml of 1 x PBS to obtain a solution containing the viral particles.

The viral particles in an aliquot of the solution were PEGylated (Croyle et al., J. Virol., 2004, Vol. 78, p. 912-921). The PEGylation was performed by adding 0.4 ml of PEGylation solution (33 mg/mL methoxy PEG succinimidyl carbonate NHS (mPEG-NHS, m.w. 10K (NANOCS, USA)), 30 mM HEPES-KOH, pH 7.5, 500 mM NaCl) to approximately 10 ml of the solution containing the viral particles and incubating the mixture at room temperature for 60 minutes on a rotary platform.

The solution containing the viral particles PEGylated or unPEGylated was dialyzed against 1 x PBS at 4°C using Slide-A-Lyzer cassette (20 kDa mw. co.) (Thermo Scientific, IL, USA) and 1 x PBS was replaced with fresh one every 24 hours for 2 days. The viral particle solution thus dialyzed was concentrated 30-fold using AmiconUltra 15 (100 kDa mw. co.) (Millipore, MA, USA). This viral particle solution concentrated was passed through a syringe filter with 0.45 μm pore size and the resulting preparation was stored at -80°C.

In this way, the pseudotyped recombinant lentiviral particles were successfully prepared. The pseudotyped recombinant lentiviral particles prepared from the recombinant lentiviral plasmid vectors pTHTK, pRBL0213T, pTHTN, pRBL001, pTHTH and pTHTC described in Example 1 are referred to as THTK, RBL0213T, THTN, THTD, THTH and THTC, respectively, in subsequent Examples.

The recombinant lentiviral particles thus prepared can infect various human proliferating cells and non-dividing and dividing cells as described later. Target cells infected by the recombinant lentiviral particles synthesize lentiviral DNA, which is in turn integrated into host chromosomal DNA. Thus, the resulting recombinant lentiviral particles can be used as lentiviral vectors.

Example 3 Study on ability of recombinant lentiviral particle to produce infectious viral particle

On the basis of the detection of LTR-Tag formed by ligation-mediated PCR (LM-PCR), the pTHTK-derived pseudotyped recombinant viral particle THTK was examined for its ability to produce infectious viral particles after infection of cells.

1. Preparation of *Bpm* I site in pTHTK

In order to carry out LM-PCR, pTHTK was first modified to prepare new *Bpm* I site in HIV-1 genome (Figure 5A). HIV-1 5' LTR region has three elements (U3, R and U5). The *Bpm* I site was prepared via overlap PCR at the 3' end of the U5 element of 5' LTR in pTHTK. *Bpm* I is a class II S restriction enzyme. *Bpm* I recognizes 5'-CTGGAG-3'. *Bpm* I cleaves DNA at 16 nucleotides 3' of its recognition site in a strand comprising this 5'-CTGGAG-3' and at 14 nucleotides 5' of its recognition site in another strand to produce a 3' overhang of 2 nucleotides (Figure 5B).

Figure 5C shows the terminal structure of lentiviral genome. Lentiviral RNA is produced by transcription from the 5'LTR R element to the 3' LTR R element of proviral DNA. Accordingly, the lentiviral RNA has neither the 5' LTR U3 element nor the 3' LTR U5 element at the ends. However, when the lentiviral RNA is reverse transcribed into lentiviral DNA, the 3' LTR U3 element and the 5' LTR U5 element are copied to the 5' LTR U3 element and the 3' LTR U5 element, respectively. Then, this lentiviral DNA is integrated into host genome to form proviral DNA.

Thus, the new *Bpm* I site in the 5' LTR U5 element produced in the HIV-1 genome of pTHTK is copied to the 3' LTR U5 element when lentiviral DNA is synthesized in infected cells. This new *Bpm* I site has no influence on THTK virion assembly, viral particle infection and proviral DNA integration in infected cells. Host cells are infected by the viral particle THTK derived from pTHTK having this new *Bpm* I site so that proviral DNA having the *Bpm* I site copied at a position distant by 2 nucleotides (5'-CA-3') from the end in the 3' LTR U5 element is integrated into host genome. Thus, host genomic DNA from the infected cells is cleaved at a position distant by 14 nucleotides from the integration site of the proviral DNA as a result of *Bpm* I treatment (Figure 5B).

2. Preparation of LTR-Tag

Human uterine cervix cancer HeLa cells expressing HIV-1 Tat protein (HeLa/tat cells) were infected by coincubation with the viral particle THTK prepared as described in Example 2 from the pTHTK having the new *Bpm* I site described above. The HeLa/tat cells were kindly provided by Dr. Sam Chow, the Department of Molecular and Medical Pharmacology of University of California Los Angeles (UCLA). 3, 5, 10 and 15 days after the infection, host genomic DNA was isolated from the HeLa/tat cells. The genomic DNA was subjected to *Kas* I and *Xho* I double digestion. Each of *Kas* I (positions 637 to 642; based on SEQ ID NO: 4; the same holds true for the description below) and *Xho* I (positions 8887 to 8892) is a unique site in HIV-1 genome. The *Kas* I site is located 4 nucleotides downstream of 5' LTR and the *Xho* I site is located approximately 190 nucleotides upstream of 3' LTR. The *Kas* I and *Xho* I double digestion separates proviral DNA from the host genomic DNA while the 5' LTR and 3' LTR sequences of the proviral DNA remain with the host genomic DNA. Further, the *Kas* I recognition site is located between the *Bpm* I recognition site (positions 627 to 632) prepared in 5' LTR and the *Bpm* I cleavage site at 16 nucleotides downstream thereof. Therefore, even *Kas* I digestion followed by *Bpm* I digestion does not result in *Bpm* I digestion based on the *Bpm* I site in 5' LTR.

Next, special DNA extension reaction shown below was performed. A primer derived from a sequence of approximately 100 nucleotides upstream of 3' LTR is used in this reaction. Therefore, the 3' LTR of the proviral DNA and the host genomic DNA downstream thereof are amplified markedly while the ratio of contaminating DNA, specifically, DNA derived from the more upstream proviral DNA drastically decreases. The nucleotide sequence of the primer B-NLR8950 for DNA extension is shown below.

B-NLR8950: 5'-B-GTGCCTGGCTAGAAGCACAAG-3' (SEQ ID NO: 11)

This sequence has biotin-labeled nucleotides and is derived from a sequence from positions 8950 to 8970 in the genomic DNA of the HIV-1 NL4-3 strain. This primer was used to perform the DNA extension reaction as shown below.

DNA extension reaction solution:

| | |
|---|---|
| B-NLR8950 | 50 μM |

(continued)

| | |
|---|---|
| *Kas* I + *Xho* I-digested host genomic DNA | 10 μg |
| dNTPs | 200 μM |
| Taq DNA polymerase | 5 units |

The tube containing the DNA extension reaction solution described above was incubated at 94°C for 5 minutes, at 55°C for 5 minutes and at 72°C for 30 minutes for DNA extension reaction to obtain biotinylated DNA.

This biotinylated DNA was purified as follows via binding to streptavidin-magnetic beads (Dynabeads M-280): approximately 5 pmol of the biotinylated DNA was mixed with 40 pmol of Dynabeads M-280 in a tube and the mixture was incubated at room temperature for 30 minutes. The tube was placed on a magnetic stand (Dynal MPC stand) and left for 2 minutes. Then, the beads were washed with TE (pH 8.0) buffer. The washing of the beads was performed using 1 ml of TE and further repeated two times. The beads were centrifuged for 2 seconds and the supernatant was discarded to purify the biotinylated DNA.

Then, the biotinylated DNA was subjected to *Bpm* I digestion. The biotinylated DNA was digested with *Bpm* I overnight at 37°C. The biotinylated DNA digested was purified via Dynabeads M-280 and then ligated with a double-stranded oligo linker. One end of the double-stranded oligo linker has a 3' overhang of 2 nucleotides and is complementary to the *Bpm* I-digested end. The other end of the double-stranded oligo linker has a 5' overhang of 3 nucleotides and efficiently prevents the self-ligation of the linker. The nucleotide sequences of oligonucleotides HD-A and HD-S (provided by Dr. Sam Chow, University of California, UCLA) constituting the double-stranded linker are shown below.

HD-A: 5'-CACGCGTCGCATCATATCTCCAGGTGTGACAG-3' (SEQ ID NO: 12)
HD-S: 5'-CCTCTGTCACACCTGGAGATATGATGCGACGCGTGNN-3' (SEQ ID NO: 13)

The 3'-terminal "NN" of HD-S represents a degenerate sequence indicated by any combination of "A", "T", "G" and "C."

The ligation of the biotinylated DNA with the double-stranded oligo linker was performed at room temperature for 16 hours. The biotinylated DNA linked to the double-stranded oligo linker was purified via Dynabeads M-280.

3. Detection of LTR-Tag

In order to detect LTR-Tag, PCR amplification was performed. The following primers were used in the PCR reaction:

Forward primer BHU5-S2: 5'-GAGTGCTCAAAGTAGTGGT-3' (SEQ ID NO: 14)
Reverse primer HDA/SBOT: 5'-CTGTCACACCTGGAGATATGAT-3' (SEQ ID NO: 15)

The nucleotide sequences of the forward primer and the reverse primer are derived from positions 9617 to 9636 of the HIV-1 genomic DNA and one strand HD-S of the double-stranded oligo linker, respectively.

The following PCR reaction was performed: 1 cycle involving 94°C for 4 minutes, and 30 cycles each involving 94°C for 60 seconds, 60°C for 30 seconds and 72°C for 90 seconds, followed by 72°C for 10 minutes and 4°C for 1 to 12 hours. The biotinylated DNA linked to the double-stranded oligo linker described above was used as template DNA in the PCR reaction.

After PCR, 5 μl of each reaction product was loaded onto a gel (2.0% agarose) and electrophoresed in 1 x TAE. Approximately 138-bp PCR product (LTR-Tag) was detected provided that the proviral DNA was integrated into the host cell genome (Figure 6).

4. Results

The results are shown in Figure 7. The detected LTR-Tag (LTR-Tag positive) indicates that the proviral DNA was integrated into the host cell genome. This means that the host cells were infected by the virus. The pseudotyped recombinant lentiviral particle THTK exhibited LTR-Tag positive and was shown to successfully infect the cells (Figure 7, After infection).

Next, the culture medium of the infected cells was collected, filtered through a filter with 0.45 μm pore size, and added to fresh cultured cells ("reinfection"). Genomic DNA was isolated from these cells and examined for LTR-Tag. As a result, the viral particle exhibited LTR-Tag negative, indicating that the cells were not infected by the viral particle (Figure 7, After "reinfection"). These results demonstrated that the cells infected by the recombinant lentiviral particle THTK did not produce infectious viral particles. LM-PCR to test whether the recombinant lentiviral particle was unable to replicate in the infected cells was accurate and ended in perfect reproducibility without false positive results, i.e., contamination by the direct PCR of the HIV-1 sequence.

The results described above demonstrated that the pseudotyped recombinant lentiviral particle THTK can infect cells, but the infected cells do not produce infectious viral particles. This indicates that the method for producing microvesicles according to the present invention using the pseudotyped recombinant lentiviral particle as the lentiviral vector does not produce infectious viral particles.

Example 4 Direct injection of recombinant lentiviral particle THTD to human tumor transplanted in nude mouse

An animal experiment was conducted to directly inject the pseudotyped recombinant lentiviral particle THTD prepared as described in Example 2 to human breast cancer Bcap-37 tumor transplanted in nude mouse. THTD (protein content: 275 $\mu$g) was administered by injection to a plurality of sites in the tumor lesion of the animal twice a week for 3 weeks. 48 hours after the final administration, the animal was euthanized by cervical dislocation. For each animal in a control group, 1 x PBS was injected to the tumor lesion. The length and width of each tumor were measured twice a week using a standard caliper after the injection. After the euthanasia of the animal by cervical dislocation, its tumor was isolated, measured, and treated for pathological examination.

The proliferation of the THTD-treated tumor was inhibited by 34.70% relative to the control group and the average weight of the tumor was significantly lower than that of the control ($p < 0.02$). The injection of THTD developed strong fibrosis in tumor tissue, thereby suppressing tumor proliferation.

The results described above demonstrated that the pseudotyped recombinant lentiviral particle THTD infects mouse cells and then CDC6 shRNA encoded by the viral genome was produced in the cells to suppress the expression of CDC6, resulting in the suppressed proliferation of tumor. This indicates the possibility that the microvesicle of the present invention comprising CDC6 shRNA also has tumor proliferation suppressive effect.

Example 5 Enhancement of viral genomic RNA splicing by hTERT promoter

The gene transcription and gene transduction activities of the recombinant viral particle THTN prepared as described in Example 2 were compared with those of other recombinant lentiviral particles and examined by detecting RNA splicing activity by RT-PCR (Figure 8).

1. Viral infection and collection of cell

Human uterine cervix cancer HeLa cells expressing HIV-1 Tat protein (HeLa/tat cells) were inoculated at a concentration of approximately 2 x $10^5$ cells/well into a 6-well plate and 3 ml/well of DMEM/high-glucose complete medium was added thereto. The cells were proliferated in an incubator at 37°C and 5% $CO_2$ for 24 hours until becoming approximately 80% confluent in observation under inverted microscope. The pseudotyped recombinant lentiviral particle THTN, THTH or THTC prepared as described in Example 2, or viral particle NL4-3 or D64V prepared by the transfection of human embryonic kidney 293T cells with pNL4-3 or pD64V was added to the wells (two wells for each lentiviral particle) in which the HeLa/tat cells were proliferated, to infect the cells by each virus. A virus solution having approximately 4 x $10^5$ infection units equivalent to 400 ng of p24 viral protein was used per 2 x $10^5$ target cells in each infection (multiplicity of infection (m.o.i.): 1.3).

On the other hand, Mock infection was performed by the incubation of cells with "dead" THTN virions. The THTN virions were prepared by boiling at 100°C for 5 minutes to make sure that the virus is "dead." The Mock infection with the "dead" virus guarantees that there is absolutely no viral particle entry into the cell, so there is no viral RNA transcribed in the RT-PCR assay.

The cells thus infected were proliferated in 5% $CO_2$ incubator at 37°C for 8 hours. The cells were observed under microscope to confirm that the cells were healthy and were not contaminated. The medium was carefully discarded and 3 ml/well of fresh DMEM/high-glucose complete medium was added to the cells. The cells were proliferated in an incubator at 37°C and 5% $CO_2$ for 48 hours. Then, the cells were rinsed three times using 1 x PBS and scraped off from the surface of the wells using Rubber Policeman to collect the cells. The cells were collected into 15-ml low-speed centrifuge tube, to which 1 x PBS was then added up to 12 ml in total. The tube was centrifuged at 3,000 rpm to form cell pellet. The supernatant was discarded and the cell pellet was frozen in dry ice for 30 minutes.

2. RNA extraction

GTC buffer was prepared as follows: 212 g of guanidine thiocyanate (mw. 118.16), 2.2 g of sodium citrate (mw. 294.1) and 15 ml of sarkosyl (10% w/v) were dissolved in an appropriate amount of $ddH_2O$ and $ddH_2O$ was added to

the solution up to 300 ml in total (final concentrations: 6 M, 25 mM and 0.5%, respectively). 1.4 ml of β-mercaptoethanol was added per 20 ml before use to prepare GTC buffer.

1,000 μl of the GTC buffer was added to the cell pellet in each tube. The cells were passed through 18 G 1/2 needle five times using a syringe to homogenize the cells.

Then, 1,000 μl of water-saturated phenol and 1,000 μl of chloroform were added to each tube and the tube was vortexed. Then, 3 ml of 1 M sodium acetate, pH 5.3 was added to each tube and the tube was vortexed and centrifuged at 9,000 x g and 4°C for 10 minutes. The formed upper layer solution was transferred to fresh 50-ml tube, to which 0.6 to 1.0 volume of isopropanol was then added. The solution was mixed by inverting the tube five times and left overnight at room temperature. The tube was vortexed for 30 seconds. 200 to 300 μl of the solution was transferred to 1.5-ml microtube and centrifuged at 13,000 rpm and 4°C for 15 minutes to form RNA pellet. The supernatant was discarded and the RNA pellet was rinsed with 1 ml of 75% ethanol and centrifuged at 13,000 rpm and 4°C for 5 minutes. The supernatant was discarded and the RNA pellet was dried in air.

3. RT-PCR

The RNA pellet was redissolved in 10 μl of $ddH_2O$. Reverse transcription reaction and PCR (RT-PCR) were performed for cDNA amplification. In this RT-PCR, 200 μg of RNA was used for each reverse transcription.

The reverse transcription (RT) reaction was performed as follows: 10 μl of DNase I-digested RNA (approximately 200 μg), 1 μl of 10 mM dATP/dCTP/dGTP/dTTP stock, 1 μl of RNasein$^{(R)}$ (30 units/μl, Promega), 2 μl of 10 x PCR buffer, 1 μl of 0.1 μg/μl random primer (random hexamer) and 1 μl of 50 mM $MgCl_2$ were gently mixed and then 1 μl of Mo-MuLV reverse transcriptase (200 units/μl) was added thereto and mixed in the tube by centrifugation for 2 seconds. The tube was incubated first at room temperature for 10 minutes and then at 42°C for 60 minutes. Then, the tube was boiled in a water bath of 100°C for 15 minutes and cooled on ice for 5 minutes. The RT reaction solution in the tube was aliquoted to four fresh tubes (in an amount corresponding to 50 μg of RNA for each tube) and stored at -80°C for further use.

Next, the PCR reaction was performed as follows: 2.5 μl of primer 5' LTRU5 (20 μM), 2.5 μl of primer 3' NL5850 (20 μM) or primer 3' NL8960, 5 μl of RT reaction solution and 40 μl of PCR mixture were mixed and subjected to the following temperature conditions using a thermal cycler for PCR reaction (Applied Biosystems): 1 cycle involving 94°C for 4 minutes, and 16 cycles each involving 94°C for 60 seconds, 60°C for 30 seconds and 72°C for 90 seconds, followed by 72°C for 10 minutes.

The nucleotide sequences of the primers used are shown below.

Primer 5' LTRU5: 5'-TCTGGCTAACTAGGGAACCCACTG-3' (SEQ ID NO: 16)
Primer 3' NL5850: 5'-GCTATGTCGACACCCAATTCTGAA-3' (SEQ ID NO: 17)
Primer 3' NL8960: 5'-TGTGCTTCTAGCCAGGCACAAGC-3' (SEQ ID NO: 18)

The PCR mixture (for six samples) was prepared at a final volume of 240 μl by mixing the followings:

| | |
|---|---|
| 10 mM dATP/dCTP/dGTP/dTTP stock | 6 μl |
| $^{32}P$-α-dCTP (3000 Ci/mmol, 10 μCi/μl, GE, USA) | 1 μl |
| 10 x PCR buffer | 30 μl |
| Taq DNA polymerase (5 units/μl) | 3 μl |
| $ddH_2O$ | 201 μl |

After PCR, 3 μl of DNA loading buffer was added to 20 μl of each PCR reaction product. The mixture was loaded onto 2.2% agarose gel and electrophoresed in 1 x TAE. The gel was wrapped, dried, and exposed to an X-ray film overnight at -80°C. The X-ray film was developed and photographs were taken. The 1 x TAE was prepared by: dissolving 242 g of Tris Base (molecular biological grade), 57.1 ml of glacial acetic acid (molecular biological grade) and 100 ml of 0.5 M EDTA, pH 8.0 (molecular biological grade) in an appropriate amount of $ddH_2O$; adding $ddH_2O$ to the solution up to 1,000 ml in total; and diluting 50 x TAE thus prepared 50-fold with $ddH_2O$.

In the HeLa/tat cells infected by different recombinant lentiviral particles, splicing of lentiviral RNAs derived from these particles was compared. The viral particle D64V deficient in HIV-1 integrase and thus deficient in HIV-1 proviral DNA integration was used as a negative control.

4. Results

The results of PCR using the primers 5' LTRU5 and 3' NL5850 are shown in Figure 8B. HIV-1 has seven major spliced RNA fragments bound to form various viral mRNAs. As a result of using the primers 5' LTRU5 and 3' NL5850, major PCR products of five out of these fragments were detected in all the tested cells infected by the lentiviral particles. Particularly, the cells infected by the viral particle THTN comprising the hTERT promoter in the viral genomic RNA exhibited very high RNA splicing activity, which was at least 100 times higher than the RNA splicing level of the cells infected by the wild-type NL4-3. By contrast, the RNA splicing level was not increased in the cells infected by the recombinant lentiviral particle THTH (lacking the 5' portion (1 kb) of the hTERT promoter) or THTC (having the CMV promoter), compared with the cells infected by the wild-type viral particle NL4-3.

The results of PCR using the primers 5' LTRU5 and 3' NL8960 are shown in Figure 8A. The PCR amplification level obtained using the primers 5' LTRU5 and 3' NL8960 represents the amount of viral genomic RNA derived from each lentiviral particle. However, PCR using the primers 5' LTRU5 and 3' NL8960 failed to efficiently produce PCR products and exhibited very weak signals. The cells infected by the viral particle THTN comprising the hTERT promoter in the viral genomic RNA had a viral genomic RNA level equivalent to that of the cells infected by other viral particles (THTH, THTC or NL4-3).

The results described above demonstrated that lentiviral RNA splicing is enhanced in the cells infected by the pseudotyped recombinant lentiviral particle carrying the lentiviral RNA comprising the human telomerase reverse transcriptase (hTERT) promoter, thereby enhancing transgene expression.

Example 6 Enhancement of gene transduction and expression by hTERT promoter

The effect of hTERT promoter on gene transduction activity was examined by p24 ELISA assay using viral protein expression as an indicator.

293T cells and HeLa/tat cells inoculated at a concentration of 2 x $10^6$ cells were infected (multiplicity of infection (m.o.i.): 1.0) by the pseudotyped recombinant lentiviral particle THTN, THTH or THTC prepared in Example 2, or the viral particle NL4-3 or D64V. Upon infection, virus genomic DNA is synthesized from viral genomic RNA in the lentiviral particle and integrated as proviral genomic DNA into host cell genome. Proteins, for example, p24 viral antigen, expressed as a result of the gene integration (gene transduction) into host genome can be secreted into a culture medium. Thus, the medium was collected and the amount of the virus-derived protein p24 in the medium was measured using HIV-1 p24 antigen ELISA assay kit (Coulter Inc., Miami, FL, USA) to examine the gene transduction activity of each viral particle.

First, the cell culture medium was collected at different points of times after the infection. Each medium collected was centrifuged to remove cell debris and then was diluted with 1 x PBS buffer and subjected to p24 assay. The dilution ratio was usually set to 1:50 to 1:200. As a result, the p24 level was elevated in approximately 36 hours after the infection, reached a peak at 72 hours after the infection, and rapidly declined at 96 hours after the infection. Thus, the p24 level was indicated by an average in the medium collected 72 hours after the infection.

The results are shown in Figure 9. The 293T cells infected by the viral particle THTC comprising the CMV promoter in the viral genomic RNA exhibited a p24 level higher than that of the 293T cells infected by the wild-type NL4-3. This indicates that the CMV promoter activates gene transduction and expression in the 293T cells. Such activation of gene transduction and expression was more apparent as to the viral particle THTN, which comprised the hTERT promoter in the viral genomic RNA and exhibited active RNA splicing as shown in Example 5. The 293T cells infected by THTN had a p24 level exceeding that of the 293T cells infected by THTC. By contrast, the 293T cells infected by the viral particle THTH lacking the 5' portion (1 kb) of the hTERT promoter sequence had a p24 level equivalent to that of the cells infected by the wild-type NL4-3. Moreover, p24 was hardly detected as to D64V, which does not cause gene transduction, demonstrating that the p24 level reflects gene transduction and expression levels.

Next referring to the HeLa/tat cells, the cells infected by THTC or THTH produced p24 at a level equivalent to that in the case of the wild-type NL4-3. Only the HeLa/tat cells infected by the viral particle THTN comprising the hTERT promoter in the viral genomic RNA had a p24 level exceeding 2,500 ng/ml, which was obviously higher than that in the case of other viral particles.

The results described above demonstrated that gene transduction and expression are enhanced by the hTERT promoter, as is evident from the fact that viral protein expression was enhanced in the cells infected by the pseudotyped recombinant lentiviral particle carrying the lentiviral RNA comprising the hTERT promoter.

Example 7 Enhancement of microvesicle release based on hTERT promoter

Cells into which the lentiviral plasmid vector pRBL0213T was introduced or cells infected by the viral particle RBL0213T were examined for their microvesicle (mv) release using, as an indicator, the activity value of transgene PTEN in the cells or in mv determined by PTEN assay.

1. Plasmid DNA transfection

200 μl of 1 μg/ml pRBL0213T and 50 μl of 3 M sodium acetate, pH 5.3 were added to 1.5-ml microtube, to which 1 ml of 100% ethanol was then added. The solution was mixed and cooled at -80°C for 60 minutes. Then, the tube was centrifuged at 13,000 rpm for 15 minutes to form DNA pellet. The supernatant was discarded. 1 ml of 70% ethanol was added to the tube, which was then centrifuged at 13,000 rpm for 5 minutes. The supernatant was carefully discarded and the DNA pellet was dried in air. The DNA pellet was dissolved by the addition of 500 μl of $ddH_2O$ to prepare pRBL0213T plasmid DNA solution. The pRBL0213T is a recombinant lentiviral plasmid vector having the PTEN gene under control of the hTERT promoter (Figure 10D, Lenti.).

Also, plasmid DNA solutions of plasmids pGL3-1375, pcDNA3.1/CMV-hPTEN and pRBL016Bn were prepared. The pGL3-1375 (Takakura et al., Cancer Res., 1999, p. 551-557) had hTERT promoter but no PTEN gene, and was used as a negative control of PTEN assay (Figure 10A, Empt.). The pcDNA3.1/CMV-hPTEN was plasmid in which PTEN gene was inserted under control of CMV promoter of general plasmid pcDNA3.1, and was used as a positive control of PTEN assay (Figure 10B, Regul.). The pRBL016Bn is a Mo-MLV retroviral plasmid having PTEN gene under control of a rat nerve growth factor receptor (rNGFR) gene promoter (Figure 10C, Retro.).

500 μl of any of these four kinds of plasmid DNA solutions (20 μg of plasmid DNA), 400 μl of $ddH_2O$ and 75 μl of 2 M $CaCl_2$ were added to fresh 50-ml tube and gently mixed. Then, 525 μl of 2 x HEPBS was added dropwise thereto with gentle stirring to circumvent the formation of large precipitates. The tube was left at room temperature for 20 minutes to obtain a transfection mixture.

The medium was discarded from T75 flask in which human embryonic kidney 293T cells were proliferated as described in Example 2. Then, 12 ml of fresh DMEM/high-glucose complete medium was added to each flask. 1,000 μl of the transfection mixture was gradually added to each flask. The cells were incubated in 5% $CO_2$ incubator at 37°C for 8 hours to transfect the cells with each plasmid DNA. Then, the medium was discarded and 15 ml of fresh DMEM/high-glucose complete medium was added to each flask. The cells were incubated in 5% $CO_2$ incubator at 37°C for 60 hours. Also, mock transfection was performed by similar procedures using water instead of the plasmid DNA solutions.

2. Infection by viral particle RBL0213T

As described in Example 2, recombinant lentiviral particle RBL0213T was prepared using the plasmid pRBL0213T, then PEGylated, and purified. 293T cells, CEM cells or HeLa cells were infected (m.o.i.: 1.3) by the resulting recombinant lentiviral particle RBL0213T. The cells were infected by the viral particle RBL0213T by incubation for 12 hours. A fresh medium was added thereto and the cells were proliferated for 48 hours.

On the other hand, the chemically inactivated viral particle was prepared by: preparing 100 mM AT-2 (2,2'-dipyridyl disulfide) (aldrithiol-2) in DMSO (Fluka); adding the AT-2 at a concentration of 1 mM to the solution containing the viral particle; and treating the mixture overnight at 4°C (Rossio JL, J. Virol., 1998, Vol. 72, p. 7992-8001). The AT-2 is a reagent that oxidizes cysteine in proteins in virions to inhibit the functions of reverse transcriptase, thereby inactivating the virus. The AT-2 treatment deletes the infectivity of HIV, though the integrity and conformation of viral surface proteins are maintained.

3. Preparation of cell lysate

A solution containing free phosphate is not preferable for PTEN assay because of producing high backgrounds. Buffers and tubes are recommended to be phosphate-free.

After the transfection or the infection described above, the cells were collected into fresh 50-ml tube and centrifuged at 3,000 rpm for 5 minutes to precipitate the cells. For use in "4. Preparation of mv lysate" described later, the medium was transferred to fresh 50-ml tube. The precipitated cells were rinsed with 30 ml of cold 1 x PBS and centrifuged again to precipitate the cells. The supernatant was discarded. 250 μl of lysis buffer (25 mM Tris-HCl, pH 8.0, 150 mM NaCl, 1% NP-40, 1 mM EDTA, 5% glycerol) was added to the cells (2 x $10^6$ cells). The cells were passed through 24 G needle 10 times using a syringe to homogenize the cells, thereby obtaining cell extracts.

The cell extracts were incubated at 4°C for 60 minutes on a rotary platform and centrifuged at 13,000 rpm and 4°C for 20 minutes. The supernatant (cell lysates) was transferred to a fresh tube. 5 μl of the cell lysates was added to another tube. Then, 1 ml of Bio-Rad protein assay solution (Bio-Rad, USA) was added thereto and $OD_{600}$ was measured. Bovine serum albumin was used as a standard to determine a protein concentration. The remaining cell lysates were stored at -80°C for further use.

4. Preparation of mv lysate

The culture medium after the transfection or the infection transferred to the 50-ml tube in "3. Preparation of cell lysate" described above was centrifuged at 9,000 x g and 4°C for 60 minutes. The supernatant was transferred to a fresh tube and ultrafiltered using Vivaspin(R) 20 (1,000 kDa mw. co.) (Sartorius, Bohemia, NY, USA), thereby replacing the buffer with TBS-D. The TBS-D was prepared by: mixing 6.5 ml of 1 M Hepes-KOH, pH 7.6, 7 ml of 5 M NaCl and 0.25 ml of 1 M KCl with an appropriate amount of $ddH_2O$; adding $ddH_2O$ to the mixture up to 250 ml in total; and, to the TBS solution thus prepared, adding 500 μl of 1 M dithiothreitol (DTT) per 50 ml of the TBS solution before use.

To 1 to 2 ml of the solution thus prepared from the medium, the same volume thereas of PEG 8,000/NaCl solution was added and the mixture was incubated overnight at 4°C on a rotary platform. The PEG 8,000/NaCl solution (300 ml) was prepared by: mixing 90 g of PEG 8,000 and 180 ml of 5 M NaCl with an appropriate amount of $ddH_2O$; and adding $ddH_2O$ to the mixture up to 300 ml (final volume).

The solution thus incubated was centrifuged at 13,000 rpm for 20 minutes to form pellet containing microvesicles (mv). The supernatant was discarded and the pellet was resuspended in 65 μl of lysis buffer and incubated at 4°C for 2 hours on a rotary platform to lyse the pellet. This solution was centrifuged at 13,000 rpm for 20 minutes and the supernatant (mv lysates) was transferred to a fresh tube. 5 μl of the mv lysates was added to another tube and $OD_{600}$ was measured to determine a protein concentration. The remaining mv lysates were stored at -80°C for further use.

5. PTEN immunoprecipitation (IP)

70 μl of the cell lysates or the mv lysates (containing 500 μg of the protein) and 5 μl of mouse anti-PTEN monoclonal antibody (clone 6H2.1, 1 mg/ml, Upstate, USA) were added to a fresh tube, to which TBS-D was then added up to 100 μl (final volume) to prepare each IP sample. The IP sample was incubated overnight at 4°C on a rotary platform. Then, 50 μl of prewashed protein A-agarose solution (Thermo Scientific, Illinois, USA) was added to each IP sample and the mixture was incubated overnight at 4°C on a rotary platform. After the incubation, the agarose beads were washed with 1 ml of TBS-D and centrifuged at 6,500 rpm for 5 minutes. The washing was further repeated three times. After the centrifugation, the supernatant was discarded and the agarose beads were resuspended in 75 μl of TBS-D to prepare an agarose bead solution. The solution was stored at 4°C for further use.

6. PTEN assay

PTEN assay was conducted by a partial modification of Echelon PTEN phosphatase malachite green assay (Echelon Biosciences, Utah, USA). For each assay, the final volume was brought up to 100 μl from the original volume of 25 μl and absorbance at 620 nm was measured using Promega GloMax-Multi Jr. reading system (Promega, USA). The assay was conducted using liquid substrate PtdIns(3,4,5)P3 (Echelon, Utah, USA) according to the manual of the manufacturer. For each assay, 23 μl of TBS-D buffer, 70 μl of the agarose bead solution obtained in "5. PTEN immunoprecipitation (IP)" described above and 7 μl of 1 mM PtdIns(3,4,5)P3 stock solution were mixed. The mixture was incubated at 37°C for 2 hours. 450 μl of malachite green solution (Echelon, Utah, USA) of room temperature was added to the mixture. The tube containing the mixture was covered with aluminum foil for protection from light and incubated at room temperature for 30 to 60 minutes. Absorbance at 620 nm was measured using a malachite green solution as a blank and a substrate as a phosphatase background. PTEN activity values were obtained from three different transfections or infections. The activity values of the substrate (background) were subtracted from the obtained values and an average of the resulting values was obtained.

7. Results

The results of PTEN assay on 293T cells transfected with each plasmid are shown in Figure 11. The highest PTEN activity in the cell lysates was confirmed for the cells transfected with the Regul. vector comprising the strong CMV promoter that drove the transcription of the PTEN gene. The PTEN activity was also high in the case of the transfection with the Retro. vector comprising the rNGFR promoter that drove the transcription of the PTEN gene. The transfection with the Lenti. vector comprising the hTERT promoter that drove the transcription of the PTEN gene gave a moderate PTEN activity value, compared with the Regul. and Retro. vectors. As for the mv lysates, the highest PTEN activity was confirmed in the case of the transfection with the Lenti. vector, compared with the Regul. and Retro. vectors.

Figure 12 shows the ratio of the PTEN activity in the mv lysates to the PTEN activity in the cell lysates shown in Figure 11. The transfection with the Lenti. vector gave the highest ratio of the PTEN activity in the mv lysates (27.37%). Moreover, it is to be noted that the much higher ratio of the PTEN activity in the mv lysates was shown from the transfection with the Empt. vector, which comprised the hTERT promoter as in the Lenti. vector for gene transcription and had no PTEN gene, compared with the Regul. and Retro. vectors. These results demonstrated that the transfection with the Empt. vector promotes the mv encapsulation of the endogenous PTEN of host cells whereas the transfection with the Lenti. vector promotes the mv encapsulation of both endogenous and transgene products PTEN, thereby enhancing the release of mv into an extracellular environment.

Further, the results of PTEN assay on 293T cells, HeLa cells and CEM cells infected by the viral particle RBL0213T are shown in Figure 13. The cells incubated with the recombinant lentiviral particle RBL0213T released a larger number of mv than that in the case of transfection, irrespective of whether the particle was infectious or inactive (mock infection) (Figure 13, mv lysates). By contrast, the cell lysates of the cells infected by the recombinant lentiviral particle RBL0213T or mock-infected had PTEN activity substantially equivalent to that in the case of transfection (Figure 13, cell lysates). However, elevated PTEN activity was observed in the lysates of the HeLa cells infected by the viral particle RBL0213T. These results demonstrated that the infection by the recombinant lentiviral particle RBL0213T tends to increase PTEN activity in the mv lysates, unlike in the cell lysates, compared with the transfection with the Lenti. vector pRBL0213T. Further, the infection by the lentiviral particle was shown to cause the human uterine cervix cancer HeLa cells to form and release a larger number of transgene product PTEN-encapsulated mv among the tested cells (Figure 13, mv lysates). As a result of conducting PTEN assay on the solution of the viral particle RBL0213T, the PTEN activity value was very small ($OD_{620}$ = 0.065), demonstrating that PTEN is hardly incorporated in the viral particle.

Figure 14 shows the ratio of the PTEN activity in the mv lysates to the PTEN activity in the cell lysates of the 293T cells transfected with the recombinant lentiviral plasmid vector pRBL0213T or the 293T, CEM or HeLa cells infected by the recombinant lentiviral particle RBL0213T or mock-infected. The infection (Infect.) and the mock infection (Mock) are indicated by values that compensate for the nonspecific stimulation of mv release for the mock infection. The ratio of the PTEN activity in the mv lysates to the PTEN activity in the cell lysates was larger for the infection than for the mock infection, demonstrating that the infection by the recombinant lentiviral particle RBL0213T enhanced mv release from the cells. In contrast to the mv release ratio of 27.37% in the cells transfected with the Lenti. vector (Figure 12), the mv release ratio reached 38.75% in the cells infected by the recombinant lentiviral particle RBL0213T (Figure 14), demonstrating that the infection by the recombinant lentiviral particle RBL0213T further significantly promoted mv release.

The results described above demonstrated that the cells transfected with the recombinant lentiviral plasmid vector comprising the hTERT promoter in the lentiviral RNA intracellularly produce a large amount of microvesicles and exhibit the enhanced release of microvesicles carrying the transgene product. The cells infected by the lentiviral particle prepared using such a plasmid were shown to exhibit the more strongly enhanced microvesicle release.

Example 8 Preparation of microvesicle

The microvesicle (mv) of the present invention was prepared by the method shown below.

1. Plasmid DNA transfection or viral infection

15 ml of DMEM/high-glucose (Hyclone, Utah, USA) complete medium (supplemented with 10% fetal bovine serum, 100 units/ml penicillin and 100 μg/ml streptomycin (Hyclone, Utah, USA)) was added to T75 flask, to which human embryonic kidney 293T cells were then inoculated. The 293T cells were proliferated in 5% $CO_2$ incubator at 37°C and increased to cultures in ten T75 flasks while subcultured. The 293T cells were subjected to plasmid DNA transfection or viral infection shown below.

(A) Plasmid DNA transfection

The followings were added to 50-ml tube:

(a) 500 μl of plasmid DNA solution containing 170 μg of the recombinant lentiviral plasmid vector pTHTN or pRBL001 described in Example 1,
(b) 1,650 μl of $ddH_2O$, and
(c) 350 μl of 2 M $CaCl_2$.

The solution was gently mixed and then 2,500 μl of 2 x HEPBS was added dropwise thereto with gentle stirring to circumvent the formation of large precipitates, thereby preparing a transfection mixture. The tube containing this transfection mixture was left at room temperature for 20 minutes. The medium was discarded from the ten T75 flasks in which the 293T cells were proliferated. Then, 12 ml of fresh DMEM/high-glucose complete medium was added to each flask. 500 μl of the transfection mixture was gradually added to each flask. The cells were incubated in 5% $CO_2$ incubator at 37°C for 8 hours.

(B) Viral infection

The 293T cells were infected by coincubation in 5% $CO_2$ incubator at 37°C for 16 hours with the pseudotyped recombinant lentiviral particle THTN or THTD prepared as described in Example 2 (multiplicity of infection: 0.3-0.4).

2. Cell culture and collection of medium

The medium was discarded and 15 ml of fresh DMEM/high-glucose complete medium was added to each flask. The cells were incubated in 5% $CO_2$ incubator at 37°C for 60 hours and the medium was collected.

3. Purification

50-ml tube containing the medium collected was centrifuged at 3,000 rpm and 4°C for 5 minutes. The medium supernatant thus centrifuged was transferred to 250-ml high-speed centrifuge tube and centrifuged at 9,000 x g and 4°C for 60 minutes. The resulting supernatant was concentrated 5-fold by ultrafiltration using Vivaspin$^{(R)}$ 20 (1,000 kDa molecular weight cutoff (mw. co.)) (Sartorius, NY, USA). MMP solution was added to the resulting solution and mv was precipitated overnight at 4°C.

The solution in which mv was precipitated was centrifuged at 9,000 x g and 4°C for 30 minutes to form pellet containing mv. The supernatant was discarded and the pellet was resuspended in 10 ml of 1 x PBS to obtain a solution containing mv.

mv in an aliquot of the solution was PEGylated (Croyle et al., J. Virol., 2004, Vol. 78, p. 912-921). The PEGylation was performed by adding 0.4 ml of PEGylation solution (33 mg/mL methoxy PEG succinimidyl carbonate NHS (mPEG-NHS, m.w. 10K (NANOCS, USA)), 30 mM HEPES-KOH, pH 7.5, 500 mM NaCl) to approximately 10 ml of the solution containing mv and incubating the mixture at room temperature for 60 minutes on a rotary platform.

The solution containing mv PEGylated or unPEGylated was dialyzed against 1 x PBS at 4°C using Slide-A-Lyzer cassette (20 kDa mw. co.) (Thermo Scientific, IL, USA) and 1 x PBS was replaced with fresh one every 24 hours for 2 days. The mv solution thus dialyzed was concentrated 30-fold using AmiconUltra 15 (100 kDa mw. co.) (Millipore, MA, USA). This mv solution concentrated was passed through a syringe filter with 0.45 μm pore size and the resulting preparation was stored at -80°C.

In this way, microvesicle Lenti-mv2010 carrying the partial fragment of SV40 large T antigen as a transgene product was prepared using the recombinant lentiviral plasmid vector pTHTN or the pseudotyped recombinant lentiviral particle THTN. Further, microvesicle Lenti-mv2010/CDC6 shRNA carrying the transgene product CDC6 shRNA was prepared using the recombinant lentiviral plasmid vector pRBL001 or the pseudotyped recombinant lentiviral particle THTD.

Example 9 Detection of protein contained in microvesicle

In this Example, proteins contained in microvesicles were examined. Also, the microvesicle-mediated delivery of substances into cells was examined.

Human embryonic kidney 293T cell were transfected with plasmids (pcDNA3.1 backbone) expressing N-terminally c-myc-tagged human DNA flap endonuclease 1 (c-myc-FEN-1) to ectopically express c-myc-tagged FEN-1. The FEN-1 has been shown to function as an important cellular helper factor for the maturation of HIV-1 virus genomic DNA and its integration into host genome. The microvesicle Lenti-mv2010 derived from the viral particle THTN-infected cells prepared as described in Example 8 was coincubated with some of the 293T cells transfected with the c-myc-FEN-1 expression plasmid or with untransfected 293T cells. After incubation for 60 hours, nuclear and cytoplasmic extracts were prepared from the cells. Further, the cell culture medium was collected and mv was prepared by the method (except for PEGylation) described in "3. Purification" of Example 8. The resulting nuclear and cytoplasmic extracts and mv were analyzed by Western blot.

The results of detecting Vpu protein using anti-HIV-1 Vpu antibody are shown in Figure 15A. The Vpu protein was detected in the cytoplasmic extracts of the 293T cells that were transfected with the c-myc-FEN-1 expression plasmid and then coincubated with Lenti-mv2010, and in mv released from the cells (Figure 15A, THTN), whereas this protein was not detected in the 293T cells that were transfected with c-myc-FEN-1 expression plasmid but were not coincubated with Lenti-mv2010 (Figure 15A, pcDNA3.1). The Vpu is a protein that suppresses the tetherin- or CD317-mediated attachment of virions to cell membranes, thereby enhancing the release of the HIV-1 virions (Sauter et al., Cell, 2010, Vol. 141, p. 392-398). This showed that: the microvesicle Lenti-mv2010 derived from the 293T cells infected by the recombinant lentiviral particle THTN comprises the viral protein Vpu; the contents were delivered to other cells via the microvesicle; and microvesicles released from the cells that received the delivery also comprised the Vpu protein.

The results of detecting RT protein using anti-HIV-1 reverse transcriptase (RT) antibody are shown in Figure 15B. The RT protein was detected in the microvesicle Lenti-mv2010 derived from the 293T cells infected by the recombinant lentiviral particle THTN (Figure 15B, Lenti-mv2010). By contrast, the RT protein was not detected in mv derived from the 293T cells mock-infected by the viral particle THTN chemically inactivated by AT-2 (Figure 15B, Mock). The RT protein was also detected in the cytoplasmic extracts (Figure 15B, THTN-cytoplasm) of the 293T cells that were transfected with the c-myc-FEN-1 expression plasmid and then incubated with Lenti-mv2010, and in mv (Figure 15B, THTN-mv) prepared from the cells. By contrast, the RT protein was detected neither in mv (Figure 15B, mv/pcDNA3.1) prepared from the 293T cells that were transfected with the c-myc-FEN-1 expression plasmid but were not coincubated with Lenti-mv2010 nor in the untreated 293T cells (Figure 15B, 293T). This showed that: the microvesicle Lenti-mv2010 derived from the 293T cells infected by the recombinant lentiviral particle THTN comprises the viral protein RT; the contents were delivered to other cells via the microvesicle; and microvesicles released from the cells that received the delivery also comprised the RT protein.

The results of detecting endogenous FEN-1 and foreign FEN-1 using anti-FEN-1 antibody are shown in Figure 15C. The endogenous FEN-1 and the foreign FEN-1 (i.e., c-myc-FEN-1) were detected in the nuclei and cytoplasms prepared from the 293T cells that were transfected with the c-myc-FEN-1 expression plasmid and then coincubated with Lenti-mv2010, and in mv released from the cells (Figure 15C, THTN/FEN-1). As for the 293T cells that were coincubated with Lenti-mv2010 but were not transfected with the c-myc-FEN-1 expression plasmid, only the endogenous FEN-1 was detected in the nuclei and cytoplasms and in mv released from the cells (Figure 15C, THTN). This showed that both the cell-endogenous protein (FEN-1) and the foreign protein (c-myc-FEN-1) are encapsulated in the microvesicle.

The results described above demonstrated that viral proteins and other cell-endogenous and foreign proteins are encapsulated in microvesicles, particularly, exosomes, probably with the aid of the viral protein Vpu and a cell-protein transport system called endosomal sorting complexes required for transport (ESCRT), in cells. These results also demonstrated that the contents encapsulated in microvesicles are delivered to other cells.

Example 10 Microvesicle-mediated delivery of substance

The delivery (gene transduction) of substances via purified PEGylated mv was examined using cultured cells.

c-myc-FEN-1-encapsulated PEGylated microvesicle Lenti-mv2010/c-myc-FEN-1 was prepared according to the method for preparing microvesicles by THTN infection described in Example 8 except that the cells used were changed to 293T cells transfected with the c-myc-FEN-1 expression plasmid. Lenti-mv2010/c-myc-FEN-1 was introduced into human uterine cervix cancer HeLa cells by coincubation. The HeLa cells coincubated therewith were subjected to indirect immunofluorescence staining using anti-c-myc antibody.

The results are shown in Figure 16. Figure 16A shows an anti-c-myc antibody stained image. Figure 16B shows a DAPI stained image. Figure 16C shows an overlaid image of Figures 16A and 16B. Figure 16D shows an image prepared from the overlaid image by the color curve program in the image "adjustment" method of Photoshop(R) (Adobe Systems Inc.). In Figure 16D, thicker red color represents increase in c-myc-FEN-1 level and greenish yellow color represents the absence of c-myc-FEN-1. Many red cells expressing c-myc-FEN-1 was seen in Figure 16D, demonstrating that c-myc-FEN1 was delivered to a larger number of cells as a result of the coincubation with Lenti-mv2010/c-myc-FEN-1. The PEGylated microvesicles were shown to be taken up by many cells and be able to deliver the contents encapsulated in the microvesicles to other cells, because of being very stable and active.

Example 11 Suppression of cancer cell proliferation by microvesicle carrying CDC6 shRNA

Microvesicles carrying CDC6 shRNA (Lenti-mv2010/CDC6 shRNA) were tested for their cancer cell proliferation suppressive effect.

Human breast cancer MCF7 cells, human neuroblastoma LA-N-2 cells and neuroblastoma KANR cells were separately incubated with the microvesicle Lenti-mv2010/CDC6 shRNA carrying CDC6 shRNA prepared as described in Example 8. As a control, the microvesicle Lenti-mv2010 carrying the partial fragment of the SV40 large T antigen prepared as described in Example 8 was used. Then, proliferation of the cells was examined by MTT Cell Viability and Proliferation Assay Kit (ScienCell, USA). In this assay, the proliferation of cultured cells was quantified using, as an indicator, the absorbance of a substrate reduced in live cells.

The results are shown in Figure 17. The horizontal axis denotes the number of mv incubated per cell and the vertical axis denotes the ratio (growth index) of the absorbance value obtained to an absorbance value obtained in the absence of mv incubation. The proliferation of the MCF7 cells and the LA-N-2 cells was significantly inhibited by the incubation with Lenti-mv2010/CDC6 shRNA, but was not inhibited by the incubation with Lenti-mv2010 (control) (Figure 17). On the other hand, the proliferation of the KANR cells was not inhibited even by the incubation with Lenti-mv2010/CDC6 shRNA. The MCF7 cells and the LA-N-2 cells exhibited an elevated CDC6 expression, whereas CDC6 expression was hardly detected in the KANR cells. This showed that the CDC6 shRNA contained in the microvesicle knocked down CDC6, resulting in the inhibited proliferation of the cancer cells.

The results described above demonstrated that the microvesicle carrying CDC6 shRNA can deliver the CDC6 shRNA to CDC6-expressing cancer cells, thereby suppressing their proliferation.

Example 12 Molecular mechanism of suppression of cancer cell proliferation by microvesicle carrying CDC6 shRNA

The molecular mechanism underlying the suppression of cancer cell proliferation by Lenti-mv2010/CDC6 shRNA was examined. The previous study has revealed that: a high level of CDC6 protein is related to oncogenic activity in human cancer; and the protein levels of CDC6 and tumor suppressor $p16^{INK4a}$ show an inverse correlation therebetween (Gonzalez, S. et al., Nature, 2006, p. 702-706). The progression of cell cycle of MCF7 cells is not inhibited even in the presence of $p16^{INK4a}$. This indicates the inactivation of the $p16^{INK4a}$-Rb pathway in the cancer cells. However, the $p16^{INK4a}$-Rb pathway may be reactivated in MCF7 cancer cells through Lenti-mv2010/CDC6 shRNA-mediated CDC6 knockdown. Thus, nonradioactive immunoprecipitation kinase assay was conducted to examine the reactivation of the $p16^{INK4a}$-Rb pathway. CDC6 is found in CDK4 kinase complex and required for Rb-C phosphorylation.

CDC6 in the MCF7 cells was removed (knocked down) by incubation with Lenti-mv2010/CDC6 shRNA. As a result, Rb-C phosphorylation was inhibited. The CDC6 knockdown increased the CDK inhibitory activity of $p16^{INK4a}$ by at least 25 times. This showed the reactivation of the $p16^{INK4a}$-Rb pathway.

The results described above demonstrated that the microvesicle carrying CDC6 shRNA knocks down CDC6 in cancer cells and reactivates the $p16^{INK4a}$-Rb pathway, thereby functioning to suppress cancer cell proliferation.

Example 13 Western bloting analysis

In this Example, PTEN protein was detected by Western blotting analysis in protein extracts of cells transfected with PTEN-encoding vectors and extracts of microvesicles obtained by culturing the cells. Plasmid vectors pGL3-1375, pcDNA3.1/CMV-hPTEN, pRBL016Bn and pRBL0213T as desribed in Example 7 and Fig. 10 and lentivirus particle vector RBL0213T as described in Examples 2 and 7 were used as a vector.

1. Preparation of protein extracts

About 1 x $10^6$ 293T cells were transfected with above-mentioned different plasmid vectors. After 60 hours of transfection, cells were harvested, pelleted, homogenized and a supernatant was collected therefrom to prepare cellular protein extracts as described in the section "3. Preparation of cell lysate" of Example 7.

Further, the media after 60 hours of transfection were collected, and microvesicle lysates were prepared therefrom as described in the section "4. Preparation of mv lysate" of Example 7.

In addition, about 1 x $10^6$ 293T cells were infected with lentiviral particle vector RBL0213T with m.o.i. at 0.3 in 25 ml of complete DMEM medium, as described in the section "2. Infection by viral particle RBL0213T" of Example 7. After infection for 60 hours, cellular protein extracts were prepared as described in the section "3. Preparation of cell lysate" of Example 7. Further, the media after infection for 60 hours were collected, and microvesicle lysates were prepared therefrom as described in the section "4. Preparation of mv lysate" of Example 7.

The prepared cellular protein extracts (about 20 micrograms of protein) or microvesicle lysates (about 50 micrograms of protein) were mixed with 2x SDS loading buffer (4% SDS, 250 mM Tris-HCl, pH 6.8, 3% β-mercaptoethanol, 15% glycerol, 0.05% bromophenol blue), and incubated in a boiling water bath for 15 minutes. Then, the protein samples are separated by electrophoresis in a 12% SDS-PAGE gel (Precise™ Protein Gel) using Thermo Scientific Owl Model P82 Minigel Protein Electrophoresis System. After the electrophoresis, the proteins were transferred from the gel to PVDF membrane, and probed with mouse anti-PTEN monoclonal antibody (primary antibody; Clone 6H2.1, MilliPore), followed by goat antimouse secondary antibody (1:5,000 dilution, MilliPore) to detect PTEN protein.

The detection results in the cellular protein extracts (lanes 1 to 5) and the microvesicle lysates (lanes 6 to 10) are shown in Fig. 18. Lanes 1 and 6 indicate cells transfected with pGL3-1375 (Empt.). Lanes 2 and 7 indicate cells transfected with pcDNA3.1/CMV-hPTEN (Regul.). Lanes 3 and 8 indicate cells transfected with pRBL016Bn (Retro.). Lanes 4 and 9 indicate cells transfected with pRBL0213T (Lenti.). Lanes 5 and 10 indicate cells infected with RBL0213T.

Endogenous PTEN was detected (lanes 1 and 6), and transgene product PTEN was detected in the cellular protein extracts (lanes 2 to 5) and microvesicle lysates (lanes 7 to 10). This result indicates that transgene product PTEN was produced, and encapsulated into microvesicles, and the microvesicles (genetically engineered microvesicles) were released from the cells.

The levels of the transgenge product PTEN prepared from cells transfected with Regul., Retro., or Lenti (lanes 2 to 4) were increased by 2 to 3 times compared to that prepared from cells transfected with Empt. (lane 1). Further, the level of the transgege product PTEN prepared from cells infected with RBL0213T lentivirus vector (lane 5) was well above that of endogenous PTEN (lane 1).

In addition, the level of the transgege product PTEN in microvesicle lysates prepared using cells infected with RBL0213T lentivirus vector (lane 10) was clearly increased compared to those of the transgene product PTEN in the microvesicle lysates prepared using cells transfected with Empt., Regul., Retro., or Lenti (lanes 6 to 9). This indicates that the infection with virus particle-like lentivirus vector according to the present invention is more suitable for enhancing production and release of microvesicles that carry transgene products.

Example 14 Inhibitory effect of genetically engineered microvesicles on tumor growth

In this Example, the effect of genetically-engineered microvesicles carrying a tumor-suppressor gene product (herein, also referred to as Cytomox) to inhibit the growth of tumor was examined by directly injecting the microvesicles into tumors.

1. Preparation of test sample

The following test samples were used for intra-tumor injection.

i) Cytomox HD (emvp 130001)

Based on the section "4. Preparation of mv lysate" of Example 7 and Example 8, human embryonic kidney 293T cells were transfected with both of the lentiviral vectors pRBL001 and pRBL0203, and the medium was collected by precipitating cultured cells, and microvesicles were prepared. The microvesicles were called Cytomox HD. Lentivirus vector pRBL001 is described in Example 1, and was used herein as a vector for producing CDC6 shRNA. Lentivirus vector pRBL0203 is a plasmid comprising human $p16^{INK4a}$ gene which has been inserted under control of hTERT promoter in the pTHTK backbone and was produced in a similar way to Example 1. Specifically, pCMV p16INK4a (Plasmid 10916; SEQ ID NO: 25; Medema et al., Proc. Natl. Acad. Sci. U S A., (1995) 92(14):6289-6293), which is the plasmid construct carrying $p16^{INK4a}$ cDNA, was cleaved with *EcoR* I and *Xho* I, and the resulting fragment (p16 cDNA of about 0.45 kb) was subcloned in the *Hind* III-*Xho* I site of pEND-HTPl, in which the 1.5 kb hTERT promoter from pGL3-1375 has been cloned at *Mlu* I-BamH I site. The DNA fragment containing the hTERT promotor and p16INK4a cDNA downstream thereof was cleaved with *Mlu* I and *Xho* I, and the 2.0 kb fragment was inserted into the pTHTK backbone to produce the vector pRBL0203.

ii) Cytomox p53 (emvp 130003)

Based on Example 8, human embryonic kidney 293T cells were transfected with the vector pGLQ-p53EX, and the medium was collected by precipitating cultured cells, and microvesicles were prepared. The vector pGLQ-p53EX is a plasmid expression vector comprising human p53 gene (SEQ ID NO: 26; CDS of GenBank accession no. BC003596). Specifically, subclone vector pBS-TP53PU carrying the human p53 tumor suppressor cDNA of about 2.0 kb that is from the plasmid pBSH19 carrying p53 cDNA was linearized with *Hin*d III at its 3' end. The generated *Hind* III site was blunted, followed by *Sal* I digestion of the vector. The backbone was purified and ligated with an about 0.3 kb DNA fragment (with blunt-ended *Xba* I plus *Sal* I ends) isolated from pGL3-378 to form pBS-TP53PA. The p53 cDNA fragment was cleaved from pBS-TB53PA with *Stu* I to *Sal* I and inserted into pGL3-378 vector at blunt-ended *Xba* I-*Sal* I site to form pGLQ-3UTR. p53 cDNA of about 1.4 kb (containing entire coding region of human p53 gene) was cleaved from another subclone vector pUTK-p53TT with *Hind* III and *Xho* I, and inserted into pGLQ-3UTR to produce pGLQ-p53EX.

iii) Cytomox PTEN (emvp 130006)

Based on the section "4. Preparation of mv lysate" of Example 7 and Example 8, human embryonic kidney 293T cells were transfected with the lentiviral vector pRBL0213T, and the medium was collected by precipitating cultured cells, and microvesicles were prepared. The microvesicles were called Cytomox PTEN. Lentivirus vector pRBL0213T is described in Example 1, and was used herein as a vector for producing human PTEN protein.

iv) Cytomox EX (T 130075)

As described in Example 2, recombinant lentivirus particles RBL001 and RBL0203 were produced by using plasmids pRBL001 and pRBL0203. Based on the section "4. Preparation of mv lysate" of Example 7 and Example 8, human embryonic kidney 293T cells were infected with the lentivirus particle, and the medium was collected by precipitating cultured cells, and microvesicles were prepared. Specifically, the cells were transfected with both of the lentivirus vectors RBL001 and RBL0203 as described in Example 8, and after 60 hours of infection, the medium was collected and the cell debits were removed by centrifugation. The medium was subjected to high speed centrifugation at 9,000 g, 4 °C for 60 minutes. The supernatants were collected for purifying microvesicles as described in Example 8, and the trace amount of pellets were resuspended in 1 x PBS and subjected to dialysis followed by concentration with Amicon Ultra 15 (100 kDa mw. co.) (Millipore). The resulting preparation (microvesicles) was called Cytomox EX.

v) Recombinant interferon α-2b (positive control)

Recombinant interferon α-2b (Schering-Plough (Brinny) Co., Ireland) was used as positive control.

2. Animals

BALB/c-nu mice (female, total eighty animals, grade SPF, 14-16 grams each) were bought from Laboratory Animal Center of Chinese Academy of Medical Sciences for using in the Example.

All of mice were kept for breeding in animal room with an exhaust-air ventilation system. The temperature of the animal facility was kept at 20-25 °C (±< 3 °C) with relative humidity around 40-60%. The animal facility was lighting 12 hours everyday with clean air flow of around grade 100, lighting for work area was around 150-300 LX, for caged animal area was 100-200 LX. Concentration of ammonia was less than 14 mg/$m^3$, noise was less than 60 dB. Food and drinking water (deionized and ultrafiltered) were given freshly everyday to mice.

3. Tumor implantation

Human breast cancer-derived Bcap-37 strain cells were implanted into a BALB/c-nu mouse to make a Bcap-37 tumor-bearing host animal. In a biosafety cabinet under sterilization condition, Bcap-37 tumor was removed from the tumor-bearing host animal, and the tumor tissues were rinsed with 1x PBS. The tumor areas with fine growth without tumor decay were collected, and cut into small pieces of blocks in 2 $mm^3$ with a scalpel. The tumor blocks were rinsed with 1x PBS, and implanted one by one underneath the skin of each mouse's right armpit with a tubing needle. Total 8 groups (ten animals per group) were prepared. Eleven days after implantation, tumors were grown to 110-120 $mm^3$ in size.

4. Injection of micro vesicle (Cytomox)

The microvesicles were injected into the above-mentioned mice having the grown tumor. Intra-tumor injection of microvesicles was performed twice a week for three weeks, for each animal into tumors with injectable solutions containing isolated microvesicles. That is, total six injections were performed.

For both Cytomox HD and Cytomox EX, the first 3 injections were done at a lower dosage ranging from 10.0 to 30.0 μg protein /kg body weight, and the subsequent 3 injections were done at a higher dosage of 1.0 to 3.0 mg protein/kg body weight. The group into which Cytomox HD were injected at 10.0 μg protein /kg body weight and subsequently 1.0 mg protein /kg body weight is herein referred to as low dose Cytomox HD injection group. The group into which Cytomox HD were injected at 30.0 μg protein /kg body weight and subsequently 3.0 mg protein /kg body weight is herein referred to as high dose Cytomox HD injection group. Further, other two groups into which Cytomox EX was injected in a similar manner are herein referred to as low dose Cytomox EX injection group and high dose Cytomox EX injection group respectively. For Cytomox p53 and Cytomox PTEN, the first 3 injections were done at 10.0 μg protein /kg body weight and the subsequent 3 injections were done at a higher dosage of 1.0 mg protein/kg body weight (Cytomox p53 injection group and Cytomox PTEN injection group). As positive control, recombinant interferon α-2b was injected at 250 x $10^4$ IU/kg body weight, twice a week for three weeks (positive control group). As negative control, 1 x PBS was injected to tumors of the tumor-bearing mice twice a week for three weeks (negative control group). Animals in each group were put for euthanasia by cervical dislocation 48 hours after final dosage. The tumors were removed from the dead mice and fixed and processed for pathological examination.

As a result, in this test, there were no acute toxic effects on the testing animals even during high-dose treatments, nor was any animal died due to the injection of genetically-engineered microvesicles (Cytomox).

5. Determination of body weight, tumor weight and tumor size

The body weights of mice were measured prior to test sample injection (tumor-including body weight) and before the euthanasia. Further, the body weights of the dead mice were measured after their tumor were removed from the bodies (net body weights). The weights of the removed tumors were measured.

Based on the measured values of the tumor weights, the efficiency of tumor growth inhibition was calculated as follows.

$$\text{Efficiency of tumor growth inhibition (I) (\%)} = [1- T/C] \times 100$$

T: Average tumor weight (g) in each group injected with each test sample (Cytomox or positive control)

C: Average tumor weight (g) in negative control group

The calculated efficiencies of tumor growth inhibition were shown in Table 1.

Table 1

| Test sample | Dose* (/injection/kg body weight) | Tumor weight (g±SD) | Efficiency of tumor growth inhibition (%) |
|---|---|---|---|
| negative control | - | 1.73±1.11 | |
| IFN α-2b | 250 x $10^4$ IU | 1.45±0.58 | 16.06 |
| Cytomox HD | 3 mg | 1.41±0.66 | 18.37 |
| Cytomox HD | 1 mg | 1.48±0.61 | 14.67 |
| Cytomox EX | 3 mg | 1.36±0.57 | 21.32 |
| Cytomox EX | 1 mg | 1.68±0.40 | 2.83 |
| Cytomox p53 | 1 mg | 1.48±0.67 | 14.73 |
| Cytomox PTEN | 1 mg | 1.57±0.59 | 9.13 |
| *Dose represents the dosage amount of 3 injections in second half. | | | |

As seen in Table 1, the direct injection into tumor foci of recombinant interferon α-2b caused inhibition of tumor growth by 16.06%. Intra-tumor injection with Cytomox HD showed inhibition of tumor growth by 18.37% at high dosage (3 mg protein/injection/kg body weight) and by 14.67% at low dosage (1 mg protein/injection/kg body weight). Thus, dose-dependent increase of tumor growth inhibitory effect was observed. Similarly, intra-tumor injection with Cytomox EX showed inhibition of tumor growth by 21.32% at high dosage (3 mg protein/injection/kg body weight) and by 2.83% at low dosage (1 mg protein/injection/kg body weight), and dose-dependent increase of the tumor growth inhibitory effect was also observed. Intra-tumor injection with Cytomox p53 or Cytomox PTEN also resulted in inhibition of tumor growth. These results indicate that the genetically-engineered microvesicles carrying transgene products can enter cells and functions of the transgene products can be transmitted to tumor cells.

In addition, the measurement of length and width of each tumor in mouse was done twice a week after the injection of test samples. A standard caliper was used to take the measurement. Based on the measured values, tumor volumes were calculated as follows.

$$\text{Tumor volume (mm}^3) = (\text{Tumor length x Tumor width})^2/2$$

In high dose Cytomox HD injection group, high dose Cytomox EX injection group, and Cytomox PTEN injection group, after 4 days of injection of test samples (i.e., after 15 days of tumor implantation), the increase of tumor volume was continuously inhibited compared to that of negative control group to the last day of the measurement (i.e., at 31 day after tumor implantation and after 20 days of injection of test samples). Also in Cytomox p53 injection group, the increase of tumor volume was significantly inhibited after 12 days of injection of the test sample. In particular in high dose Cytomox HD injection group and high dose Cytomox EX injection group, the differences of tumor volumes between the groups and negative control group were increased over time. At 31 day after tumor implantation (the last day of the measurement), the tumor volume is 1,250 $mm^3$ for negative control (PBS); about 1,000 $mm^3$ for Interferon-a 2b; about 950 $mm^3$ for Cytomox HD (evmp 130001, high dosage); about 1,000 $mm^3$ for Cytomox p53 (emvp 130003, high dosage); about 1,100 $mm^3$ for Cytomox PTEN (emvp 130006, high dosage); and about 1,000 $mm^3$ for Cytomox EX (T130075, high dosage).

6. Pathomorphological evaluation of tumor

The removed tumors were subjected to pathological examination to evaluate their molphology. The evaluation criterion is as follows:

"-", there is necrosis in the central area of the tumor foci, but there is no fibrosis;

"+", there is less degree of inflammation in tumor tissues, with low grade of fibrosis;

"++", there is inflammation in tumor tissues and decay of the tumor tissues, and fibrosis is observed all over the tumor; and

"+++" there is significant inflammation with tumor tissue decay and the highest grade of fibrosis is observed all over the tumor.

The results are shown in Table 2.

Table 2

| Mouse no. | Negative control | IFN α-2b 250x10$^4$ IU/kg | HD 1mg/kg | HD 3mg/kg | P53 1mg/kg | PTEN 1mg/kg | EX 1mg/kg | EX 3mg/kg |
|---|---|---|---|---|---|---|---|---|
| 1 | - | + | ++ | + | ++ | + | ++ | +++ |
| 2 | + | ++ | + | +++ | + | + | + | ++ |
| 3 | - | + | + | + | ++ | + | ++ | +++ |
| 4 | + | ++ | ++ | ++ | + | + | + | + |
| 5 | + | +++ | + | +++ | + | ++ | + | ++ |
| 6 | - | ++ | + | + | ++ | + | + | +++ |
| 7 | - | ++ | + | + | + | ++ | ++ | ++ |
| 8 | + | +++ | ++ | ++ | + | + | + | + |
| 9 | + | + | + | + | + | ++ | + | +++ |
| 10 | + | ++ | + | + | ++ | + | ++ | + |

As seen in Table 2, unlike the negative control, the administration of microvesicles carrying a tumor suppresor gene product into tumor foci frequently caused decay of tumor tissue and enhance inflammation and fibrosis in the tumor. In particular, the high dose injection of Cytomox HD or Cytomox ED to the tumor foci resulted in inflammation and decay of tumor tissues at higher levels and fibrosis over a wide range, which are different from tumor necrosis resulted from the lack of blood vessel angiogenesis in the central areas of tumor masses. It was considered that the decayed tumor tissues became fibrosis, which blocked the tumor expansion.

Figures 19 and 20 show typical morphologies observed for tumors from respective groups by pathological examination. 7. Western blotting analysis of tumor

Protein extraction was performed from the removed tumors of Cytomox PTEN injection group and negative control group according to conventional methods, and PTEN protein therein was detected by Western blotting analysis as described in Example 13. As a result, PTEN was detected in both the tumor of the negative control group (microvesicle-uninjected tumor tissues) and the tumor of the microvesicle injection group (microvesicle-injected tumor tissues), and the levels of PTEN in the microvesicle-injected tumor tissues, however, were higher by two times than those of the microvesicle-uninjected tumor tissues. This indicates that the efficiencies of cell entry by the microvescles and of delivery of transgenes/transgene products via the microvescles are sufficiently high. In addition, as seen from the result, with the increased PTEN levels in the Cytomox PTEN injected tumor cells, the growth of tumor was inhibited (see, 9.13% inhibition in Table 1).

Industrual Applicability

The present invention is useful for the efficient production of genetically engineered microvesicles. The microvesicles according to the present application can be used for delivering biological substances to cells.

## Claims

1. A method for producing microvesicles comprising a transgene product and/or a lentiviral RNA comprising a transgene, comprising the steps of:

   culturing a cell into which the transgene has been introduced using a lentiviral vector in vitro to extracellularly release microvesicles comprising the transgene product and/or the lentiviral RNA comprising the transgene, wherein said lentiviral vector is deficient in at least one structural protein gene, wherein said at least one structural protein gene is selected from the group consisting of gag, pol and env genes, and said lentiviral vector comprises the transgene under control of a telomerase reverse transcriptase (TERT) gene promoter in a lentiviral genome sequence, and
   collecting the microvesicles released.

2. The method according to claim 1, wherein said lentiviral vector is deficient in env gene.

3. The method according to claim 1 or 2, wherein said telomerase reverse transcriptase (TERT) gene promoter is a human TERT gene promoter.

4. The method according to claim 3, wherein said human TERT gene promoter comprises the nucleotide sequence of SEQ ID NO: 1 or a nucleotide sequence having 90% or more sequence identity to the nucleotide sequence of SEQ ID NO: 1.

5. The method according to any one of claims 1 to 4, wherein said lentiviral vector is:

   (i) an RNA vector comprising the lentiviral genome sequence,
   (ii) a DNA vector encoding an RNA comprising the lentiviral genome sequence, or
   (iii) a viral particle carrying an RNA comprising the lentiviral genome sequence.

6. The method according to any one of claims 1 to 5, wherein said lentiviral genome sequence is an HIV genome sequence.

7. The method according to any one of claims 1 to 6, wherein said lentiviral vector comprises said transgene being a tumor-suppressor gene.

8. The method according to claim 7, wherein said tumor-suppressor gene is PTEN or p16 gene.

9. The method according to any one of claims 1 to 6, wherein said lentiviral vector comprises said transgene that encodes a shRNA.

10. The method according to claim 9, wherein said shRNA targets a gene encoding a cell proliferation regulator.

11. The method according to claim 10, wherein said cell proliferation regulator is CDC6.

12. The method according to any one of claims 1 to 11, wherein said cell is a kidney-derived cell.

13. A microvesicle comprising (i) a transgene product and a lentiviral RNA comprising a transgene or (ii) a lentiviral RNA comprising a transgene, wherein said microvesicle is produced by the method according to any one of claims 1 to 12, and wherein said lentiviral RNA comprises a TERT gene promoter sequence upstream of the transgene.

14. A method of gene transduction comprising, contacting a target cell with the microvesicle according to claim 13 to fuse them, thereby introducing the transgene into the cell.

15. A composition comprising the microvesicle according to claim 13.

16. A pharmaceutical composition comprising the microvesicle according to claim 13.

17. The pharmaceutical composition according to claim 16, further comprising a pharmaceutically acceptable carrier.

**18.** A microvesicle according to claim 13 for use in treating a disease.

**19.** The microvesicle for use of claim 18, wherein said disease is cancer.

**20.** A method for producing a pharmaceutical composition, comprising producing microvesicles by the method of any one of claims 1-12 and preparing a pharmaceutical composition comprising the microvesicles, wherein said pharmaceutical composition preferably is suitable for treating a disease, such as cancer.

**21.** A method for increasing the release of microvesicles comprising a transgene product and/or a lentiviral RNA comprising a transgene, comprising the steps of:

introducing the transgene into a cell using a lentiviral vector in vitro, wherein said lentiviral vector is deficient in at least one structural protein gene, wherein said at least one structural protein gene is selected from the group consisting of gag, pol and env genes, and comprises the transgene under control of a telomease reverse transcriptase (TERT) gene promoter in a lentiviral genome sequence, and
culturing the cell.

## Patentansprüche

**1.** Verfahren zur Herstellung von Mikrovesikeln, die ein Transgenprodukt und/oder eine ein Transgen umfassende lentivirale RNA umfassen, umfassend die folgenden Schritte:

Kultivieren einer Zelle, in die das Transgen mithilfe eines lentiviralen Vektors eingebracht wurde, in vitro zur extrazellulären Freisetzung von Mikrovesikeln, die das Transgenprodukt und/oder die das Transgen enthaltende lentivirale RNA umfassen, wobei dem lentiviralen Vektor mindestens ein Strukturproteingen fehlt, wobei das mindestens eine Strukturproteingen ausgewählt ist aus der Gruppe, bestehend aus gag-, pol- und env-Genen, und der lentivirale Vektor das Transgen unter der Kontrolle eines Genpromotors für Telomerase-Reverse-Transkriptase (TERT) in einer lentiviralen Genomsequenz umfasst, und
Auffangen der freigesetzten Mikrovesikel.

**2.** Verfahren nach Anspruch 1, wobei dem lentiviralen Vektor das env-Gen fehlt.

**3.** Verfahren nach Anspruch 1 oder 2, wobei es sich bei dem Genpromotor für Telomerase-Reverse-Transkriptase (TERT) um einen humanen TERT-Genpromotor handelt.

**4.** Verfahren nach Anspruch 3, wobei der humane TERT-Genpromotor die Nukleotidsequenz von SEQ ID NO: 1 oder eine Nukleotidsequenz mit 90 % oder mehr Sequenzidentität mit der Nukleotidsequenz von SEQ ID NO: 1 umfasst.

**5.** Verfahren nach einem der Ansprüche 1 bis 4, wobei es sich bei dem lentiviralen Vektor um Folgendes handelt:

(i) einen RNA-Vektor, der die lentivirale Genomsequenz umfasst,
(ii) ein DNA-Vektor, der für eine RNA kodiert, die die lentivirale Genomsequenz umfasst, oder
(iii) ein Viruspartikel, das eine RNA enthält, die die lentivirale Genomsequenz umfasst.

**6.** Verfahren nach einem der Ansprüche 1 bis 5, wobei es sich bei der lentiviralen Genomsequenz um eine HIV-Genomsequenz handelt.

**7.** Verfahren nach einem der Ansprüche 1 bis 6, wobei der lentivirale Vektor das Transgen umfasst, bei dem es sich um ein Tumorsuppressor-Gen handelt.

**8.** Verfahren nach Anspruch 7, wobei es sich bei dem Tumorsuppressor-Gen um das PTEN- oder p16-Gen handelt.

**9.** Verfahren nach einem der Ansprüche 1 bis 6, wobei der lentivirale Vektor das Transgen umfasst, das für eine shRNA kodiert.

**10.** Verfahren nach Anspruch 9, wobei die shRNA auf ein Gen gerichtet ist, das für einen Zellproliferationsregulator kodiert.

**11.** Verfahren nach Anspruch 10, wobei es sich bei dem Zellproliferationsregulator um CDC6 handelt.

**12.** Verfahren nach einem der Ansprüche 1 bis 11, wobei es sich bei der Zelle um eine aus der Niere stammende Zelle handelt.

**13.** Mikrovesikel, umfassend (i) ein Transgenprodukt und eine ein Transgen umfassende lentivirale RNA oder (ii) eine ein Transgen umfassende lentivirale RNA, wobei das Mikrovesikel durch das Verfahren nach einem der Ansprüche 1 bis 12 hergestellt wird und wobei die lentivirale RNA eine TERT-Genpromotorsequenz stromaufwärts des Transgens umfasst.

**14.** Verfahren zur Gentransduktion, umfassend das Inkontaktbringen einer Zielzelle mit dem Mikrovesikel nach Anspruch 13, um diese miteinander zu verschmelzen und dadurch das Transgen in die Zelle einzubringen.

**15.** Zusammensetzung, die das Mikrovesikel nach Anspruch 13 umfasst.

**16.** Pharmazeutische Zusammensetzung, die das Mikrovesikel nach Anspruch 13 umfasst.

**17.** Pharmazeutische Zusammensetzung nach Anspruch 16, die ferner einen pharmazeutisch verträglichen Träger umfasst.

**18.** Mikrovesikel nach Anspruch 13 zur Verwendung für die Behandlung einer Erkrankung.

**19.** Mikrovesikel zur Verwendung nach Anspruch 18, wobei es sich bei der Erkrankung um Krebs handelt.

**20.** Verfahren zur Herstellung einer pharmazeutischen Zusammensetzung, umfassend die Herstellung von Mikrovesikeln durch das Verfahren nach einem der Ansprüche 1 bis 12 und die Herstellung einer die Mikrovesikel umfassenden pharmazeutischen Zusammensetzung, wobei die pharmazeutische Zusammensetzung vorzugsweise zur Behandlung einer Erkrankung, wie etwa Krebs, geeignet ist.

**21.** Verfahren zur Erhöhung der Freisetzung von Mikrovesikeln, die ein Transgenprodukt und/oder eine ein Transgen umfassende lentivirale RNA umfassen, umfassend die folgenden Schritte:

Einbringen des Transgens in eine Zelle mithilfe eines lentiviralen Vektors in vitro, wobei dem lentiviralen Vektor mindestens ein Strukturproteingen fehlt, wobei das mindestens eine Strukturproteingen ausgewählt ist aus der Gruppe, bestehend aus gag-, pol- und env-Genen, und der lentivirale Vektor das Transgen unter der Kontrolle eines Genpromotors für Telomerase-Reverse-Transkriptase (TERT) in einer lentiviralen Genomsequenz umfasst, und
Kultivieren der Zelle.

## Revendications

**1.** Procédé pour la production de microvésicules comprenant un produit transgénique et/ou un ARN lentiviral comprenant un transgène, comprenant les étapes consistant à :

effectuer la culture d'une cellule dans laquelle le transgène a été introduit à l'aide d'un vecteur lentiviral *in vitro* pour libérer de manière extracellulaire des microvésicules comprenant le produit transgénique et/ou l'ARN lentiviral comprenant le transgène, ledit vecteur lentiviral ayant au moins un gène de protéine structurale défectueux, ledit au moins un gène de protéine structurale étant choisi dans le groupe constitué par les gènes gag, pol et env, et ledit vecteur lentiviral comprenant le transgène sous le contrôle d'un promoteur de gène de transcriptase inverse de télomérase (TERT) dans une séquence de génome lentiviral, et
récupérer les microvésicules libérées.

**2.** Procédé selon la revendication 1, dans lequel ledit vecteur lentiviral a un gène env défectueux.

**3.** Procédé selon la revendication 1 ou 2, dans lequel ledit promoteur de gène de transcriptase inverse de télomérase (TERT) est un promoteur de gène de TERT humain.

4. Procédé selon la revendication 3, dans lequel ledit promoteur de gène de TERT humain comprend la séquence de nucléotides de SEQ ID n° : 1 ou une séquence de nucléotides ayant une identité de séquence supérieure ou égale à 90 % avec la séquence de nucléotides de SEQ ID n° : 1.

5. Procédé selon l'une quelconque des revendications 1 à 4, dans lequel ledit vecteur lentiviral est :

(i) un vecteur à ARN comprenant la séquence de génome lentiviral,
(ii) un vecteur à ADN codant pour un ARN comprenant la séquence de génome lentiviral ou
(iii) une particule virale portant un ARN comprenant la séquence de génome lentiviral.

6. Procédé selon l'une quelconque des revendications 1 à 5, dans lequel ladite séquence de génome lentiviral est une séquence de génome de VIH.

7. Procédé selon l'une quelconque des revendications 1 à 6, dans lequel ledit vecteur lentiviral comprend ledit transgène qui est un gène suppresseur de tumeur.

8. Procédé selon la revendication 7, dans lequel ledit gène suppresseur de tumeur est le gène PTEN ou p16.

9. Procédé selon l'une quelconque des revendications 1 à 6, dans lequel ledit vecteur lentiviral comprend ledit transgène qui code pour un petit ARN en épingle à cheveux (shRNA).

10. Procédé selon la revendication 9, dans lequel ledit shRNA cible un gène codant pour un régulateur de la prolifération cellulaire.

11. Procédé selon la revendication 10, dans lequel ledit régulateur de la prolifération cellulaire est CDC6.

12. Procédé selon l'une quelconque des revendications 1 à 11, dans lequel ladite cellule est une cellule d'origine rénale.

13. Microvésicule comprenant (i) un produit transgénique et un ARN lentiviral comprenant un transgène ou (ii) un ARN lentiviral comprenant un transgène, ladite microvésicule étant produite par le procédé selon l'une quelconque des revendications 1 à 12 et ledit ARN lentiviral comprenant une séquence promoteur de gène de TERT en amont du transgène.

14. Procédé de transduction de gène comprenant la mise en contact d'une cellule cible avec la microvésicule selon la revendication 13 pour les fusionner, ce qui introduit ainsi le transgène dans la cellule.

15. Composition comprenant la microvésicule selon la revendication 13.

16. Composition pharmaceutique comprenant la microvésicule selon la revendication 13.

17. Composition pharmaceutique selon la revendication 16, comprenant en outre un véhicule pharmaceutiquement acceptable.

18. Microvésicule selon la revendication 13 destinée à être utilisée en traitement d'une maladie.

19. Microvésicule destinée à être utilisée selon la revendication 18, ladite maladie étant un cancer.

20. Procédé pour la production d'une composition pharmaceutique, comprenant la production de microvésicules par le procédé selon l'une quelconque des revendications 1-12 et la préparation d'une composition pharmaceutique comprenant les microvésicules, dans lequel ladite composition pharmaceutique est de préférence appropriée pour le traitement d'une maladie, telle qu'un cancer.

21. Procédé pour l'augmentation de la libération de microvésicules comprenant un produit transgénique et/ou un ARN lentiviral comprenant un transgène, comprenant les étapes consistant à :

introduire le transgène dans une cellule à l'aide d'un vecteur lentiviral *in vitro,* ledit vecteur lentiviral ayant au moins un gène de protéine structurale défectueux, ledit au moins un gène de protéine structurale étant choisi dans le groupe constitué par les gènes gag, pol et env, et comprenant le transgène sous le contrôle d'un promoteur de gène de transcriptase inverse de télomérase (TERT) dans une séquence de génome lentiviral, et effectuer la culture de la cellule.

Fig. 1

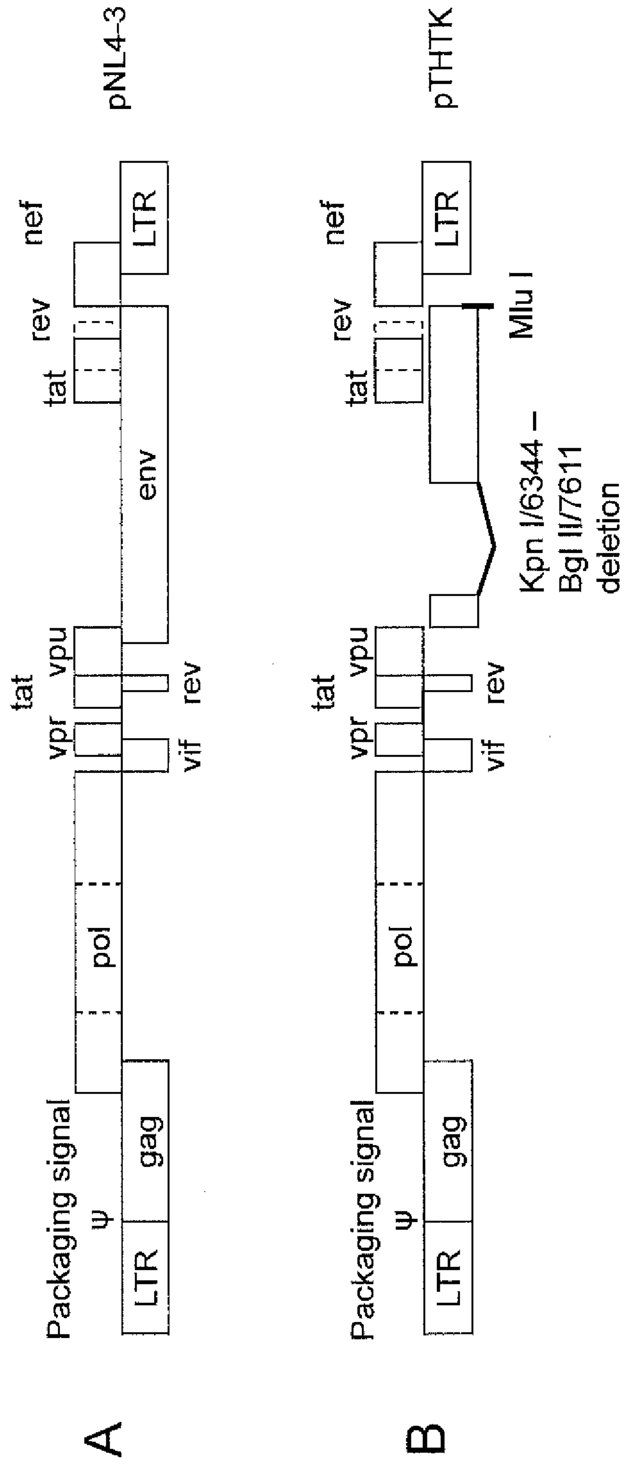
A
B
pNL4-3
pTHTK
LTR
Packaging signal
ψ
gag
pol
vif
vpr
tat
vpu
rev
env
tat
rev
nef
LTR
Kpn I/6344 –
Bgl II/7611
deletion
Mlu I

Fig. 2

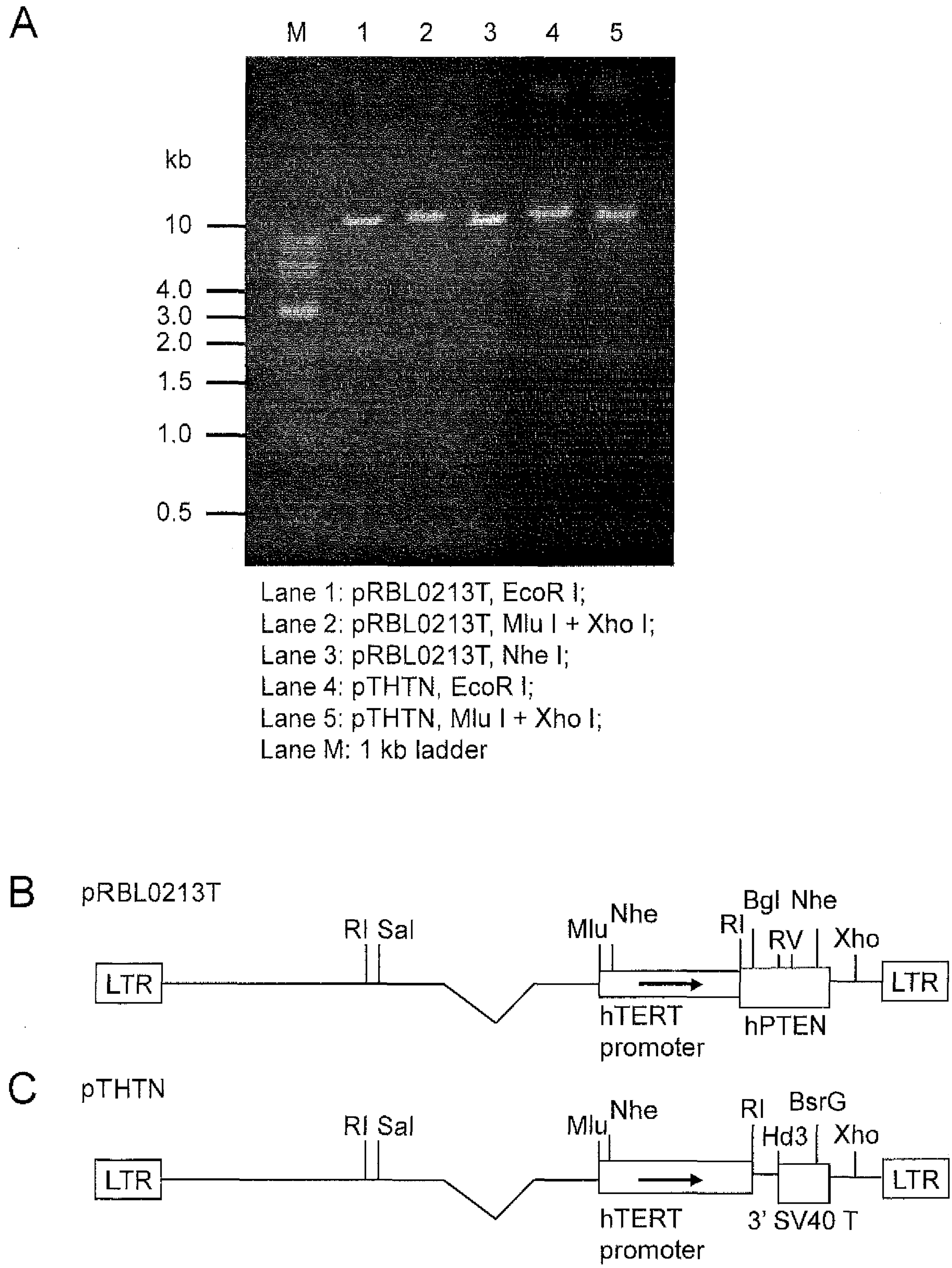
A
M 1 2 3 4 5
kb
10
4.0
3.0
2.0
1.5
1.0
0.5
Lane 1: pRBL0213T, EcoR I;
Lane 2: pRBL0213T, Mlu I + Xho I;
Lane 3: pRBL0213T, Nhe I;
Lane 4: pTHTN, EcoR I;
Lane 5: pTHTN, Mlu I + Xho I;
Lane M: 1 kb ladder
B
pRBL0213T
LTR
RI Sal
Mlu
Nhe
RI
Bgl Nhe
RV
Xho
LTR
hTERT promoter
hPTEN
C
pTHTN
LTR
RI Sal
Mlu
Nhe
RI
BsrG
Hd3
Xho
LTR
hTERT promoter
3' SV40 T

# Fig. 3

A

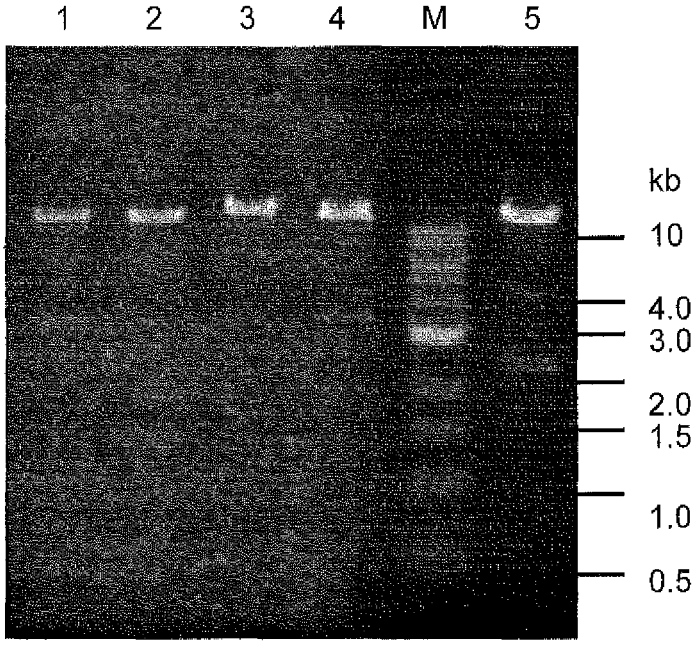


Lane 1: pRBL001, EcoR I;
Lane 2: pRBL001, EcoR I + Nhe I;
Lane 3: pRBL001, Mlu I + Xho I;
Lane 4: pRBL001, Sal I;
Lane 5: pRBL0213T, Nhe I;
Lane M: 1 kb ladder

B pRBL001

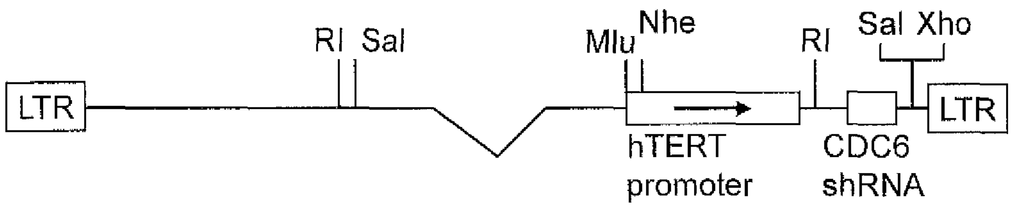

Fig. 4

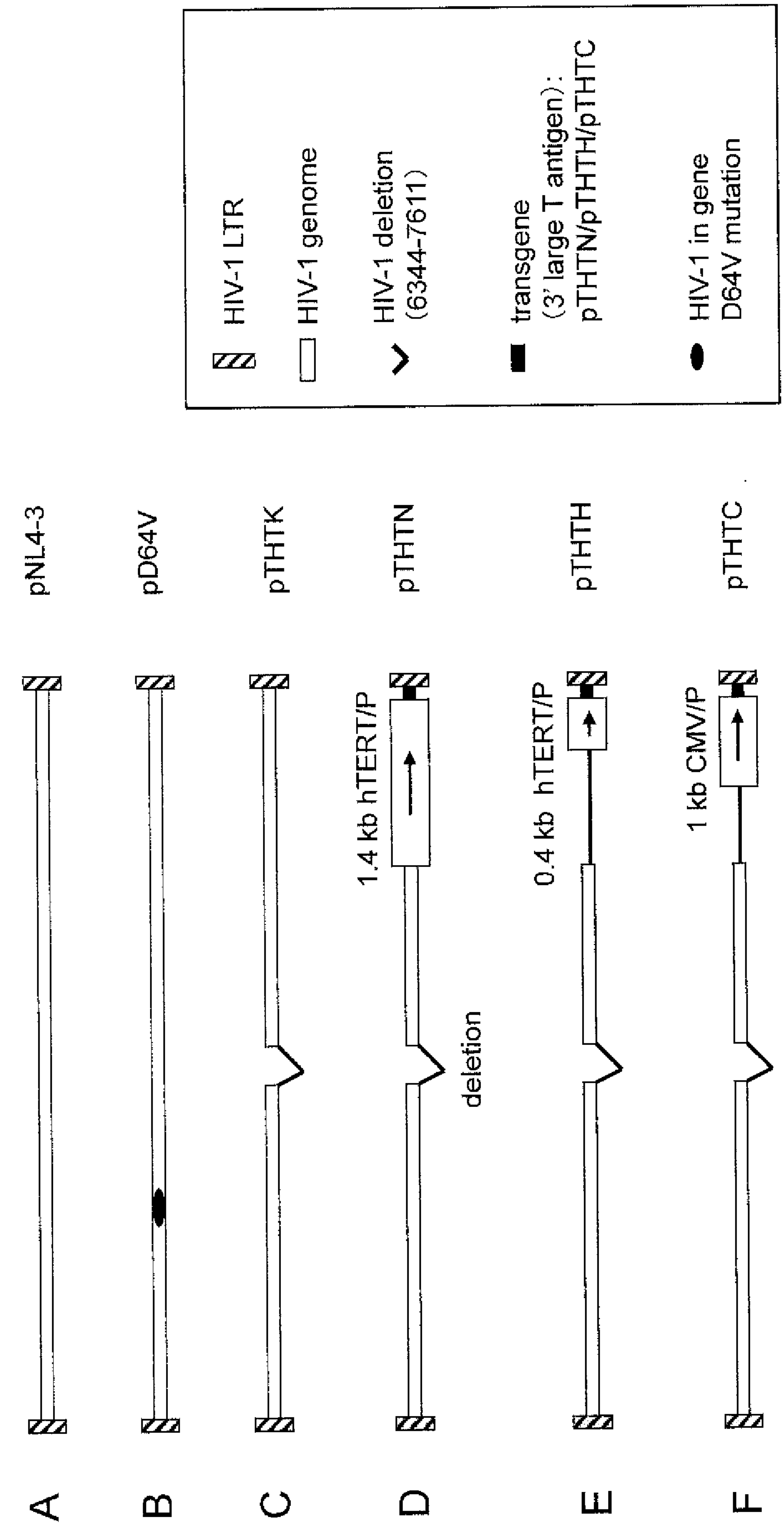
HIV-1 LTR
HIV-1 genome
HIV-1 deletion (6344-7611)
transgene (3' large T antigen): pTHTN/pTHTH/pTHTC
HIV-1 in gene D64V mutation
A
B
C
D
E
F
pNL4-3
pD64V
pTHTK
pTHTN
pTHTH
pTHTC
1.4 kb hTERT/P
0.4 kb hTERT/P
1 kb CMV/P
deletion

Fig. 5

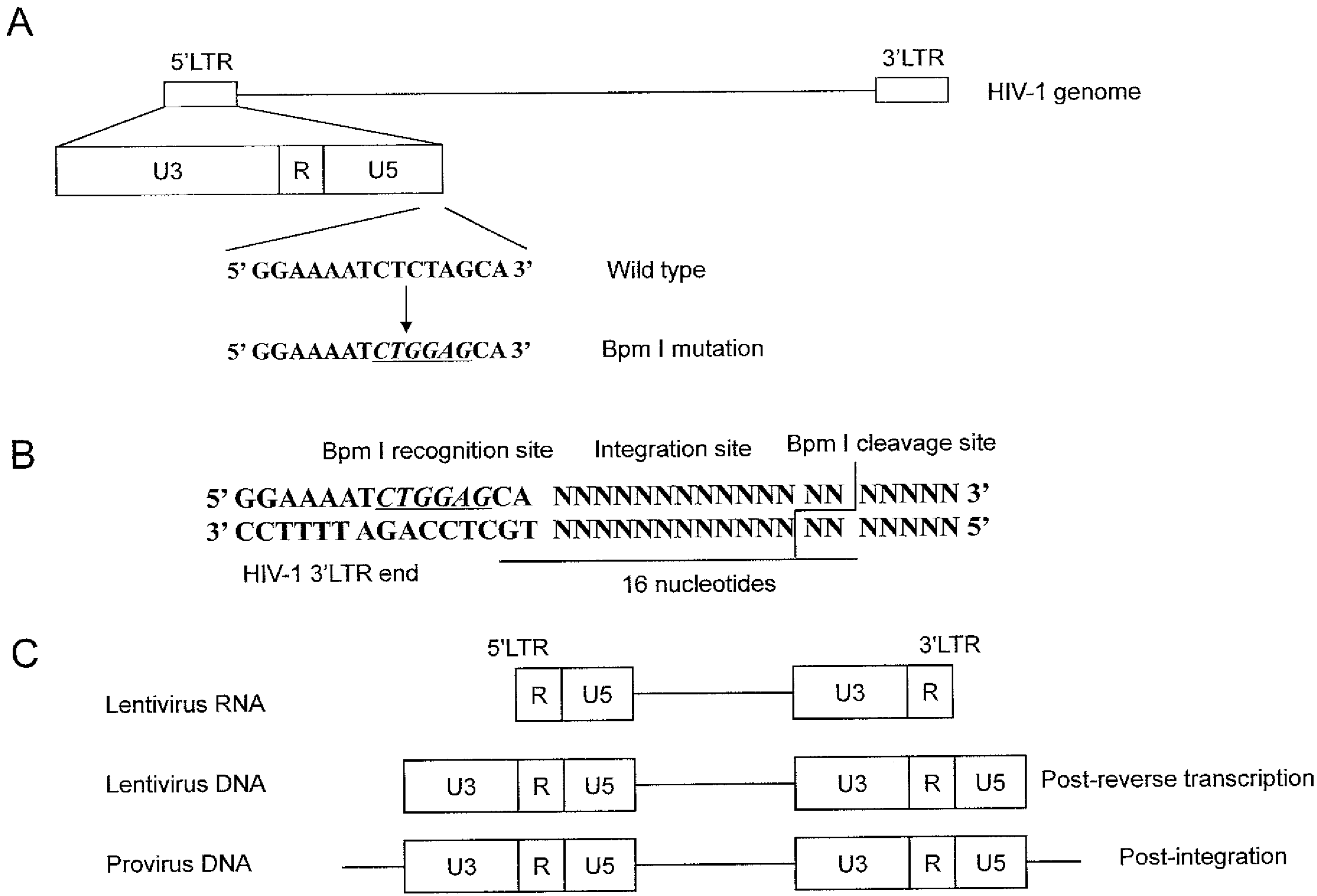
A
5'LTR
3'LTR
HIV-1 genome
U3
R
U5
5' GGAAAATCTCTAGCA 3'
Wild type
5' GGAAAATCTGGAGCA 3'
Bpm I mutation
B
Bpm I recognition site
Integration site
Bpm I cleavage site
5' GGAAAATCTGGAGCA NNNNNNNNNNNN NN NNNNN 3'
3' CCTTTT AGACCTCGT NNNNNNNNNNNN NN NNNNN 5'
HIV-1 3'LTR end
16 nucleotides
C
5'LTR
3'LTR
Lentivirus RNA
Lentivirus DNA
Provirus DNA
Post-reverse transcription
Post-integration
U3
R
U5

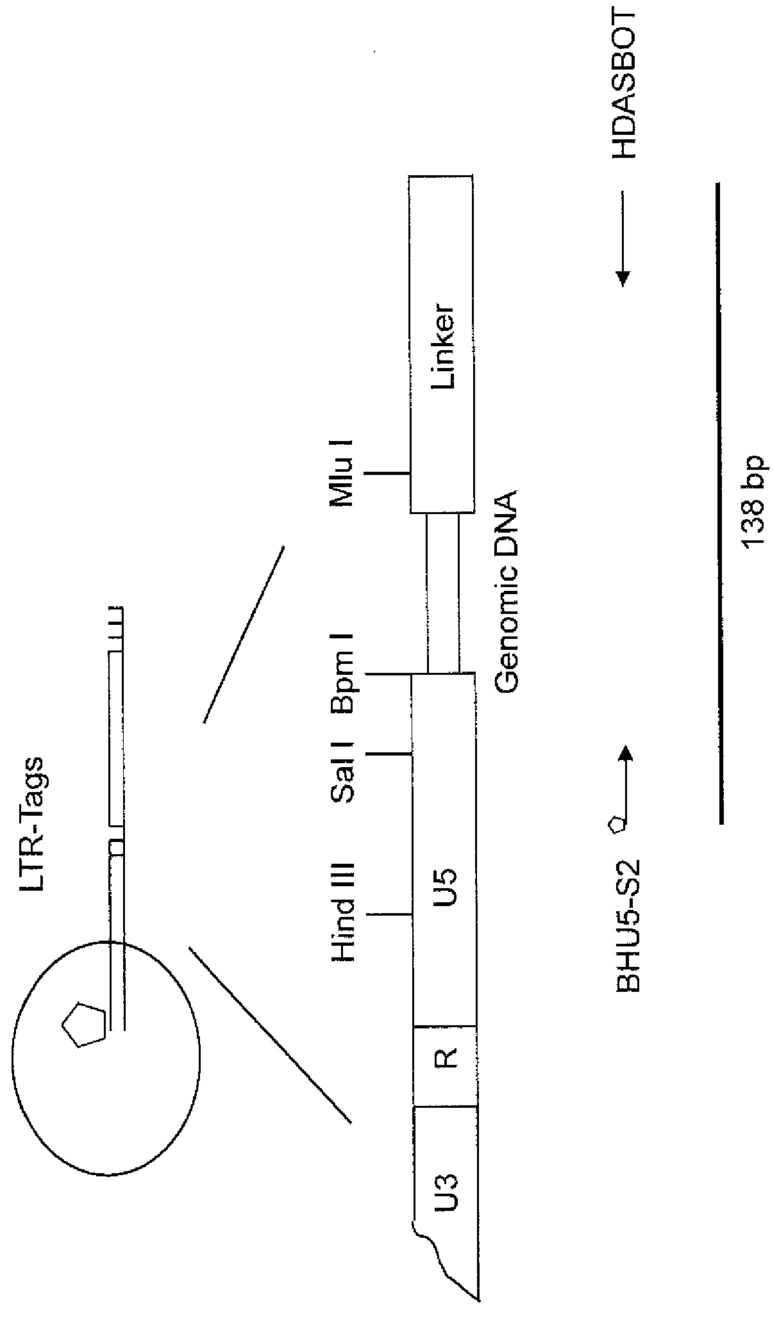
LTR-Tags
U3
R
U5
Hind III
Sal I
Bpm I
Mlu I
Genomic DNA
Linker
BHU5-S2
HDASBOT
138 bp

Fig. 6

Fig. 7

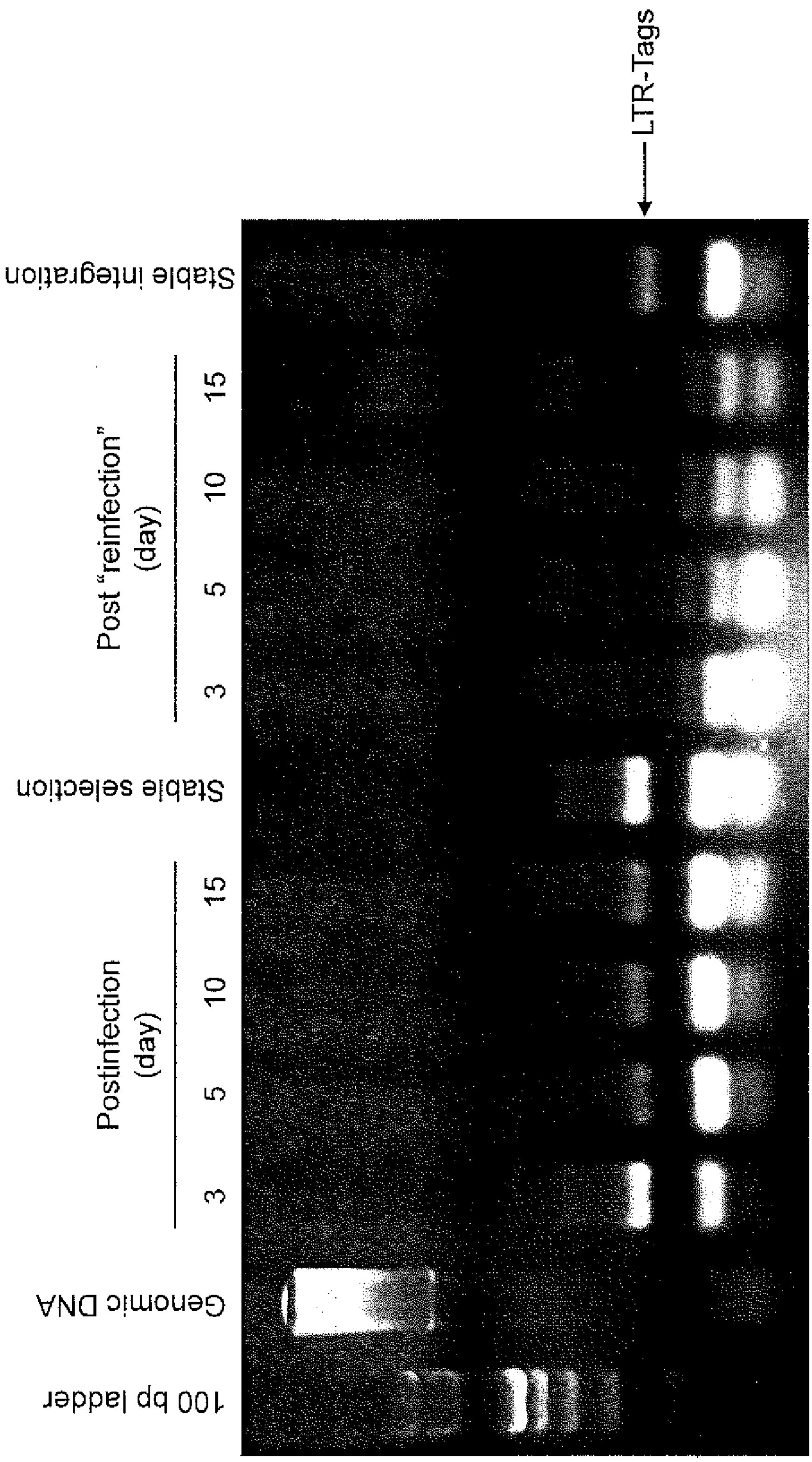
100 bp ladder
Genomic DNA
Postinfection
(day)
3
5
10
15
Stable selection
Post "reinfection"
(day)
3
5
10
15
Stable integration
LTR-Tags

Fig. 8

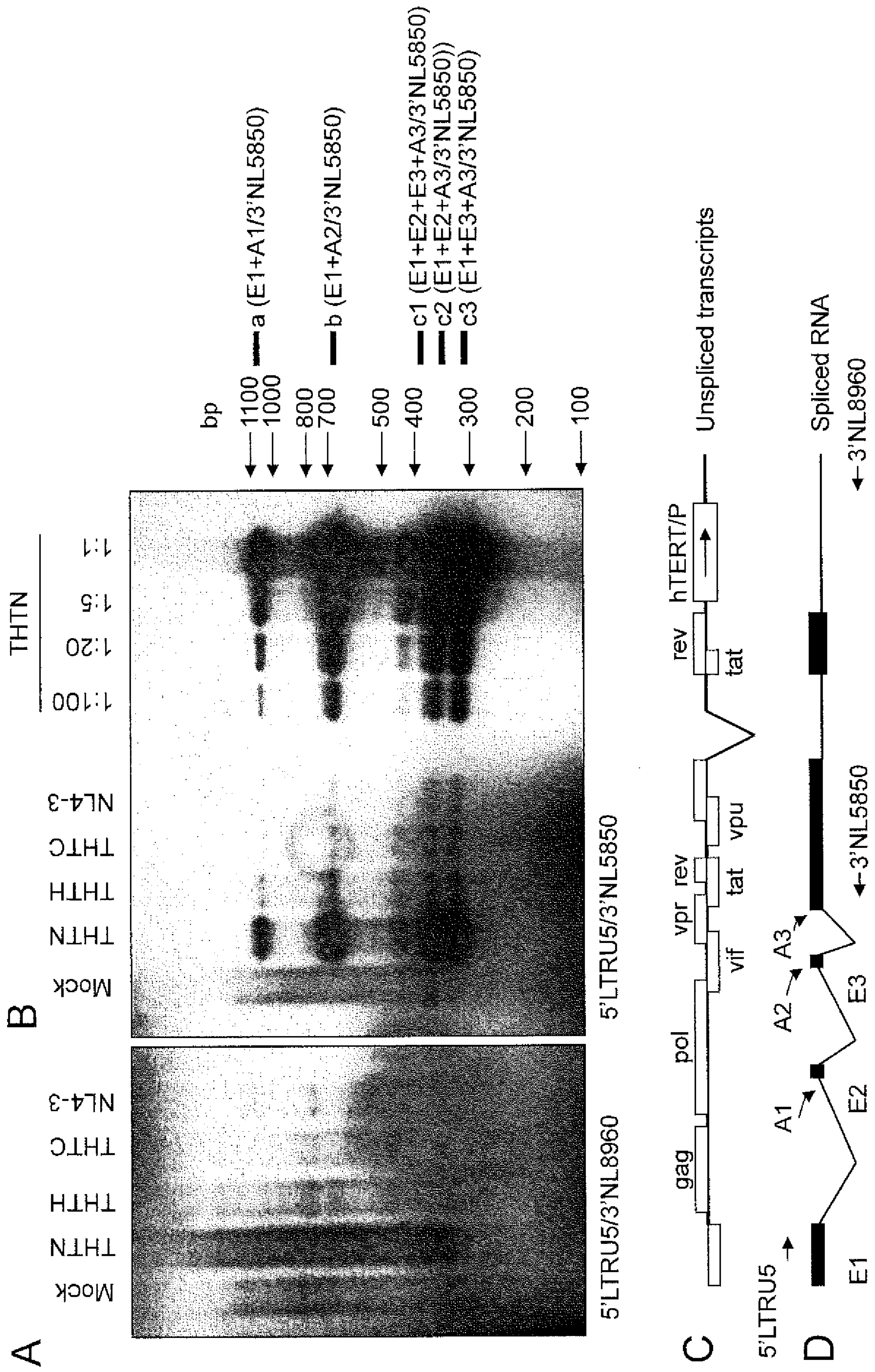
A
Mock
THTN
THTH
THTC
NL4-3
5'LTRU5/3'NL8960
B
Mock
THTN
THTH
THTC
NL4-3
THTN
1:100
1:20
1:5
1:1
5'LTRU5/3'NL5850
bp
1100
1000
800
700
500
400
300
200
100
a (E1+A1/3'NL5850)
b (E1+A2/3'NL5850)
c1 (E1+E2+E3+A3/3'NL5850)
c2 (E1+E2+A3/3'NL5850))
c3 (E1+E3+A3/3'NL5850)
C
gag
pol
vif
vpr
rev
tat
vpu
rev
tat
hTERT/P
Unspliced transcripts
D
5'LTRU5
E1
A1
E2
A2
E3
A3
3'NL5850
3'NL8960
Spliced RNA

Fig. 9

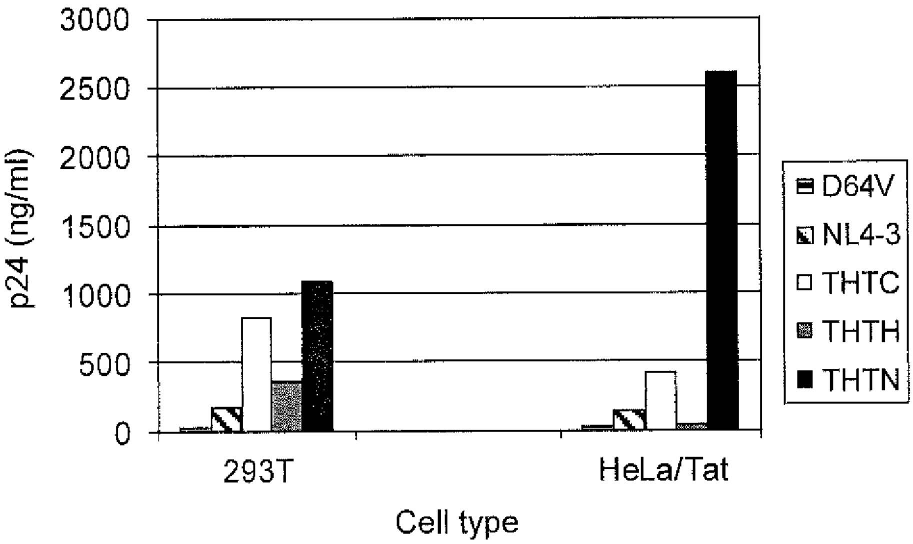
3000
2500
2000
1500
1000
500
0
p24 (ng/ml)
D64V
NL4-3
THTC
THTH
THTN
293T
HeLa/Tat
Cell type

Fig. 10

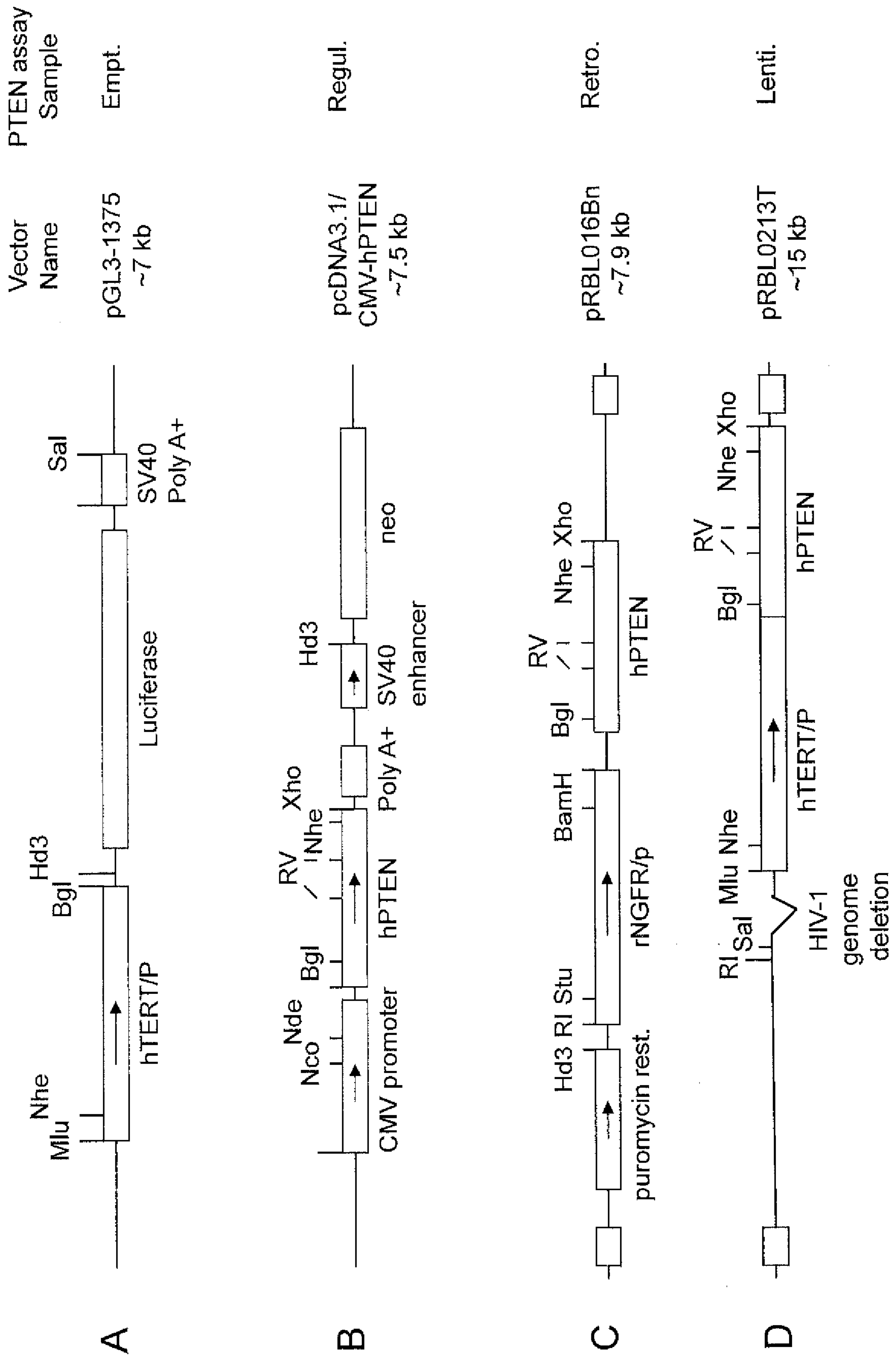
Vector Name
PTEN assay Sample
A
Mlu Nhe
hTERT/P
Bgl Hd3
Luciferase
Sal
SV40 Poly A+
pGL3-1375 ~7 kb
Empt.
B
Nco Nde
CMV promoter
Bgl
RV
Nhe
Xho
hPTEN
Poly A+
SV40 enhancer
Hd3
neo
pcDNA3.1/ CMV-hPTEN ~7.5 kb
Regul.
C
puromycin rest.
Hd3 RI Stu
rNGFR/p
BamH
Bgl
RV
Nhe Xho
hPTEN
pRBL016Bn ~7.9 kb
Retro.
D
RI Sal
HIV-1 genome deletion
Mlu Nhe
hTERT/P
Bgl
RV
Nhe Xho
hPTEN
pRBL0213T ~15 kb
Lenti.

Fig. 11

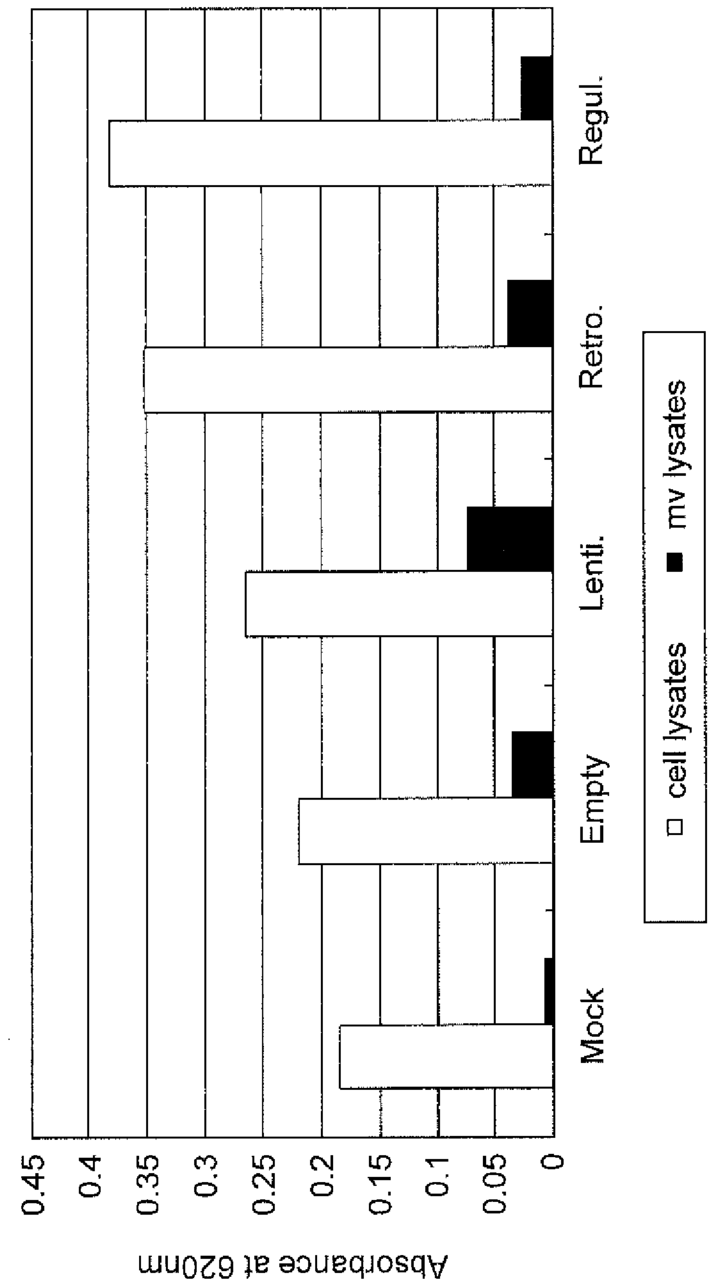
Absorbance at 620nm
0
0.05
0.1
0.15
0.2
0.25
0.3
0.35
0.4
0.45
Mock
Empty
Lenti.
Retro.
Regul.
cell lysates
mv lysates

Fig. 12

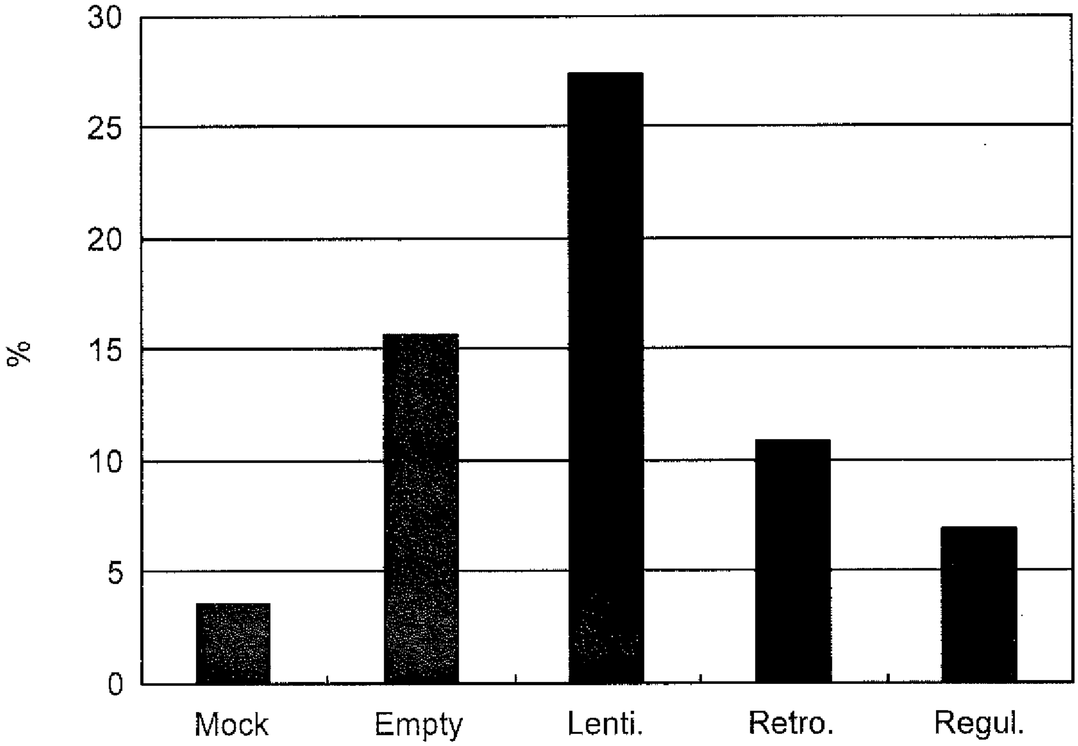

Fig. 13

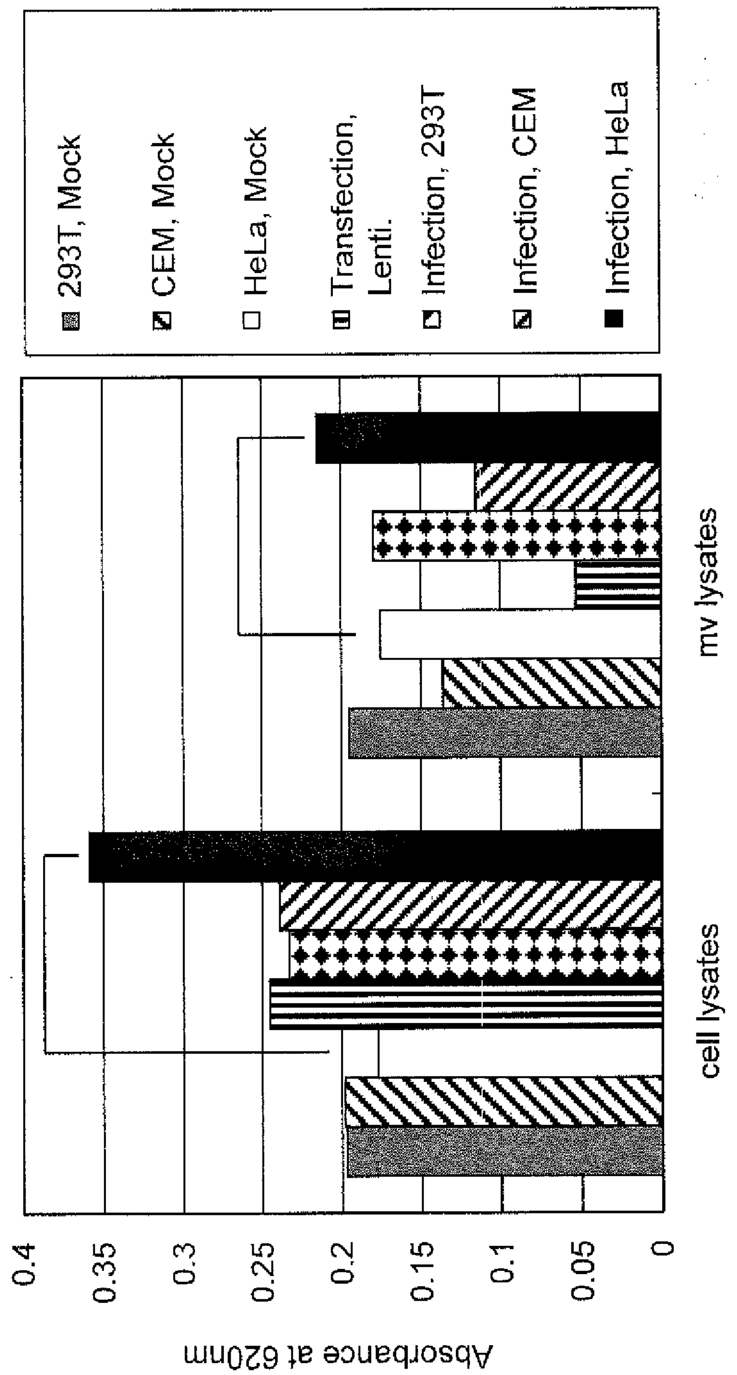

Absorbance at 620nm
0
0.05
0.1
0.15
0.2
0.25
0.3
0.35
0.4
cell lysates
mv lysates
293T, Mock
CEM, Mock
HeLa, Mock
Transfection, Lenti.
Infection, 293T
Infection, CEM
Infection, HeLa

Fig. 14

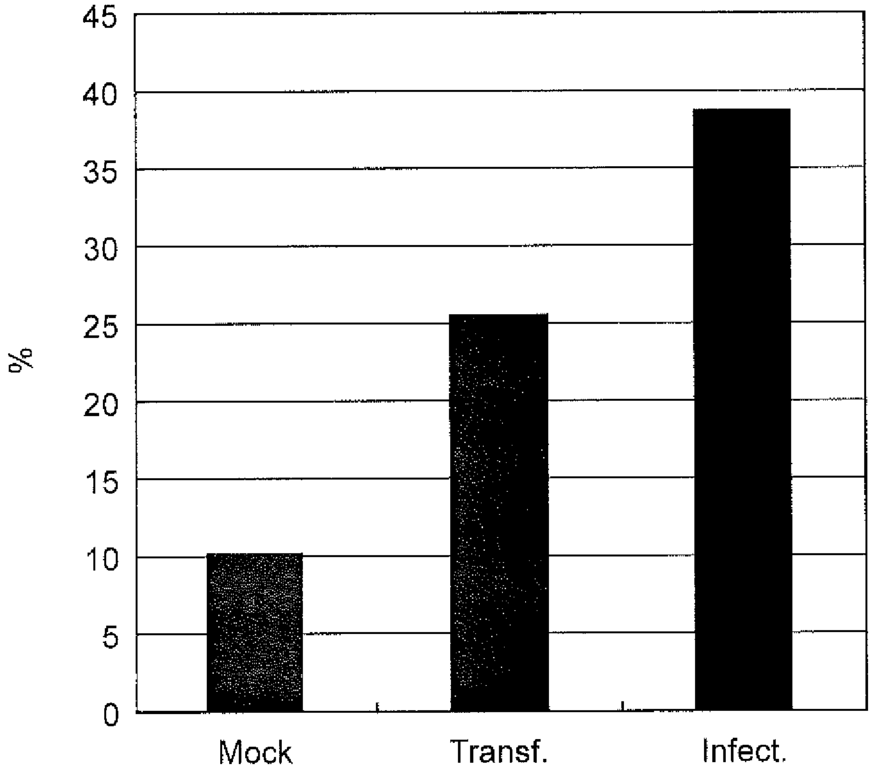

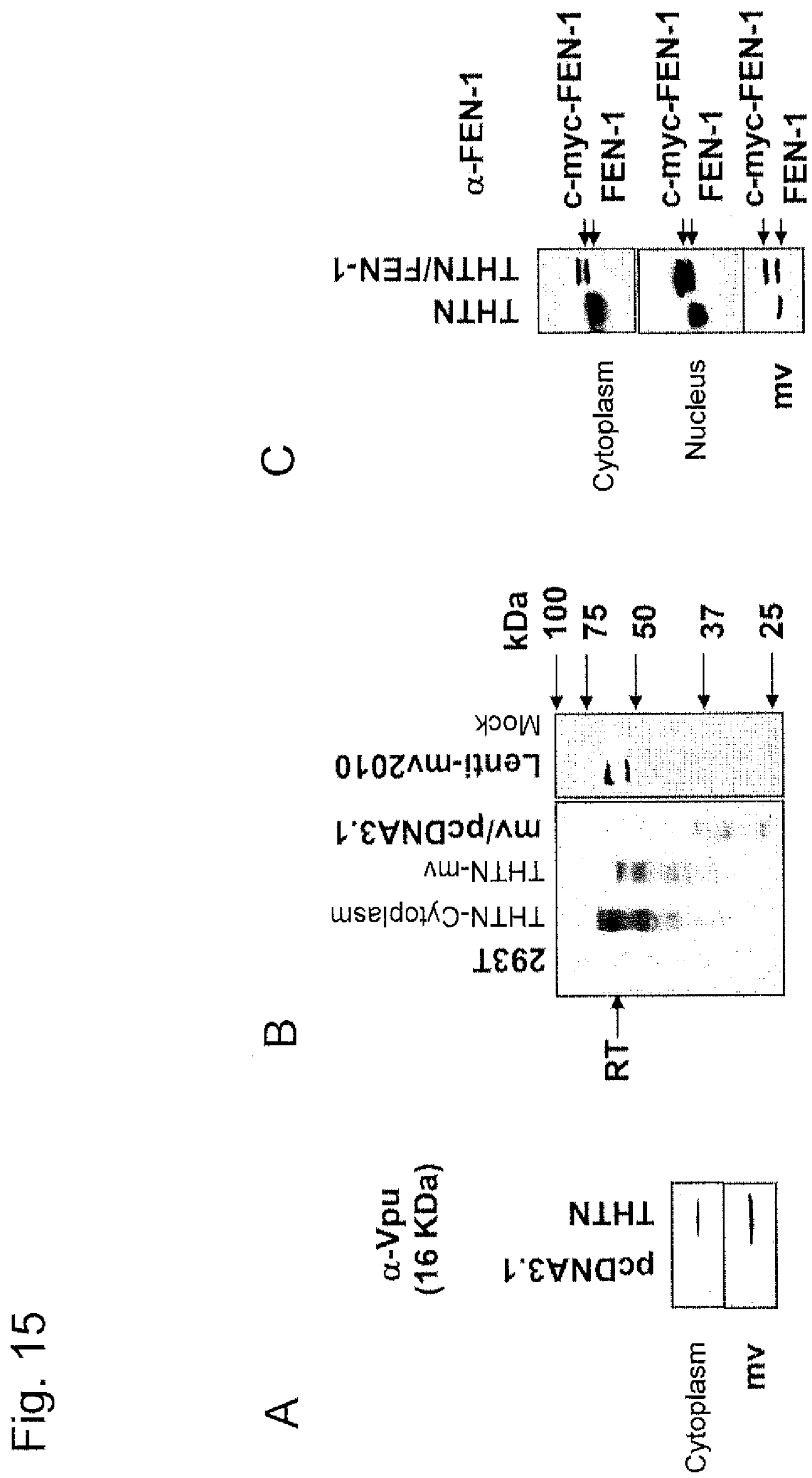
Fig. 15
A
α-Vpu
(16 KDa)
pcDNA3.1
THTN
Cytoplasm
mv
B
RT
293T
THTN-Cytoplasm
THTN-mv
mv/pcDNA3.1
Lenti-mv2010
Mock
kDa
100
75
50
37
25
C
α-FEN-1
THTN
THTN/FEN-1
Cytoplasm
Nucleus
mv
c-myc-FEN-1
FEN-1
c-myc-FEN-1
FEN-1
c-myc-FEN-1
FEN-1

Fig. 16

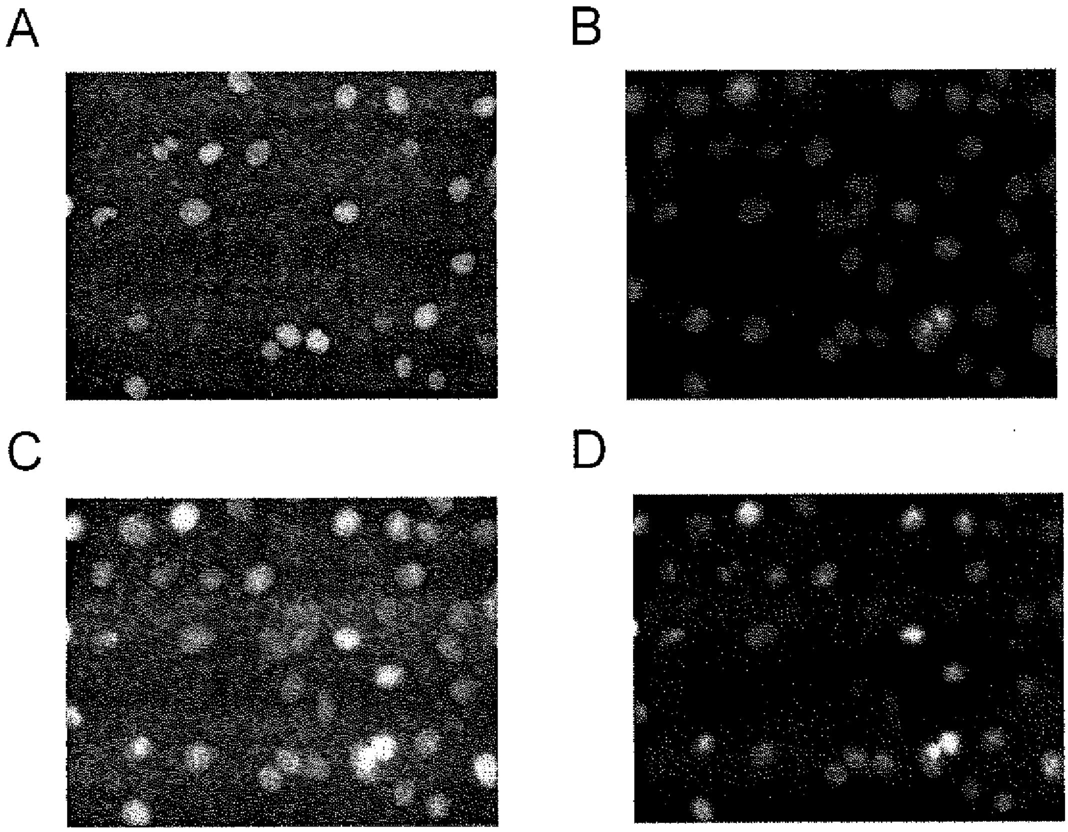

Fig. 17

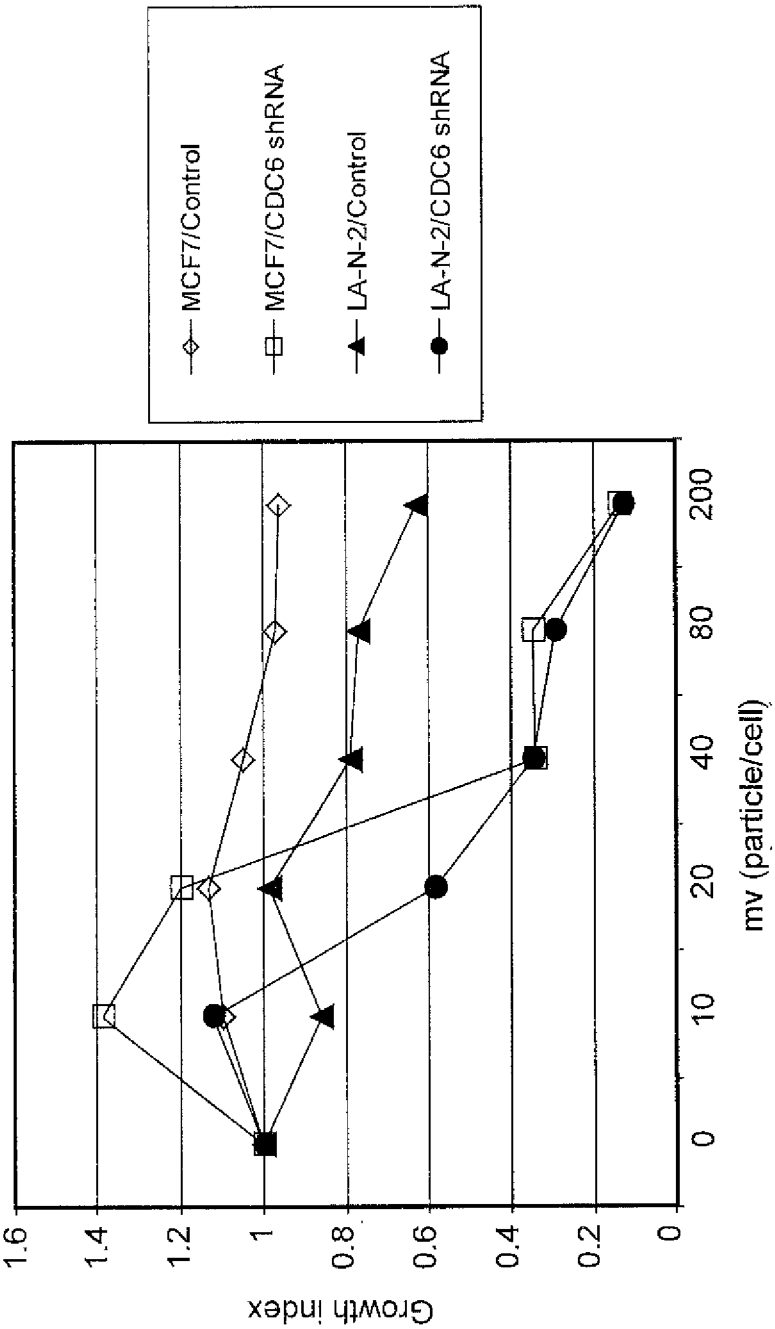
MCF7/Control
MCF7/CDC6 shRNA
LA-N-2/Control
LA-N-2/CDC6 shRNA
Growth index
1.6
1.4
1.2
1
0.8
0.6
0.4
0.2
0
0
10
20
40
80
200
mv (particle/cell)

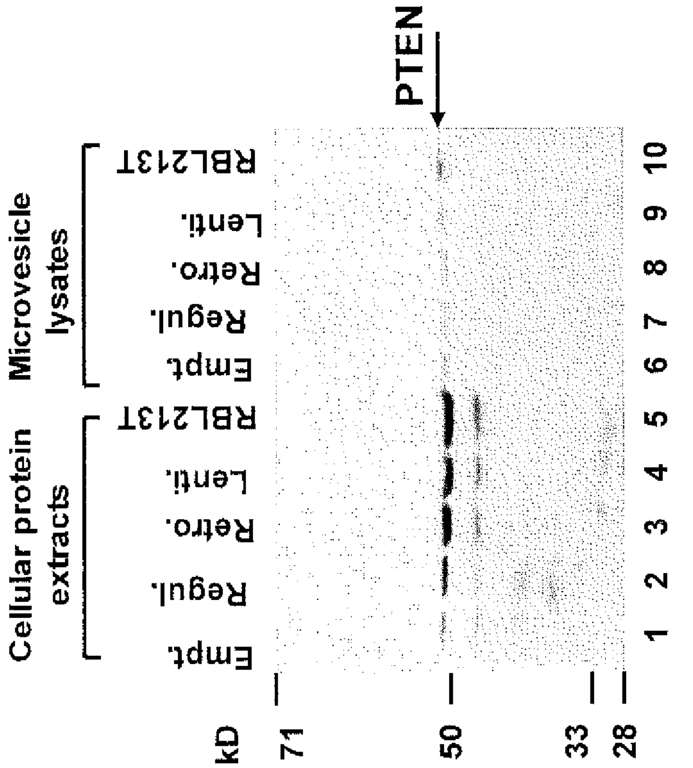
Cellular protein extracts
Microvesicle lysates
Empt.
Regul.
Retro.
Lenti.
RBL213T
Empt.
Regul.
Retro.
Lenti.
RBL213T
PTEN
kD
71
50
33
28
1
2
3
4
5
6
7
8
9
10

Fig. 18

Fig. 19

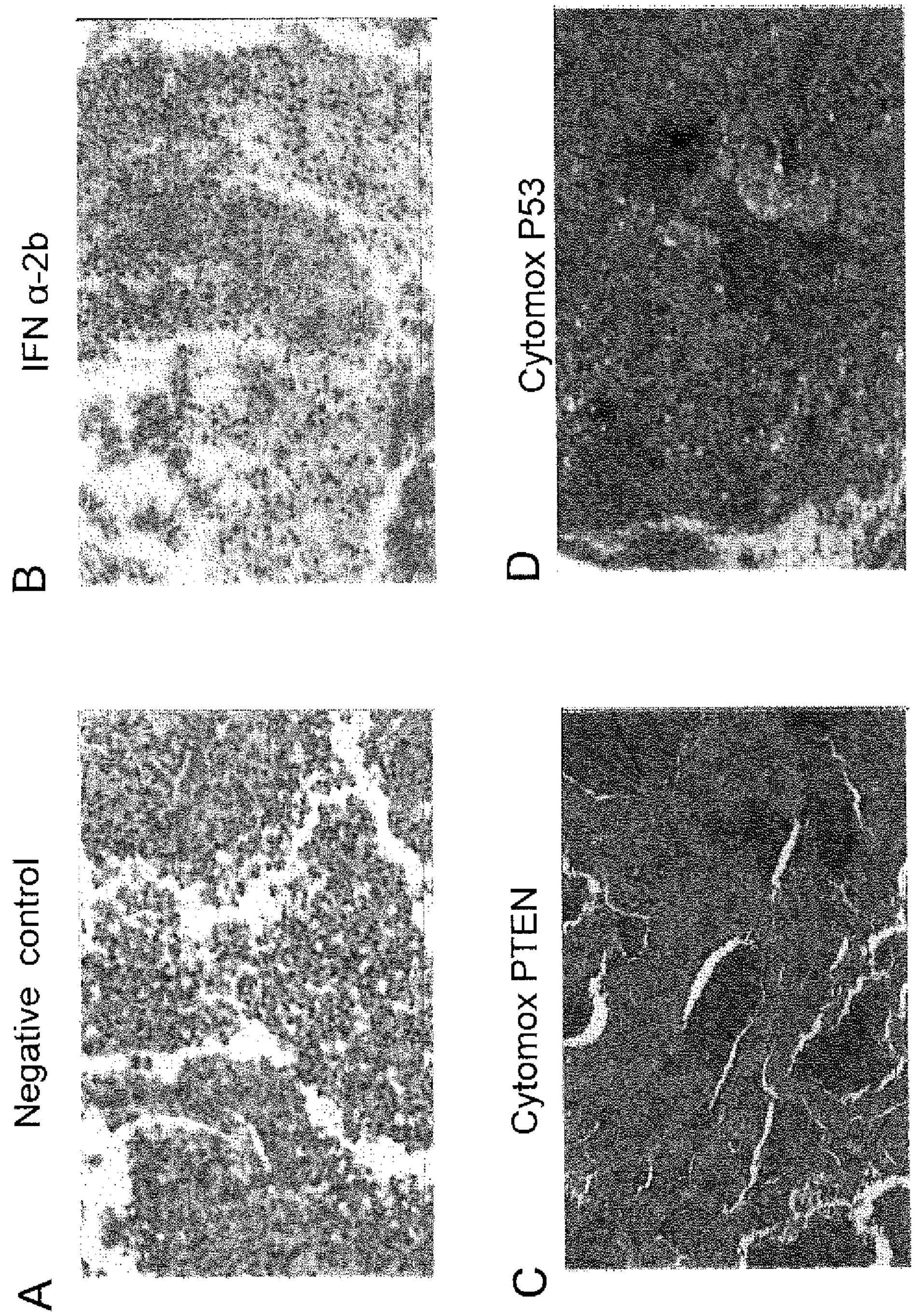

Fig. 20

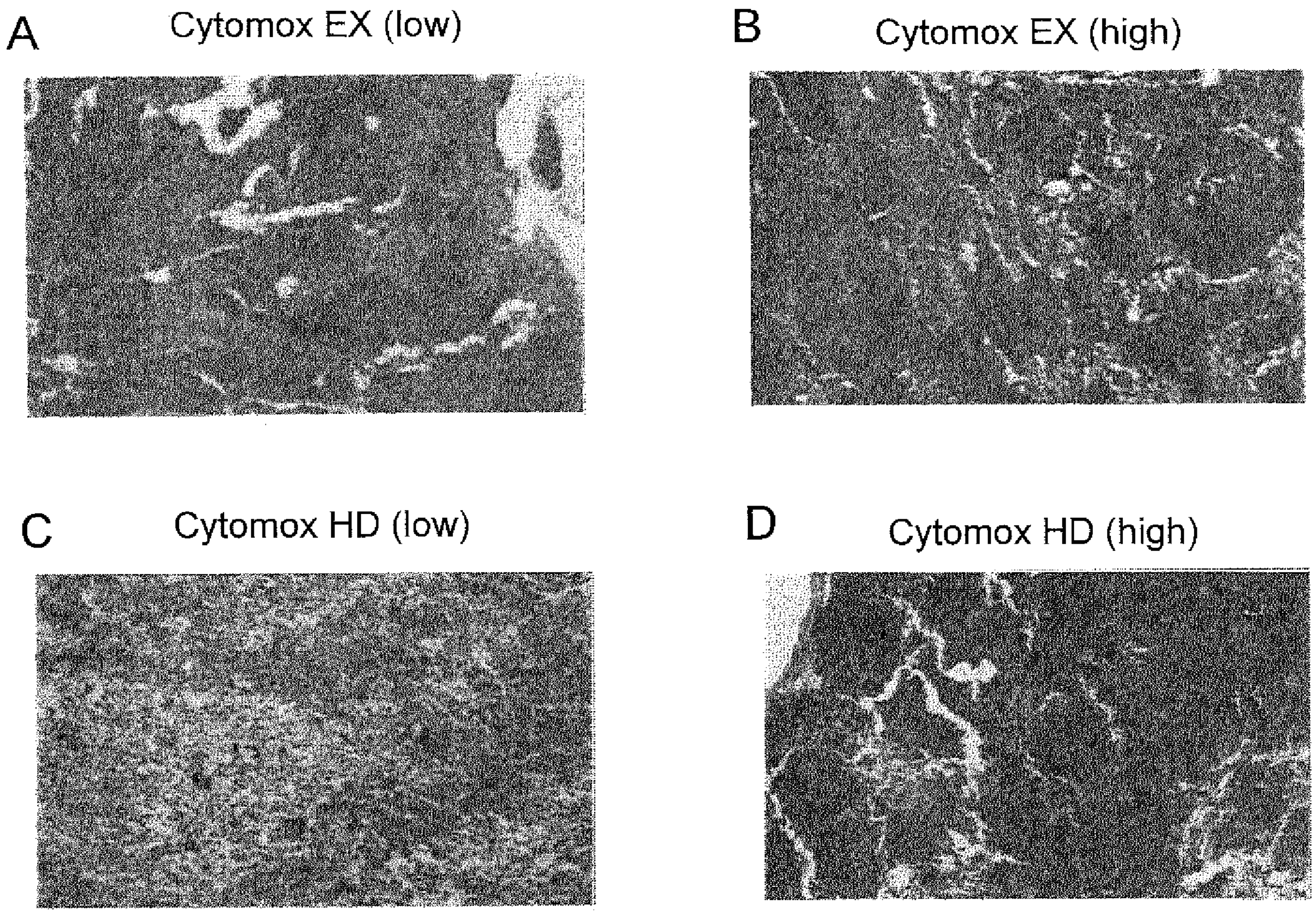
A
Cytomox EX (low)
B
Cytomox EX (high)
C
Cytomox HD (low)
D
Cytomox HD (high)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011080271 A
- WO 2011000551 A
- WO 2009057165 A
- US 61779556 B
- US 61894563 B


### Non-patent literature cited in the description


- **FENG et al.** *Cancer Res.,* 2003, vol. 63, 7356-7364
- **LAU et al.** *EMBO Rep.,* 2006, vol. 7, 425-430
- **FENG et al.** *Mol. Cell. Biochem.,* 2008, vol. 311, 189-197
- **CROYLE et al.** *J. Virol.,* 2004, vol. 78, 912-921
- **ADACHI A et al.** *J. Virol.,* 1986, 284-291
- **TAKAKURA et al.** *Cancer Res.,* 1999, 551-557
- **ROSSIO JL.** *J. Virol.,* 1998, vol. 72, 7992-8001
- **SAUTER et al.** *Cell,* 2010, vol. 141, 392-398
- **GONZALEZ, S. et al.** *Nature,* 2006, 702-706
- **MEDEMA et al.** *Proc. Natl. Acad. Sci. U S A.,* 1995, vol. 92 (14), 6289-6293

SEQUENCE LISTING

<110> Li, Zhong Katsura, Misako

<120> Microvesicles and method for producing the same

<130> PH-5503-PCT

<150> US 61/779,556
<151> 2013-03-13

<150> US 61/894,563
<151> 2013-10-23

<160> 27

<170> PatentIn version 3.4

<210> 1
<211> 1389
<212> DNA
<213> Homo sapiens

<220>
<223> The nucleotide sequence of the TERT gene promoter

<400> 1

```
gacaattcac aaacacagcc ctttaaaaag gcttagggat cactaagggg atttctagaa      60
gagcgacctg taatcctaag tatttacaag acgaggctaa cctccagcga gcgtgacagc     120
ccagggaggg tgcgaggcct gttcaaatgc tagctccata aataaagcaa tttcctccgg     180
cagtttctga aagtaggaaa ggttacattt aaggttgcgt ttgttagcat ttcagtgttt     240
gccgacctca gctacagcat ccctgcaagg cctcgggaga cccagaagtt tctcgccccc     300
ttagatccaa acttgagcaa cccggagtct ggattcctgg gaagtcctca gctgtcctgc     360
ggttgtgccg gggccccagg tctggagggg accagtggcc gtgtggcttc tactgctggg     420
ctggaagtcg ggcctcctag ctctgcagtc cgaggcttgg agccaggtgc ctggaccccg     480
aggctgccct ccaccctgtg cgggcgggat gtgaccagat gttggcctca tctgccagac     540
agagtgccgg ggcccagggt caaggccgtt gtggctggtg tgaggcgccc ggtgcgcggc     600
cagcaggagc gcctggctcc atttcccacc ctttctcgac gggaccgccc cggtgggtga     660
ttaacagatt tggggtggtt tgctcatggt ggggacccct cgccgcctga gaacctgcaa     720
agagaaatga cgggcctgtg tcaaggagcc caagtcgcgg ggaagtgttg cagggaggca     780
ctccgggagg tcccgcgtgc ccgtccaggg agcaatgcgt cctcgggttc gtccccagcc     840
gcgtctacgc gcctccgtcc tccccttcac gtccggcatt cgtggtgccc ggagcccgac     900
gccccgcgtc cggacctgga ggcagccctg ggtctccgga tcaggccagc ggccaaaggg     960
tcgccgcacg cacctgttcc cagggcctcc acatcatggc ccctccctcg ggttacccca    1020
cagcctaggc cgattcgacc tctctccgct ggggccctcg ctggcgtccc tgcaccctgg    1080
```

gagcgcgagc ggcgcgcggg cggggaagcg cggcccagac cccgggtcc gcccggagca 1140

gctgcgctgt cggggccagg ccgggctccc agtggattcg cgggcacaga cgcccaggac 1200

cgcgctcccc acgtggcgga gggactgggg acccgggcac ccgtcctgcc ccttcacctt 1260

ccagctccgc ctcctccgcg cggaccccgc cccgtcccga cccctcccgg gtccccggcc 1320

cagcccccctc cgggccctcc cagcccctcc ccttcctttc cgcggccccg ccctctcctc 1380

gcggcgcga 1389

<210> 2
<211> 429
<212> DNA
<213> Homo sapiens

<220>
<223> The nucleotide sequence of 5’ part of TERT transcribed region

<400> 2

gtttcaggca gcgctgcgtc ctgctgcgca cgtgggaagc cctggccccg gccacccccg 60

cgatgccgcg cgctccccgc tgccgagccg tgcgctccct gctgcgcagc cactaccgcg 120

aggtgctgcc gctggccacg ttcgtgcggc gcctggggcc ccagggctgg cggctggtgc 180

agcgcgggga cccggcggct ttccgcgcgc tggtggccca gtgcctggtg tgcgtgccct 240

gggacgcacg gccgcccccc gccgcccct ccttccgcca ggtgggcctc cccggggtcg 300

gcgtccggct ggggttgagg gcggccgggg ggaaccagcg acatgcggag agcagcgcag 360

gcgactcagg gcgcttcccc cgcaggtgtc ctgcctgaag gagctggtgg cccgagtgct 420

gcagaggct 429

<210> 3
<211> 1818
<212> DNA
<213> Homo sapiens

<220>
<223> The nucleotide sequence comprising the TERT gene promoter and 5’ part of TERT transcribed region

<400> 3

```
gacaattcac aaacacagcc ctttaaaaag gcttagggat cactaagggg atttctagaa     60
gagcgacctg taatcctaag tatttacaag acgaggctaa cctccagcga gcgtgacagc    120
ccagggaggg tgcgaggcct gttcaaatgc tagctccata aataaagcaa tttcctccgg    180
cagtttctga aagtaggaaa ggttacattt aaggttgcgt ttgttagcat ttcagtgttt    240
gccgacctca gctacagcat ccctgcaagg cctcgggaga cccagaagtt tctcgccccc    300
ttagatccaa acttgagcaa cccggagtct ggattcctgg gaagtcctca gctgtcctgc    360
ggttgtgccg gggccccagg tctggagggg accagtggcc gtgtggcttc tactgctggg    420
```

```
ctggaagtcg ggcctcctag ctctgcagtc cgaggcttgg agccaggtgc ctggaccccg     480
aggctgccct ccaccctgtg cgggcgggat gtgaccagat gttggcctca tctgccagac     540
agagtgccgg ggcccagggt caaggccgtt gtggctggtg tgaggcgccc ggtgcgcggc     600
cagcaggagc gcctggctcc atttcccacc ctttctcgac gggaccgccc cggtgggtga     660
ttaacagatt tggggtggtt tgctcatggt ggggacccct cgccgcctga gaacctgcaa     720
agagaaatga cgggcctgtg tcaaggagcc caagtcgcgg ggaagtgttg cagggaggca     780
ctccgggagg tcccgcgtgc ccgtccaggg agcaatgcgt cctcgggttc gtccccagcc     840
gcgtctacgc gcctccgtcc tccccttcac gtccggcatt cgtggtgccc ggagcccgac     900
gccccgcgtc cggacctgga ggcagccctg ggtctccgga tcaggccagc ggccaaaggg     960
tcgccgcacg cacctgttcc cagggcctcc acatcatggc ccctccctcg ggttacccca    1020
cagcctaggc cgattcgacc tctctccgct ggggccctcg ctggcgtccc tgcaccctgg    1080
gagcgcgagc ggcgcgcggg cggggaagcg cggcccagac cccgggtcc gcccggagca    1140
gctgcgctgt cggggccagg ccgggctccc agtggattcg cgggcacaga cgcccaggac    1200
cgcgctcccc acgtggcgga gggactgggg acccgggcac ccgtcctgcc ccttcacctt    1260
ccagctccgc ctcctccgcg cggaccccgc cccgtcccga cccctcccgg gtccccggcc    1320
cagccccctc cgggccctcc cagcccctcc ccttcctttc cgcggccccg ccctctcctc    1380
gcggcgcgag tttcaggcag cgctgcgtcc tgctgcgcac gtgggaagcc ctggccccgg    1440
ccacccccgc gatgccgcgc gctccccgct gccgagccgt gcgctccctg ctgcgcagcc    1500
actaccgcga ggtgctgccg ctggccacgt tcgtgcggcg cctggggccc cagggctggc    1560
ggctggtgca gcgcggggac ccggcggctt tccgcgcgct ggtggcccag tgcctggtgt    1620
gcgtgccctg ggacgcacgg ccgccccccg ccgccccctc cttccgccag gtgggcctcc    1680
ccggggtcgg cgtccggctg gggttgaggg cggccggggg gaaccagcga catgcggaga    1740
gcagcgcagg cgactcaggg cgcttccccc gcaggtgtcc tgcctgaagg agctggtggc    1800
ccgagtgctg cagaggct                                                 1818
```

<210> 4
<211> 14825
<212> DNA
<213> Artificial sequence

<220>
<223> pNL4-3

<400> 4

```
tggaagggct aatttggtcc caaaaaagac aagagatcct tgatctgtgg atctaccaca      60
cacaaggcta cttccctgat tggcagaact acacaccagg gccagggatc agatatccac     120
```

```
tgacctttgg atggtgcttc aagttagtac cagttgaacc agagcaagta gaagaggcca    180
atgaaggaga gaacaacagc ttgttacacc ctatgagcca gcatgggatg gaggacccgg    240
agggagaagt attagtgtgg aagtttgaca gcctcctagc atttcgtcac atggcccgag    300
agctgcatcc ggagtactac aaagactgct gacatcgagc tttctacaag ggactttccg    360
ctggggactt tccagggagg tgtggcctgg gcgggactgg ggagtggcga gccctcagat    420
gctacatata agcagctgct ttttgcctgt actgggtctc tctggttaga ccagatctga    480
gcctgggagc tctctggcta actagggaac ccactgctta agcctcaata aagcttgcct    540
tgagtgctca aagtagtgtg tgcccgtctg ttgtgtgact ctggtaacta gagatccctc    600
agaccctttt agtcagtgtg gaaaatctct agcagtggcg cccgaacagg gacttgaaag    660
cgaaagtaaa gccagaggag atctctcgac gcaggactcg gcttgctgaa gcgcgcacgg    720
caagaggcga ggggcggcga ctggtgagta cgccaaaaat tttgactagc ggaggctaga    780
aggagagaga tgggtgcgag agcgtcggta ttaagcgggg gagaattaga taaatgggaa    840
aaaattcggt taaggccagg gggaaagaaa caatataaac taaaacatat agtatgggca    900
agcagggagc tagaacgatt cgcagttaat cctggccttt tagagacatc agaaggctgt    960
agacaaatac tgggacagct acaaccatcc cttcagacag gatcagaaga acttagatca   1020
ttatataata caatagcagt cctctattgt gtgcatcaaa ggatagatgt aaaagacacc   1080
aaggaagcct tagataagat agaggaagag caaaacaaaa gtaagaaaaa ggcacagcaa   1140
gcagcagctg acacaggaaa caacagccag gtcagccaaa attaccctat agtgcagaac   1200
ctccaggggc aaatggtaca tcaggccata tcacctagaa ctttaaatgc atgggtaaaa   1260
gtagtagaag agaaggcttt cagcccagaa gtaataccca tgttttcagc attatcagaa   1320
ggagccaccc cacaagattt aaataccatg ctaaacacag tggggggaca tcaagcagcc   1380
atgcaaatgt taaaagagac catcaatgag gaagctgcag aatgggatag attgcatcca   1440
gtgcatgcag ggcctattgc accaggccag atgagagaac caaggggaag tgacatagca   1500
ggaactacta gtacccttca ggaacaaata ggatggatga cacataatcc acctatccca   1560
gtaggagaaa tctataaaag atggataatc ctgggattaa ataaaatagt aagaatgtat   1620
agccctacca gcattctgga cataagacaa ggaccaaagg aaccctttag agactatgta   1680
gaccgattct ataaaactct aagagccgag caagcttcac aagaggtaaa aaattggatg   1740
acagaaacct tgttggtcca aaatgcgaac ccagattgta agactatttt aaaagcattg   1800
ggaccaggag cgacactaga agaaatgatg acagcatgtc agggagtggg gggacccggc   1860
cataaagcaa gagttttggc tgaagcaatg agccaagtaa caaatccagc taccataatg   1920
atacagaaag gcaattttag gaaccaaaga aagactgtta agtgtttcaa ttgtggcaaa   1980
gaagggcaca tagccaaaaa ttgcagggcc cctaggaaaa agggctgttg gaaatgtgga   2040
```

aaggaaggac accaaatgaa agattgtact gagagacagg ctaattttt agggaagatc 2100
tggccttccc acaagggaag gccagggaat tttcttcaga gcagaccaga gccaacagcc 2160
ccaccagaag agagcttcag gtttggggaa gagacaacaa ctccctctca gaagcaggag 2220
ccgatagaca aggaactgta tcctttagct tccctcagat cactctttgg cagcgacccc 2280
tcgtcacaat aaagataggg gggcaattaa aggaagctct attagataca ggagcagatg 2340
atacagtatt agaagaaatg aatttgccag gaagatggaa accaaaaatg atagggggaa 2400
ttggaggttt tatcaaagta agacagtatg atcagatact catagaaatc tgcggacata 2460
aagctatagg tacagtatta gtaggaccta cacctgtcaa cataattgga agaaatctgt 2520
tgactcagat tggctgcact ttaaattttc ccattagtcc tattgagact gtaccagtaa 2580
aattaaagcc aggaatggat ggcccaaaag ttaaacaatg gccattgaca gaagaaaaaa 2640
taaaagcatt agtagaaatt tgtacagaaa tggaaaagga aggaaaaatt tcaaaaattg 2700
ggcctgaaaa tccatacaat actccagtat ttgccataaa gaaaaaagac agtactaaat 2760
ggagaaaatt agtagatttc agagaactta ataagagaac tcaagatttc tgggaagttc 2820
aattaggaat accacatcct gcagggttaa aacagaaaaa atcagtaaca gtactggatg 2880
tgggcgatgc atatttttca gttcccttag ataaagactt caggaagtat actgcattta 2940
ccatacctag tataaacaat gagacaccag ggattagata tcagtacaat gtgcttccac 3000
agggatggaa aggatcacca gcaatattcc agtgtagcat gacaaaaatc ttagagcctt 3060
ttagaaaaca aaatccagac atagtcatct atcaatacat ggatgatttg tatgtaggat 3120
ctgacttaga aatagggcag catagaacaa aaatagagga actgagacaa catctgttga 3180
ggtggggatt taccacacca gacaaaaaac atcagaaaga acctccattc ctttggatgg 3240
gttatgaact ccatcctgat aaatggacag tacagcctat agtgctgcca gaaaaggaca 3300
gctggactgt caatgacata cagaaattag tgggaaaatt gaattgggca agtcagattt 3360
atgcagggat taaagtaagg caattatgta aacttcttag gggaaccaaa gcactaacag 3420
aagtagtacc actaacagaa gaagcagagc tagaactggc agaaaacagg gagattctaa 3480
aagaaccggt acatggagtg tattatgacc catcaaaaga cttaatagca gaaatacaga 3540
agcaggggca aggccaatgg acatatcaaa tttatcaaga gccatttaaa aatctgaaaa 3600
caggaaagta tgcaagaatg aagggtgccc acactaatga tgtgaaacaa ttaacagagg 3660
cagtacaaaa aatagccaca gaaagcatag taatatgggg aaagactcct aaatttaaat 3720
tacccataca aaaggaaaca tgggaagcat ggtggacaga gtattggcaa gccacctgga 3780
ttcctgagtg ggagtttgtc aatacccctc ccttagtgaa gttatggtac cagttagaga 3840
aagaacccat aataggagca gaaactttct atgtagatgg ggcagccaat agggaaacta 3900

aattaggaaa agcaggatat gtaactgaca gaggaagaca aaaagttgtc cccctaacgg 3960
acacaacaaa tcagaagact gagttacaag caattcatct agctttgcag gattcgggat 4020
tagaagtaaa catagtgaca gactcacaat atgcattggg aatcattcaa gcacaaccag 4080
ataagagtga atcagagtta gtcagtcaaa taatagagca gttaataaaa aaggaaaaag 4140
tctacctggc atgggtacca gcacacaaag gaattggagg aaatgaacaa gtagataaat 4200
tggtcagtgc tggaatcagg aaagtactat ttttagatgg aatagataag gcccaagaag 4260
aacatgagaa atatcacagt aattggagag caatggctag tgattttaac ctaccacctg 4320
tagtagcaaa agaaatagta gccagctgtg ataaatgtca gctaaaaggg gaagccatgc 4380
atggacaagt agactgtagc ccaggaatat ggcagctaga ttgtacacat ttagaaggaa 4440
aagttatctt ggtagcagtt catgtagcca gtggatatat agaagcagaa gtaattccag 4500
cagagacagg gcaagaaaca gcatacttcc tcttaaaatt agcaggaaga tggccagtaa 4560
aaacagtaca tacagacaat ggcagcaatt tcaccagtac tacagttaag gccgcctgtt 4620
ggtgggcggg gatcaagcag gaatttggca ttccctacaa tccccaaagt caaggagtaa 4680
tagaatctat gaataaagaa ttaaagaaaa ttataggaca ggtaagagat caggctgaac 4740
atcttaagac agcagtacaa atggcagtat tcatccacaa ttttaaaaga aaagggggga 4800
ttggggggta cagtgcaggg gaaagaatag tagacataat agcaacagac atacaaacta 4860
aagaattaca aaaacaaatt acaaaaattc aaaattttcg ggtttattac agggacagca 4920
gagatccagt ttggaaagga ccagcaaagc tcctctggaa aggtgaaggg gcagtagtaa 4980
tacaagataa tagtgacata aaagtagtgc caagaagaaa agcaaagatc atcagggatt 5040
atggaaaaca gatggcaggt gatgattgtg tggcaagtag acaggatgag gattaacaca 5100
tggaaaagat tagtaaaaca ccatatgtat atttcaagga aagctaagga ctggttttat 5160
agacatcact atgaaagtac taatccaaaa ataagttcag aagtacacat cccactaggg 5220
gatgctaaat tagtaataac aacatattgg ggtctgcata caggagaaag agactggcat 5280
ttgggtcagg gagtctccat agaatggagg aaaaagagat atagcacaca agtagaccct 5340
gacctagcag accaactaat tcatctgcac tattttgatt gtttttcaga atctgctata 5400
agaaatacca tattaggacg tatagttagt cctaggtgtg aatatcaagc aggacataac 5460
aaggtaggat ctctacagta cttggcacta gcagcattaa taaaaccaaa acagataaag 5520
ccacctttgc ctagtgttag gaaactgaca gaggacagat ggaacaagcc ccagaagacc 5580
aagggccaca gagggagcca tacaatgaat ggacactaga gcttttagag gaacttaaga 5640
gtgaagctgt tagacatttt cctaggatat ggctccataa cttaggacaa catatctatg 5700
aaacttacgg ggatacttgg gcaggagtgg aagccataat aagaattctg caacaactgc 5760
tgtttatcca tttcagaatt gggtgtcgac atagcagaat aggcgttact cgacagagga 5820

gagcaagaaa tggagccagt agatcctaga ctagagccct ggaagcatcc aggaagtcag 5880
cctaaaactg cttgtaccaa ttgctattgt aaaaagtgtt gctttcattg ccaagtttgt 5940
ttcatgacaa aagccttagg catctcctat ggcaggaaga agcggagaca gcgacgaaga 6000
gctcatcaga acagtcagac tcatcaagct tctctatcaa agcagtaagt agtacatgta 6060
atgcaaccta taatagtagc aatagtagca ttagtagtag caataataat agcaatagtt 6120
gtgtggtcca tagtaatcat agaatatagg aaaatattaa gacaaagaaa aatagacagg 6180
ttaattgata gactaataga aagagcagaa gacagtggca atgagagtga aggagaagta 6240
tcagcacttg tggagatggg ggtggaaatg gggcaccatg ctccttggga tattgatgat 6300
ctgtagtgct acagaaaaat tgtgggtcac agtctattat ggggtacctg tgtggaagga 6360
agcaaccacc actctatttt gtgcatcaga tgctaaagca tatgatacag aggtacataa 6420
tgtttgggcc acacatgcct gtgtacccac agaccccaac ccacaagaag tagtattggt 6480
aaatgtgaca gaaaatttta acatgtggaa aaatgacatg gtagaacaga tgcatgagga 6540
tataatcagt ttatgggatc aaagcctaaa gccatgtgta aaattaaccc cactctgtgt 6600
tagtttaaag tgcactgatt tgaagaatga tactaatacc aatagtagta gcgggagaat 6660
gataatggag aaaggagaga taaaaaactg ctctttcaat atcagcacaa gcataagaga 6720
taaggtgcag aaagaatatg cattctttta taaacttgat atagtaccaa tagataatac 6780
cagctatagg ttgataagtt gtaacacctc agtcattaca caggcctgtc caaaggtatc 6840
ctttgagcca attcccatac attattgtgc cccggctggt tttgcgattc taaaatgtaa 6900
taataagacg ttcaatggaa caggaccatg tacaaatgtc agcacagtac aatgtacaca 6960
tggaatcagg ccagtagtat caactcaact gctgttaaat ggcagtctag cagaagaaga 7020
tgtagtaatt agatctgcca atttcacaga caatgctaaa accataatag tacagctgaa 7080
cacatctgta gaaattaatt gtacaagacc caacaacaat acaagaaaaa gtatccgtat 7140
ccagagggga ccagggagag catttgttac aataggaaaa ataggaaata tgagacaagc 7200
acattgtaac attagtagag caaaatggaa tgccacttta aaacagatag ctagcaaatt 7260
aagagaacaa tttggaaata ataaaacaat aatctttaag caatcctcag gaggggaccc 7320
agaaattgta acgcacagtt ttaattgtgg aggggaattt ttctactgta attcaacaca 7380
actgtttaat agtacttggt ttaatagtac ttggagtact gaagggtcaa ataacactga 7440
aggaagtgac acaatcacac tcccatgcag aataaaacaa tttataaaca tgtggcagga 7500
agtaggaaaa gcaatgtatg cccctcccat cagtggacaa attagatgtt catcaaatat 7560
tactgggctg ctattaacaa gagatggtgg taataacaac aatgggtccg agatcttcag 7620
acctggagga ggcgatatga gggacaattg gagaagtgaa ttatataaat ataaagtagt 7680

aaaaattgaa ccattaggag tagcacccac caaggcaaag agaagagtgg tgcagagaga 7740
aaaaagagca gtgggaatag gagctttgtt ccttgggttc ttgggagcag caggaagcac 7800
tatgggcgca gcgtcaatga cgctgacggt acaggccaga caattattgt ctgatatagt 7860
gcagcagcag aacaatttgc tgagggctat tgaggcgcaa cagcatctgt tgcaactcac 7920
agtctggggc atcaaacagc tccaggcaag aatcctggct gtggaaagat acctaaagga 7980
tcaacagctc ctggggattt ggggttgctc tggaaaactc atttgcacca ctgctgtgcc 8040
ttggaatgct agttggagta ataaatctct ggaacagatt tggaataaca tgacctggat 8100
ggagtgggac agagaaatta acaattacac aagcttaata cactccttaa ttgaagaatc 8160
gcaaaaccag caagaaaaga atgaacaaga attattggaa ttagataaat gggcaagttt 8220
gtggaattgg tttaacataa caaattggct gtggtatata aaattattca taatgatagt 8280
aggaggcttg gtaggtttaa gaatagtttt tgctgtactt tctatagtga atagagttag 8340
gcagggatat tcaccattat cgtttcagac ccacctccca atcccgaggg gacccgacag 8400
gcccgaagga atagaagaag aaggtggaga gagagacaga gacagatcca ttcgattagt 8460
gaacggatcc ttagcactta tctgggacga tctgcggagc ctgtgcctct tcagctacca 8520
ccgcttgaga gacttactct tgattgtaac gaggattgtg gaacttctgg gacgcagggg 8580
gtgggaagcc ctcaaatatt ggtggaatct cctacagtat tggagtcagg aactaaagaa 8640
tagtgctgtt aacttgctca atgccacagc catagcagta gctgagggga cagatagggt 8700
tatagaagta ttacaagcag cttatagagc tattcgccac atacctagaa gaataagaca 8760
gggcttggaa aggattttgc tataagatgg gtggcaagtg gtcaaaaagt agtgtgattg 8820
gatggcctgc tgtaagggaa agaatgagac gagctgagcc agcagcagat ggggtgggag 8880
cagtatctcg agacctagaa aaacatggag caatcacaag tagcaataca gcagctaaca 8940
atgctgcttg tgcctggcta gaagcacaag aggaggaaga ggtgggtttt ccagtcacac 9000
ctcaggtacc tttaagacca atgacttaca aggcagctgt agatcttagc cactttttaa 9060
aagaaaaggg gggactggaa gggctaattc actcccaaag aagacaagat atccttgatc 9120
tgtggatcta ccacacacaa ggctacttcc ctgattggca gaactacaca ccagggccag 9180
gggtcagata tccactgacc tttggatggt gctacaagct agtaccagtt gagccagata 9240
aggtagaaga ggccaataaa ggagagaaca ccagcttgtt acaccctgtg agcctgcatg 9300
gaatggatga ccctgagaga gaagtgttag agtggaggtt tgacagccgc ctagcatttc 9360
atcacgtggc ccgagagctg catccggagt acttcaagaa ctgctgacat cgagcttgct 9420
acaagggact ttccgctggg gactttccag ggaggcgtgg cctgggcggg actggggagt 9480
ggcgagccct cagatgctgc atataagcag ctgctttttg cctgtactgg gtctctctgg 9540
ttagaccaga tctgagcctg ggagctctct ggctaactag ggaacccact gcttaagcct 9600

caataaagct tgccttgagt gcttcaagta gtgtgtgccc gtctgttgtg tgactctggt 9660
aactagagat ccctcagacc cttttagtca gtgtggaaaa tctctagcac ccaggaggta 9720
gaggttgcag tgagccaaga tcgcgccact gcattccagc ctgggcaaga aaacaagact 9780
gtctaaaata ataataataa gttaagggta ttaaatatat ttatacatgg aggtcataaa 9840
aatatatata tttgggctgg gcgcagtggc tcacacctgc gcccggccct ttgggaggcc 9900
gaggcaggtg gatcacctga gtttgggagt tccagaccag cctgaccaac atggagaaac 9960
cccttctctg tgtattttta gtagatttta ttttatgtgt attttattca caggtatttc 10020
tggaaaactg aaactgtttt tcctctactc tgataccaca agaatcatca gcacagagga 10080
agacttctgt gatcaaatgt ggtgggagag ggaggttttc accagcacat gagcagtcag 10140
ttctgccgca gactcggcgg gtgtccttcg gttcagttcc aacaccgcct gcctggagag 10200
aggtcagacc acagggtgag ggctcagtcc ccaagacata aacacccaag acataaacac 10260
ccaacaggtc caccccgcct gctgcccagg cagagccgat tcaccaagac gggaattagg 10320
atagagaaag agtaagtcac acagagccgg ctgtgcggga gaacggagtt ctattatgac 10380
tcaaatcagt ctccccaagc attcggggat cagagttttt aaggataact tagtgtgtag 10440
ggggccagtg agttggagat gaaagcgtag ggagtcgaag gtgtcctttt gcgccgagtc 10500
agttcctggg tgggggccac aagatcggat gagccagttt atcaatccgg gggtgccagc 10560
tgatccatgg agtgcagggt ctgcaaaata tctcaagcac tgattgatct taggttttac 10620
aatagtgatg ttaccccagg aacaatttgg ggaaggtcag aatcttgtag cctgtagctg 10680
catgactcct aaaccataat ttcttttttg tttttttttt tttattttg agacagggtc 10740
tcactctgtc acctaggctg gagtgcagtg gtgcaatcac agctcactgc agcctcaacg 10800
tcgtaagctc aagcgatcct cccacctcag cctgcctggt agctgagact acaagcgacg 10860
ccccagttaa tttttgtatt tttggtagag gcagcgtttt gccgtgtggc cctggctggt 10920
ctcgaactcc tgggctcaag tgatccagcc tcagcctccc aaagtgctgg gacaaccggg 10980
gccagtcact gcacctggcc ctaaaccata atttctaatc ttttggctaa tttgttagtc 11040
ctacaaaggc agtctagtcc ccaggcaaaa aggggggtttg tttcgggaaa gggctgttac 11100
tgtctttgtt tcaaactata aactaagttc ctcctaaact tagttcggcc tacacccagg 11160
aatgaacaag gagagcttgg aggttagaag cacgatggaa ttggttaggt cagatctctt 11220
tcactgtctg agttataatt ttgcaatggt ggttcaaaga ctgcccgctt ctgacaccag 11280
tcgctgcatt aatgaatcgg ccaacgcgcg gggagaggcg gtttgcgtat tgggcgctct 11340
tccgcttcct cgctcactga ctcgctgcgc tcggtcgttc ggctgcggcg agcggtatca 11400
gctcactcaa aggcggtaat acggttatcc acagaatcag gggataacgc aggaaagaac 11460

```
atgtgagcaa aaggccagca aaaggccagg aaccgtaaaa aggccgcgtt gctggcgttt  11520
ttccataggc tccgcccccc tgacgagcat cacaaaaatc gacgctcaag tcagaggtgg  11580
cgaaacccga caggactata aagataccag gcgtttcccc ctggaagctc cctcgtgcgc  11640
tctcctgttc cgaccctgcc gcttaccgga tacctgtccg cctttctccc ttcgggaagc  11700
gtggcgcttt ctcatagctc acgctgtagg tatctcagtt cggtgtaggt cgttcgctcc  11760
aagctgggct gtgtgcacga accccccgtt cagcccgacc gctgcgcctt atccggtaac  11820
tatcgtcttg agtccaaccc ggtaagacac gacttatcgc cactggcagc agccactggt  11880
aacaggatta gcagagcgag gtatgtaggc ggtgctacag agttcttgaa gtggtggcct  11940
aactacggct acactagaag aacagtattt ggtatctgcg ctctgctgaa gccagttacc  12000
ttcggaaaaa gagttggtag ctcttgatcc ggcaaacaaa ccaccgctgg tagcggtggt  12060
tttttgttt gcaagcagca gattacgcgc agaaaaaaag gatctcaaga agatcctttg  12120
atcttttcta cggggtctga cgctcagtgg aacgaaaact cacgttaagg gattttggtc  12180
atgagattat caaaaaggat cttcacctag atccttttaa attaaaaatg aagttttaaa  12240
tcaatctaaa gtatatatga gtaaacttgg tctgacagtt accaatgctt aatcagtgag  12300
gcacctatct cagcgatctg tctatttcgt tcatccatag ttgcctgact ccccgtcgtg  12360
tagataacta cgatacggga gggcttacca tctggcccca gtgctgcaat gataccgcga  12420
gacccacgct caccggctcc agatttatca gcaataaacc agccagccgg aagggccgag  12480
cgcagaagtg gtcctgcaac tttatccgcc tccatccagt ctattaattg ttgccgggaa  12540
gctagagtaa gtagttcgcc agttaatagt ttgcgcaacg ttgttgccat tgctacaggc  12600
atcgtggtgt cacgctcgtc gtttggtatg gcttcattca gctccggttc ccaacgatca  12660
aggcgagtta catgatcccc catgttgtgc aaaaaagcgg ttagctcctt cggtcctccg  12720
atcgttgtca gaagtaagtt ggccgcagtg ttatcactca tggttatggc agcactgcat  12780
aattctctta ctgtcatgcc atccgtaaga tgcttttctg tgactggtga gtactcaacc  12840
aagtcattct gagaatagtg tatgcggcga ccgagttgct cttgcccggc gtcaatacgg  12900
gataataccg cgccacatag cagaacttta aaagtgctca tcattggaaa acgttcttcg  12960
gggcgaaaac tctcaaggat cttaccgctg ttgagatcca gttcgatgta acccactcgt  13020
gcacccaact gatcttcagc atcttttact ttcaccagcg tttctgggtg agcaaaaaca  13080
ggaaggcaaa atgccgcaaa aaagggaata agggcgacac ggaaatgttg aatactcata  13140
ctcttccttt ttcaatatta ttgaagcatt tatcagggtt attgtctcat gagcggatac  13200
atatttgaat gtatttagaa aaataaacaa ataggggttc cgcgcacatt tccccgaaaa  13260
gtgccacctg acgtctaaga aaccattatt atcatgacat taacctataa aaataggcgt  13320
atcacgaggc cctttcgtct cgcgcgtttc ggtgatgacg gtgaaaacct ctgacacatg  13380
```

cagctcccgg agacggtcac agcttgtctg taagcggatg ccgggagcag acaagcccgt 13440

cagggcgcgt cagcgggtgt tggcgggtgt cggggctggc ttaactatgc ggcatcagag 13500

cagattgtac tgagagtgca ccatatgcgg tgtgaaatac cgcacagatg cgtaaggaga 13560

aaataccgca tcaggcgcca ttcgccattc aggctgcgca actgttggga agggcgatcg 13620

gtgcgggcct cttcgctatt acgccagggg aggcagagat tgcagtaagc tgagatcgca 13680

gcactgcact ccagcctggg cgacagagta agactctgtc tcaaaaataa aataaataaa 13740

tcaatcagat attccaatct tttcctttat ttatttattt attttctatt ttggaaacac 13800

agtccttcct tattccagaa ttacacatat attctatttt tctttatatg ctccagtttt 13860

ttttagacct tcacctgaaa tgtgtgtata caaaatctag gccagtccag cagagcctaa 13920

aggtaaaaaa taaaataata aaaaataaat aaaatctagc tcactccttc acatcaaaat 13980

ggagatacag ctgttagcat taaataccaa ataacccatc ttgtcctcaa taattttaag 14040

cgcctctctc caccacatct aactcctgtc aaaggcatgt gccccttccg ggcgctctgc 14100

tgtgctgcca accaactggc atgtggactc tgcagggtcc ctaactgcca agccccacag 14160

tgtgccctga ggctgcccct tccttctagc ggctgccccc actcggcttt gctttcccta 14220

gtttcagtta cttgcgttca gccaaggtct gaaactaggt gcgcacagag cggtaagact 14280

gcgagagaaa gagaccagct ttacaggggg tttatcacag tgcaccctga cagtcgtcag 14340

cctcacaggg ggtttatcac attgcaccct gacagtcgtc agcctcacag gggtttatc 14400

acagtgcacc cttacaatca ttccatttga ttcacaattt ttttagtctc tactgtgcct 14460

aacttgtaag ttaaatttga tcagaggtgt gttcccagag gggaaaacag tatatacagg 14520

gttcagtact atcgcatttc aggcctccac ctgggtcttg gaatgtgtcc cccgaggggt 14580

gatgactacc tcagttggat ctccacaggt cacagtgaca caagataacc aagacacctc 14640

ccaaggctac cacaatgggc cgccctccac gtgcacatgg ccggaggaac tgccatgtcg 14700

gaggtgcaag cacacctgcg catcagagtc cttggtgtgg agggagggac cagcgcagct 14760

tccagccatc cacctgatga acagaaccta gggaaagccc cagttctact tacaccagga 14820

aaggc 14825

<210> 5
<211> 8733
<212> DNA
<213> Artificial sequence

<220>
<223> The nucleotide sequence from 5' LTR to 3' LTR in pTHTK

<400> 5

tggaagggct aatttggtcc caaaaaagac aagagatcct tgatctgtgg atctaccaca 60

cacaaggcta cttccctgat tggcagaact acacaccagg gccagggatc agatatccac 120
tgacctttgg atggtgcttc aagttagtac cagttgaacc agagcaagta gaagaggcca 180
atgaaggaga gaacaacagc ttgttacacc ctatgagcca gcatgggatg gaggacccgg 240
agggagaagt attagtgtgg aagtttgaca gcctcctagc atttcgtcac atggcccgag 300
agctgcatcc ggagtactac aaagactgct gacatcgagc tttctacaag ggactttccg 360
ctggggactt tccagggagg tgtggcctgg gcgggactgg ggagtggcga gccctcagat 420
gctacatata agcagctgct ttttgcctgt actgggtctc tctggttaga ccagatctga 480
gcctgggagc tctctggcta actagggaac ccactgctta agcctcaata aagcttgcct 540
tgagtgctca aagtagtgtg tgcccgtctg ttgtgtgact ctggtaacta gagatccctc 600
agaccctttt agtcagtgtg gaaaatctct agcagtggcg cccgaacagg gacttgaaag 660
cgaaagtaaa gccagaggag atctctcgac gcaggactcg gcttgctgaa gcgcgcacgg 720
caagaggcga ggggcggcga ctggtgagta cgccaaaaat tttgactagc ggaggctaga 780
aggagagaga tgggtgcgag agcgtcggta ttaagcgggg gagaattaga taaatgggaa 840
aaaattcggt taaggccagg gggaaagaaa caatataaac taaaacatat agtatgggca 900
agcagggagc tagaacgatt cgcagttaat cctggccttt tagagacatc agaaggctgt 960
agacaaatac tgggacagct acaaccatcc cttcagacag gatcagaaga acttagatca 1020
ttatataata caatagcagt cctctattgt gtgcatcaaa ggatagatgt aaaagacacc 1080
aaggaagcct tagataagat agaggaagag caaaacaaaa gtaagaaaaa ggcacagcaa 1140
gcagcagctg acacaggaaa caacagccag gtcagccaaa attaccctat agtgcagaac 1200
ctccaggggc aaatggtaca tcaggccata tcacctagaa ctttaaatgc atgggtaaaa 1260
gtagtagaag agaaggcttt cagcccagaa gtaataccca tgttttcagc attatcagaa 1320
ggagccaccc cacaagattt aaataccatg ctaaacacag tggggggaca tcaagcagcc 1380
atgcaaatgt taaaagagac catcaatgag gaagctgcag aatgggatag attgcatcca 1440
gtgcatgcag ggcctattgc accaggccag atgagagaac caaggggaag tgacatagca 1500
ggaactacta gtacccttca ggaacaaata ggatggatga cacataatcc acctatccca 1560
gtaggagaaa tctataaaag atggataatc ctgggattaa ataaaatagt aagaatgtat 1620
agccctacca gcattctgga cataagacaa ggaccaaagg aaccctttag agactatgta 1680
gaccgattct ataaaactct aagagccgag caagcttcac aagaggtaaa aaattggatg 1740
acagaaacct tgttggtcca aaatgcgaac ccagattgta agactatttt aaaagcattg 1800
ggaccaggag cgacactaga agaaatgatg acagcatgtc agggagtggg gggacccggc 1860
cataaagcaa gagttttggc tgaagcaatg agccaagtaa caaatccagc taccataatg 1920
atacagaaag gcaattttag gaaccaaaga aagactgtta agtgtttcaa ttgtggcaaa 1980

gaagggcaca tagccaaaaa ttgcagggcc cctaggaaaa agggctgttg gaaatgtgga 2040
aaggaaggac accaaatgaa agattgtact gagagacagg ctaatttttt agggaagatc 2100
tggccttccc acaagggaag gccagggaat tttcttcaga gcagaccaga gccaacagcc 2160
ccaccagaag agagcttcag gtttggggaa gagacaacaa ctccctctca gaagcaggag 2220
ccgatagaca aggaactgta tcctttagct tccctcagat cactctttgg cagcgacccc 2280
tcgtcacaat aaagataggg gggcaattaa aggaagctct attagataca ggagcagatg 2340
atacagtatt agaagaaatg aatttgccag gaagatggaa accaaaaatg atagggggaa 2400
ttggaggttt tatcaaagta agacagtatg atcagatact catagaaatc tgcggacata 2460
aagctatagg tacagtatta gtaggaccta cacctgtcaa cataattgga agaaatctgt 2520
tgactcagat tggctgcact ttaaattttc ccattagtcc tattgagact gtaccagtaa 2580
aattaaagcc aggaatggat ggcccaaaag ttaaacaatg gccattgaca gaagaaaaaa 2640
taaaagcatt agtagaaatt tgtacagaaa tggaaaagga aggaaaaatt tcaaaaattg 2700
ggcctgaaaa tccatacaat actccagtat ttgccataaa gaaaaaagac agtactaaat 2760
ggagaaaatt agtagatttc agagaactta ataagagaac tcaagatttc tgggaagttc 2820
aattaggaat accacatcct gcagggttaa aacagaaaaa atcagtaaca gtactggatg 2880
tgggcgatgc atatttttca gttcccttag ataaagactt caggaagtat actgcattta 2940
ccatacctag tataaacaat gagacaccag ggattagata tcagtacaat gtgcttccac 3000
agggatggaa aggatcacca gcaatattcc agtgtagcat gacaaaaatc ttagagcctt 3060
ttagaaaaca aaatccagac atagtcatct atcaatacat ggatgatttg tatgtaggat 3120
ctgacttaga aatagggcag catagaacaa aaatagagga actgagacaa catctgttga 3180
ggtggggatt taccacacca gacaaaaaac atcagaaaga acctccattc ctttggatgg 3240
gttatgaact ccatcctgat aaatggacag tacagcctat agtgctgcca gaaaaggaca 3300
gctggactgt caatgacata cagaaattag tgggaaaatt gaattgggca agtcagattt 3360
atgcagggat taaagtaagg caattatgta aacttcttag gggaaccaaa gcactaacag 3420
aagtagtacc actaacagaa gaagcagagc tagaactggc agaaaacagg gagattctaa 3480
aagaaccggt acatggagtg tattatgacc catcaaaaga cttaatagca gaaatacaga 3540
agcaggggca aggccaatgg acatatcaaa tttatcaaga gccatttaaa aatctgaaaa 3600
caggaaagta tgcaagaatg aagggtgccc acactaatga tgtgaaacaa ttaacagagg 3660
cagtacaaaa aatagccaca gaaagcatag taatatgggg aaagactcct aaatttaaat 3720
tacccataca aaaggaaaca tgggaagcat ggtggacaga gtattggcaa gccacctgga 3780
ttcctgagtg ggagtttgtc aatacccctc ccttagtgaa gttatggtac cagttagaga 3840

aagaacccat aataggagca gaaactttct atgtagatgg ggcagccaat agggaaacta 3900
aattaggaaa agcaggatat gtaactgaca gaggaagaca aaaagttgtc cccctaacgg 3960
acacaacaaa tcagaagact gagttacaag caattcatct agctttgcag gattcgggat 4020
tagaagtaaa catagtgaca gactcacaat atgcattggg aatcattcaa gcacaaccag 4080
ataagagtga atcagagtta gtcagtcaaa taatagagca gttaataaaa aaggaaaaag 4140
tctacctggc atgggtacca gcacacaaag gaattggagg aaatgaacaa gtagataaat 4200
tggtcagtgc tggaatcagg aaagtactat ttttagatgg aatagataag gcccaagaag 4260
aacatgagaa atatcacagt aattggagag caatggctag tgattttaac ctaccacctg 4320
tagtagcaaa agaaatagta gccagctgtg ataaatgtca gctaaaaggg gaagccatgc 4380
atggacaagt agactgtagc ccaggaatat ggcagctaga ttgtacacat ttagaaggaa 4440
aagttatctt ggtagcagtt catgtagcca gtggatatat agaagcagaa gtaattccag 4500
cagagacagg gcaagaaaca gcatacttcc tcttaaaatt agcaggaaga tggccagtaa 4560
aaacagtaca tacagacaat ggcagcaatt tcaccagtac tacagttaag gccgcctgtt 4620
ggtgggcggg gatcaagcag gaatttggca ttccctacaa tccccaaagt caaggagtaa 4680
tagaatctat gaataaagaa ttaaagaaaa ttataggaca ggtaagagat caggctgaac 4740
atcttaagac agcagtacaa atggcagtat tcatccacaa ttttaaaaga aaagggggga 4800
ttggggggta cagtgcaggg gaaagaatag tagacataat agcaacagac atacaaacta 4860
aagaattaca aaaacaaatt acaaaaattc aaaattttcg ggtttattac agggacagca 4920
gagatccagt ttggaaagga ccagcaaagc tcctctggaa aggtgaaggg gcagtagtaa 4980
tacaagataa tagtgacata aaagtagtgc caagaagaaa agcaaagatc atcagggatt 5040
atggaaaaca gatggcaggt gatgattgtg tggcaagtag acaggatgag gattaacaca 5100
tggaaaagat tagtaaaaca ccatatgtat atttcaagga aagctaagga ctggttttat 5160
agacatcact atgaaagtac taatccaaaa ataagttcag aagtacacat cccactaggg 5220
gatgctaaat tagtaataac aacatattgg ggtctgcata caggagaaag agactggcat 5280
ttgggtcagg gagtctccat agaatggagg aaaaagagat atagcacaca agtagaccct 5340
gacctagcag accaactaat tcatctgcac tattttgatt gtttttcaga atctgctata 5400
agaaatacca tattaggacg tatagttagt cctaggtgtg aatatcaagc aggacataac 5460
aaggtaggat ctctacagta cttggcacta gcagcattaa taaaaccaaa acagataaag 5520
ccacctttgc ctagtgttag gaaactgaca gaggacagat ggaacaagcc ccagaagacc 5580
aagggccaca gagggagcca tacaatgaat ggacactaga gcttttagag gaacttaaga 5640
gtgaagctgt tagacatttt cctaggatat ggctccataa cttaggacaa catatctatg 5700
aaacttacgg ggatacttgg gcaggagtgg aagccataat aagaattctg caacaactgc 5760

tgtttatcca tttcagaatt gggtgtcgac atagcagaat aggcgttact cgacagagga 5820

gagcaagaaa tggagccagt agatcctaga ctagagccct ggaagcatcc aggaagtcag 5880

cctaaaactg cttgtaccaa ttgctattgt aaaaagtgtt gctttcattg ccaagtttgt 5940

ttcatgacaa aagccttagg catctcctat ggcaggaaga agcggagaca gcgacgaaga 6000

gctcatcaga acagtcagac tcatcaagct tctctatcaa agcagtaagt agtacatgta 6060

atgcaaccta taatagtagc aatagtagca ttagtagtag caataataat agcaatagtt 6120

gtgtggtcca tagtaatcat agaatatagg aaaatattaa gacaaagaaa aatagacagg 6180

ttaattgata gactaataga aagagcagaa gacagtggca atgagagtga aggagaagta 6240

tcagcacttg tggagatggg ggtggaaatg gggcaccatg ctccttggga tattgatgat 6300

ctgtagtgct acagaaaaat tgtgggtcac agtctattat ggggatcttc agacctggag 6360

gaggcgatat gagggacaat tggagaagtg aattatataa atataaagta gtaaaaattg 6420

aaccattagg agtagcaccc accaaggcaa agagaagagt ggtgcagaga gaaaaaagag 6480

cagtgggaat aggagctttg ttccttgggt tcttgggagc agcaggaagc actatgggcg 6540

cagcgtcaat gacgctgacg gtacaggcca gacaattatt gtctgatata gtgcagcagc 6600

agaacaattt gctgagggct attgaggcgc aacagcatct gttgcaactc acagtctggg 6660

gcatcaaaca gctccaggca agaatcctgg ctgtggaaag atacctaaag gatcaacagc 6720

tcctggggat ttggggttgc tctggaaaac tcatttgcac cactgctgtg ccttggaatg 6780

ctagttggag taataaatct ctggaacaga tttggaataa catgacctgg atggagtggg 6840

acagagaaat taacaattac acaagcttaa tacactcctt aattgaagaa tcgcaaaacc 6900

agcaagaaaa gaatgaacaa gaattattgg aattagataa atgggcaagt ttgtggaatt 6960

ggtttaacat aacaaattgg ctgtggtata taaaattatt cataatgata gtaggaggct 7020

tggtaggttt aagaatagtt tttgctgtac tttctatagt gaatagagtt aggcagggat 7080

attcaccatt atcgtttcag acccacctcc caatcccgag gggacccgac aggcccgaag 7140

gaatagaaga agaaggtgga gagagagaca gagacagatc cattcgatta gtgaacggat 7200

ccttagcact tatctgggac gatctgcgga gcctgtgcct cttcagctac caccgcttga 7260

gagacttact cttgattgta acgaggattg tggaacttct gggacgcagg gggtgggaag 7320

ccctcaaata ttggtggaat ctcctacagt attggagtca ggaactaaag aatagtgctg 7380

ttatcgaatt cctgcagccc gggggatccg agctcggtac caagcttaag tttaaacgct 7440

agcatggctt cgtacccccg ccatcaacac gcgtctgcgt tcgaccaggc tgcgcgttct 7500

cgcggccata gcaaccgacg tacggcgttg cgccctcgcc ggcagcaaga agccacggaa 7560

gtccgcccgg agcagaaaat gcccacgcta ctgcgggttt atatagacgg tccccacggg 7620

atggggaaaa ccaccaccac gcaactgctg gtggccctgg gttcgcgcga cgataacttg 7680
ctcaatgcca cagccatagc agtagctgag gggacagata gggttataga agtattacaa 7740
gcagcttata gagctattcg ccacatacct agaagaataa gacagggctt ggaaaggatt 7800
ttgctataag atgggtggca agtggtcaaa aagtagtgtg attggatggc ctgctgtaag 7860
ggaaagaatg agacgagctg agccagcagc agatggggtg ggagcagtat ctcgagacct 7920
agaaaaacat ggagcaatca caagtagcaa tacagcagct aacaatgctg cttgtgcctg 7980
gctagaagca caagaggagg aagaggtggg ttttccagtc acacctcagg tacctttaag 8040
accaatgact tacaaggcag ctgtagatct tagccacttt ttaaaagaaa aggggggact 8100
ggaagggcta attcactccc aaagaagaca agatatcctt gatctgtgga tctaccacac 8160
acaaggctac ttccctgatt ggcagaacta cacaccaggg ccaggggtca gatatccact 8220
gacctttgga tggtgctaca agctagtacc agttgagcca gataaggtag aagaggccaa 8280
taaaggagag aacaccagct tgttacaccc tgtgagcctg catggaatgg atgaccctga 8340
gagagaagtg ttagagtgga ggtttgacag ccgcctagca tttcatcacg tggcccgaga 8400
gctgcatccg gagtacttca agaactgctg acatcgagct tgctacaagg gactttccgc 8460
tggggacttt ccagggaggc gtggcctggg cgggactggg gagtggcgag ccctcagatg 8520
ctgcatataa gcagctgctt tttgcctgta ctgggtctct ctggttagac cagatctgag 8580
cctgggagct ctctggctaa ctagggaacc cactgcttaa gcctcaataa agcttgcctt 8640
gagtgcttca agtagtgtgt gcccgtctgt tgtgtgactc tggtaactag agatccctca 8700
gacccttttta gtcagtgtgg aaaatctcta gca 8733

<210> 6
<211> 1212
<212> DNA
<213> Homo sapiens

<220>
<223> PTEN CDS

<400> 6

atgacagcca tcatcaaaga gatcgttagc agaaacaaaa ggagatatca agaggatgga 60

ttcgacttag acttgaccta tatttatcca aacattattg ctatgggatt tcctgcagaa 120

agacttgaag gcgtatacag gaacaatatt gatgatgtag taaggttttt ggattcaaag 180

cataaaaacc attacaagat atacaatctt tgtgctgaaa gacattatga caccgccaaa 240

tttaattgca gagttgcaca atatcctttt gaagaccata acccaccaca gctagaactt 300

atcaaaccct tttgtgaaga tcttgaccaa tggctaagtg aagatgacaa tcatgttgca 360

gcaattcact gtaaagctgg aaagggacga actggtgtaa tgatatgtgc atatttatta 420

catcggggca atttttaaa ggcacaagag gccctagatt tctatgggga agtaaggacc 480

agagacaaaa agggagtaac tattcccagt cagaggcgct atgtgtatta ttatagctac 540

ctgttaaaga atcatctgga ttatagacca gtggcactgt tgtttcacaa gatgatgttt 600

gaaactattc caatgttcag tggcggaact tgcaatcctc agtttgtggt ctgccagcta 660

aaggtgaaga tatattcctc caattcagga cccacacgac gggaagacaa gttcatgtac 720

tttgagttcc ctcagccgtt acctgtgtgt ggtgatatca aagtagagtt cttccacaaa 780

cagaacaaga tgctaaaaaa ggacaaaatg tttcactttt gggtaaatac attcttcata 840

ccaggaccag aggaaacctc agaaaaagta gaaaatggaa gtctatgtga tcaagaaatc 900

gatagcattt gcagtataga gcgtgcagat aatgacaagg aatatctagt acttacttta 960

acaaaaaatg atcttgacaa agcaaataaa gacaaagcca accgatactt ttctccaaat 1020

tttaaggtga agctgtactt cacaaaaaca gtagaggagc cgtcaaatcc agaggctagc 1080

agttcaactt ctgtaacacc agatgttagt gacaatgaac ctgatcatta tagatattct 1140

gacaccactg actctgatcc agagaatgaa ccttttgatg aagatcagca tacacaaatt 1200

acaaaagtct ga 1212

<210> 7
<211> 1683
<212> DNA
<213> Homo sapiens

<220>
<223> CDC6 CDS

<400> 7

```
atgcctcaaa cccgatccca ggcacaggct acaatcagtt ttccaaaaag gaagctgtct     60
cgggcattga acaaagctaa aaactccagt gatgccaaac tagaaccaac aaatgtccaa    120
accgtaacct gttctcctcg tgtaaaagcc ctgcctctca gccccaggaa acgtctgggc    180
gatgacaacc tatgcaacac tccccattta cctccttgtt ctccaccaaa gcaaggcaag    240
aaagagaatg gtccccctca ctcacataca cttaagggac gaagattggt atttgacaat    300
cagctgacaa ttaagtctcc tagcaaaaga gaactagcca aagttcacca aaacaaaata    360
ctttcttcag ttagaaaaag tcaagagatc acaacaaatt ctgagcagag atgtccactg    420
aagaaagaat ctgcatgtgt gagactattc aagcaagaag gcacttgcta ccagcaagca    480
aagctggtcc tgaacacagc tgtcccagat cggctgcctg ccagggaaag ggagatggat    540
gtcatcagga atttcttgag ggaacacatc tgtgggaaaa aagctggaag cctttacctt    600
tctggtgctc ctggaactgg aaaaactgcc tgcttaagcc ggattctgca agacctcaag    660
aaggaactga aaggctttaa aactatcatg ctgaattgca tgtccttgag gactgcccag    720
gctgtattcc cagctattgc tcaggagatt tgtcaggaag aggtatccag gccagctggg    780
aaggacatga tgaggaaatt ggaaaaacat atgactgcag agaagggccc catgattgtg    840
ttggtattgg acgagatgga tcaactggac agcaaaggcc aggatgtatt gtacacgcta    900
tttgaatggc catggctaag caattctcac ttggtgctga ttggtattgc taataccctg    960
gatctcacag atagaattct acctaggctt caagctagag aaaaatgtaa gccacagctg   1020
ttgaacttcc caccttatac cagaaatcag atagtcacta ttttgcaaga tcgacttaat   1080
caggtatcta gagatcaggt tctggacaat gctgcagttc aattctgtgc ccgcaaagtc   1140
tctgctgttt caggagatgt tcgcaaagca ctggatgttt gcaggagagc tattgaaatt   1200
gtagagtcag atgtcaaaag ccagactatt ctcaaaccac tgtctgaatg taaatcacct   1260
tctgagcctc tgattcccaa gagggttggt cttattcaca tatcccaagt catctcagaa   1320
gttgatggta acaggatgac cttgagccaa gaaggagcac aagattcctt ccctcttcag   1380
cagaagatct tggtttgctc tttgatgctc ttgatcaggc agttgaaaat caaagaggtc   1440
actctgggga agttatatga agcctacagt aaagtctgtc gcaaacagca ggtggcggct   1500
gtggaccagt cagagtgttt gtcactttca gggctcttgg aagccagggg cattttagga   1560
ttaaagagaa acaaggaaac ccgtttgaca aaggtgtttt tcaagattga agagaaagaa   1620
atagaacatg ctctgaaaga taaagcttta attggaaata tcttagctac tggattgcct   1680
taa                                                                 1683
```

<210> 8
<211> 64
<212> DNA

<213> Artificial sequence

<220>
<223> Oligonucleotide Cdc6-5-A

<400> 8

**gatccccagg cacttgctac cagcaattca agagattgct ggtagcaagt gccttttttg 60**

**gaaa 64**

<210> 9
<211> 64
<212> DNA
<213> Artificial sequence

<220>
<223> Oligonucleotide Cdc6-3-A

<400> 9

**agcttttcca aaaaaggcac ttgctaccag caatctcttg aattgctggt agcaagtgcc 60**

**tggg 64**

<210> 10
<211> 60
<212> DNA
<213> Artificial sequence

<220>
<223> DNA sequence which encodes CDC6 shRNA

<400> 10
cccaggcact tgctaccagc aattcaagag attgctggta gcaagtgcct tttttggaaa 60

<210> 11
<211> 21
<212> DNA
<213> Artificial sequence

<220>
<223> Primer B-NLR8950

<220>
<221> misc_feature
<222> (1)..(1)
<223> Biotynylated

<400> 11
gtgcctggct agaagcacaa g 21

<210> 12
<211> 32
<212> DNA
<213> Artificial sequence

<220>
<223> Oligonucleotide HD-A

<400> 12
cacgcgtcgc atcatatctc caggtgtgac ag 32

<210> 13
<211> 37
<212> DNA
<213> Artificial sequence

<220>
<223> Oligonucleotide HD-S

<220>
<221> misc_feature
<222> (36)..(37)
<223> n is a, c, g, or t

<400> 13
cctctgtcac acctggagat atgatgcgac gcgtgnn 37

<210> 14
<211> 19
<212> DNA
<213> Artificial sequence

<220>
<223> Forward primer BHU5-S2

<400> 14
gagtgctcaa agtagtggt 19

<210> 15
<211> 22
<212> DNA
<213> Artificial sequence

<220>
<223> Reverse primer HDA/SBOT

<400> 15
ctgtcacacc tggagatatg at 22

<210> 16
<211> 24
<212> DNA
<213> Artificial sequence

<220>
<223> Primer 5’ LTRU5

<400> 16
tctggctaac tagggaaccc actg 24

<210> 17
<211> 24
<212> DNA

<213> Artificial sequence

<220>
<223> Primer 3’ NL5850

<400> 17
gctatgtcga cacccaattc tgaa 24

<210> 18
<211> 23
<212> DNA
<213> Artificial sequence

<220>
<223> Primer 3’ NL8960

<400> 18
tgtgcttcta gccaggcaca agc 23

<210> 19
<211> 19
<212> RNA
<213> Artificial sequence

<220>
<223> The nucleotide sequence of antisense sequence of CDC6 shRNA

<400> 19
uugcugguag caagugccu 19

<210> 20
<211> 9
<212> RNA
<213> Artificial sequence

<220>
<223> The nucleotide sequence of linker of CDC6 shRNA

<400> 20
uucaagaga 9

<210> 21
<211> 312
<212> DNA
<213> Simian virus 40

<400> 21

```
agctttgcaa agatggataa agttttaaac agagaggaat ctttgcagct aatggacctt      60
ctaggtcttg aaaggagtgc ctgggggaat attcctctga tgagaaaggc atatttaaaa     120
aaatgcaagg agtttcatcc tgataaagga ggagatgaag aaaaaatgaa gaaaatgaat     180
actctgtaca agaaaatgga agatggagta aaatatgctc atcaacctga ctttggaggc     240
ttctgggatg caactgaggt atttgcttct tccttaaatc ctggtgttga tgcaatgtac     300
tgcaaacaat gg                                                          312
```

<210> 22
<211> 403
<212> PRT
<213> Homo sapiens

<400> 22

```
Met Thr Ala Ile Ile Lys Glu Ile Val Ser Arg Asn Lys Arg Arg Tyr
1               5                   10                  15

Gln Glu Asp Gly Phe Asp Leu Asp Leu Thr Tyr Ile Tyr Pro Asn Ile
            20                  25                  30

Ile Ala Met Gly Phe Pro Ala Glu Arg Leu Glu Gly Val Tyr Arg Asn
        35                  40                  45

Asn Ile Asp Asp Val Val Arg Phe Leu Asp Ser Lys His Lys Asn His
    50                  55                  60

Tyr Lys Ile Tyr Asn Leu Cys Ala Glu Arg His Tyr Asp Thr Ala Lys
65                  70                  75                  80

Phe Asn Cys Arg Val Ala Gln Tyr Pro Phe Glu Asp His Asn Pro Pro
                85                  90                  95
```

```
Gln Leu Glu Leu Ile Lys Pro Phe Cys Glu Asp Leu Asp Gln Trp Leu
            100                 105                 110

Ser Glu Asp Asp Asn His Val Ala Ala Ile His Cys Lys Ala Gly Lys
        115                 120                 125

Gly Arg Thr Gly Val Met Ile Cys Ala Tyr Leu Leu His Arg Gly Lys
    130                 135                 140

Phe Leu Lys Ala Gln Glu Ala Leu Asp Phe Tyr Gly Glu Val Arg Thr
145                 150                 155                 160

Arg Asp Lys Lys Gly Val Thr Ile Pro Ser Gln Arg Arg Tyr Val Tyr
                165                 170                 175

Tyr Tyr Ser Tyr Leu Leu Lys Asn His Leu Asp Tyr Arg Pro Val Ala
            180                 185                 190

Leu Leu Phe His Lys Met Met Phe Glu Thr Ile Pro Met Phe Ser Gly
        195                 200                 205

Gly Thr Cys Asn Pro Gln Phe Val Val Cys Gln Leu Lys Val Lys Ile
    210                 215                 220

Tyr Ser Ser Asn Ser Gly Pro Thr Arg Arg Glu Asp Lys Phe Met Tyr
225                 230                 235                 240

Phe Glu Phe Pro Gln Pro Leu Pro Val Cys Gly Asp Ile Lys Val Glu
                245                 250                 255

Phe Phe His Lys Gln Asn Lys Met Leu Lys Lys Asp Lys Met Phe His
            260                 265                 270

Phe Trp Val Asn Thr Phe Phe Ile Pro Gly Pro Glu Glu Thr Ser Glu
        275                 280                 285

Lys Val Glu Asn Gly Ser Leu Cys Asp Gln Glu Ile Asp Ser Ile Cys
    290                 295                 300

Ser Ile Glu Arg Ala Asp Asn Asp Lys Glu Tyr Leu Val Leu Thr Leu
305                 310                 315                 320

Thr Lys Asn Asp Leu Asp Lys Ala Asn Lys Asp Lys Ala Asn Arg Tyr
                325                 330                 335

Phe Ser Pro Asn Phe Lys Val Lys Leu Tyr Phe Thr Lys Thr Val Glu
            340                 345                 350
```

```
Glu Pro Ser Asn Pro Glu Ala Ser Ser Ser Thr Ser Val Thr Pro Asp
        355                 360                 365

Val Ser Asp Asn Glu Pro Asp His Tyr Arg Tyr Ser Asp Thr Thr Asp
    370                 375                 380

Ser Asp Pro Glu Asn Glu Pro Phe Asp Glu Asp Gln His Thr Gln Ile
385                 390                 395                 400

Thr Lys Val
```

<210> 23
<211> 471
<212> DNA
<213> Homo sapiens

<220>
<221> CDS
<222> (1)..(471)

<400> 23

```
atg gag ccg gcg gcg ggg agc agc atg gag cct tcg gct gac tgg ctg        48
Met Glu Pro Ala Ala Gly Ser Ser Met Glu Pro Ser Ala Asp Trp Leu
1               5                   10                  15

gcc acg gcc gcg gcc cgg ggt cgg gta gag gag gtg cgg gcg ctg ctg        96
Ala Thr Ala Ala Ala Arg Gly Arg Val Glu Glu Val Arg Ala Leu Leu
            20                  25                  30

gag gcg ggg gcg ctg ccc aac gca ccg aat agt tac ggt cgg agg ccg       144
Glu Ala Gly Ala Leu Pro Asn Ala Pro Asn Ser Tyr Gly Arg Arg Pro
        35                  40                  45

atc cag gtc atg atg atg ggc agc gcc cga gtg gcg gag ctg ctg ctg       192
Ile Gln Val Met Met Met Gly Ser Ala Arg Val Ala Glu Leu Leu Leu
    50                  55                  60

ctc cac ggc gcg gag ccc aac tgc gcc gac ccc gcc act ctc acc cga       240
Leu His Gly Ala Glu Pro Asn Cys Ala Asp Pro Ala Thr Leu Thr Arg
65                  70                  75                  80

ccc gtg cac gac gct gcc cgg gag ggc ttc ctg gac acg ctg gtg gtg       288
Pro Val His Asp Ala Ala Arg Glu Gly Phe Leu Asp Thr Leu Val Val
                85                  90                  95

ctg cac cgg gcc ggg gcg cgg ctg gac gtg cgc gat gcc tgg ggc cgt       336
Leu His Arg Ala Gly Ala Arg Leu Asp Val Arg Asp Ala Trp Gly Arg
            100                 105                 110

ctg ccc gtg gac ctg gct gag gag ctg ggc cat cgc gat gtc gca cgg       384
Leu Pro Val Asp Leu Ala Glu Glu Leu Gly His Arg Asp Val Ala Arg
        115                 120                 125

tac ctg cgc gcg gct gcg ggg ggc acc aga ggc agt aac cat gcc cgc       432
Tyr Leu Arg Ala Ala Ala Gly Gly Thr Arg Gly Ser Asn His Ala Arg
```

```
    130                 135                 140

ata gat gcc gcg gaa ggt ccc tca gac atc ccc gat tga                    471
Ile Asp Ala Ala Glu Gly Pro Ser Asp Ile Pro Asp
145                 150                 155
```

<210> 24
<211> 156
<212> PRT
<213> Homo sapiens

<400> 24

```
Met Glu Pro Ala Ala Gly Ser Ser Met Glu Pro Ser Ala Asp Trp Leu
1               5                   10                  15

Ala Thr Ala Ala Ala Arg Gly Arg Val Glu Glu Val Arg Ala Leu Leu
            20                  25                  30

Glu Ala Gly Ala Leu Pro Asn Ala Pro Asn Ser Tyr Gly Arg Arg Pro
        35                  40                  45

Ile Gln Val Met Met Met Gly Ser Ala Arg Val Ala Glu Leu Leu Leu
    50                  55                  60

Leu His Gly Ala Glu Pro Asn Cys Ala Asp Pro Ala Thr Leu Thr Arg
65                  70                  75                  80

Pro Val His Asp Ala Ala Arg Glu Gly Phe Leu Asp Thr Leu Val Val
                85                  90                  95

Leu His Arg Ala Gly Ala Arg Leu Asp Val Arg Asp Ala Trp Gly Arg
            100                 105                 110

Leu Pro Val Asp Leu Ala Glu Glu Leu Gly His Arg Asp Val Ala Arg
        115                 120                 125

Tyr Leu Arg Ala Ala Ala Gly Gly Thr Arg Gly Ser Asn His Ala Arg
    130                 135                 140

Ile Asp Ala Ala Glu Gly Pro Ser Asp Ile Pro Asp
145                 150                 155
```

<210> 25
<211> 831
<212> DNA
<213> Artificial

<220>
<223> pCMV p16INK4a

<400> 25

```
agtacggccg ccagtgtgct ggaattcacc accatggagc cttcggctga ctggctggcc      60
acggccgcgg cccggggtcg ggtagaggag gtgcgggcgc tgctggaggc gggggcgctg     120
cccaacgcac cgaatagtta cggtcggagg ccgatccagg tcatgatgat gggcagcgcc     180
cgagtggcgg agctgctgct gctccacggc gcggagccca actgcgccga ccccgccact     240
ctcacccgac ccgtgcacga cgctgcccgg gagggcttcc tggacacgct ggtggtgctg     300
caccgggccg gggcgcggct ggacgtgcgc gatgcctggg gccgtctgcc cgtggacctg     360
gctgaggagc tgggccatcg cgatgtcgca cggtacctgc gcgcggctgc gggggggcacc    420
agaggcagta accatgcccg catagatgcc gcggaaggtc cctcagatat ccccgattga     480
ctcgagcatg catctagagg gccctattct atagtgtcac ctaaatgcta gagctcgctg     540
atcagcctcg actgtgcctt ctagttgcca gccatctgtt gtttgcccct cccccgtgcc     600
ttccttgacc ctggaaggtg ccactcccac tgtcctttcc taataaaatg aggaaattgc     660
atcgcattgt ctgagtaggt gtcattctat tctggggggt ggggtggggc aggacagcaa     720
gggggaggat tgggaagaca atagcaagca tgctggggat gcggtgggct ctatggcttc     780
tgaggcggaa agaaccagct ggggctctag gggtatcccc cacgcgccct g              831
```

<210> 26
<211> 1182
<212> DNA
<213> Homo sapiens

<220>
<221> CDS
<222> (1)..(1182)

<400> 26

```
atg gag gag ccg cag tca gat cct agc gtc gag ccc cct ctg agt cag        48
Met Glu Glu Pro Gln Ser Asp Pro Ser Val Glu Pro Pro Leu Ser Gln
1               5                   10                  15

gaa aca ttt tca gac cta tgg aaa cta ctt cct gaa aac aac gtt ctg        96
Glu Thr Phe Ser Asp Leu Trp Lys Leu Leu Pro Glu Asn Asn Val Leu
            20                  25                  30

tcc ccc ttg ccg tcc caa gca atg gat gat ttg atg ctg tcc ccg gac       144
Ser Pro Leu Pro Ser Gln Ala Met Asp Asp Leu Met Leu Ser Pro Asp
        35                  40                  45

gat att gaa caa tgg ttc act gaa gac cca ggt cca gat gaa gct ccc       192
Asp Ile Glu Gln Trp Phe Thr Glu Asp Pro Gly Pro Asp Glu Ala Pro
    50                  55                  60

aga atg cca gag gct gct ccc cgc gtg gcc cct gca cca gca gct cct       240
Arg Met Pro Glu Ala Ala Pro Arg Val Ala Pro Ala Pro Ala Ala Pro
65                  70                  75                  80

aca ccg gcg gcc cct gca cca gcc ccc tcc tgg ccc ctg tca tct tct       288
Thr Pro Ala Ala Pro Ala Pro Ala Pro Ser Trp Pro Leu Ser Ser Ser
                85                  90                  95
```

```
gtc cct tcc cag aaa acc tac cag ggc agc tac ggt ttc cgt ctg ggc       336
Val Pro Ser Gln Lys Thr Tyr Gln Gly Ser Tyr Gly Phe Arg Leu Gly
            100                 105                 110

ttc ttg cat tct ggg aca gcc aag tct gtg act tgc acg tac tcc cct       384
Phe Leu His Ser Gly Thr Ala Lys Ser Val Thr Cys Thr Tyr Ser Pro
        115                 120                 125

gcc ctc aac aag atg ttt tgc caa ctg gcc aag acc tgc cct gtg cag       432
Ala Leu Asn Lys Met Phe Cys Gln Leu Ala Lys Thr Cys Pro Val Gln
    130                 135                 140

ctg tgg gtt gat tcc aca ccc ccg ccc ggc acc cgc gtc cgc gcc atg       480
Leu Trp Val Asp Ser Thr Pro Pro Pro Gly Thr Arg Val Arg Ala Met
145                 150                 155                 160

gcc atc tac aag cag tca cag cac atg acg gag gtt gtg agg cgc tgc       528
Ala Ile Tyr Lys Gln Ser Gln His Met Thr Glu Val Val Arg Arg Cys
                165                 170                 175

ccc cac cat gag cgc tgc tca gat agc gat ggt ctg gcc cct cct cag       576
Pro His His Glu Arg Cys Ser Asp Ser Asp Gly Leu Ala Pro Pro Gln
            180                 185                 190

cat ctt atc cga gtg gaa gga aat ttg cgt gtg gag tat ttg gat gac       624
His Leu Ile Arg Val Glu Gly Asn Leu Arg Val Glu Tyr Leu Asp Asp
        195                 200                 205

aga aac act ttt cga cat agt gtg gtg gtg ccc tat gag ccg cct gag       672
Arg Asn Thr Phe Arg His Ser Val Val Val Pro Tyr Glu Pro Pro Glu
    210                 215                 220

gtt ggc tct gac tgt acc acc atc cac tac aac tac atg tgt aac agt       720
Val Gly Ser Asp Cys Thr Thr Ile His Tyr Asn Tyr Met Cys Asn Ser
225                 230                 235                 240

tcc tgc atg ggc ggc atg aac cgg agg ccc atc ctc acc atc atc aca       768
Ser Cys Met Gly Gly Met Asn Arg Arg Pro Ile Leu Thr Ile Ile Thr
                245                 250                 255

ctg gaa gac tcc agt ggt aat cta ctg gga cgg aac agc ttt gag gtg       816
Leu Glu Asp Ser Ser Gly Asn Leu Leu Gly Arg Asn Ser Phe Glu Val
            260                 265                 270

cgt gtt tgt gcc tgt gct ggg aga gac cgg cgc aca gag gaa gag aat       864
Arg Val Cys Ala Cys Ala Gly Arg Asp Arg Arg Thr Glu Glu Glu Asn
        275                 280                 285

ctc cgc aag aaa ggg gag cct cac cac gag ctg ccc cca ggg agc act       912
Leu Arg Lys Lys Gly Glu Pro His His Glu Leu Pro Pro Gly Ser Thr
    290                 295                 300

aag cga gca ctg ccc aac aac acc agc tcc tct ccc cag cca aag aag       960
Lys Arg Ala Leu Pro Asn Asn Thr Ser Ser Ser Pro Gln Pro Lys Lys
305                 310                 315                 320

aaa cca ctg gat gga gaa tat ttc acc ctt cag atc cgt ggg cgt gag      1008
Lys Pro Leu Asp Gly Glu Tyr Phe Thr Leu Gln Ile Arg Gly Arg Glu
                325                 330                 335

cgc ttc gag atg ttc cga gag ctg aat gag gcc ttg gaa ctc aag gat      1056
Arg Phe Glu Met Phe Arg Glu Leu Asn Glu Ala Leu Glu Leu Lys Asp
```

```
            340                 345                 350

gcc cag gct ggg aag gag cca ggg ggg agc agg gct cac tcc agc cac      1104
Ala Gln Ala Gly Lys Glu Pro Gly Gly Ser Arg Ala His Ser Ser His


ctg aag tcc aaa aag ggt cag tct acc tcc cgc cat aaa aaa ctc atg      1152
Leu Lys Ser Lys Lys Gly Gln Ser Thr Ser Arg His Lys Lys Leu Met
    370                 375                 380

ttc aag aca gaa ggg cct gac tca gac tga                              1182
Phe Lys Thr Glu Gly Pro Asp Ser Asp
385                 390
```

<210> 27
<211> 393
<212> PRT
<213> Homo sapiens

<400> 27

```
Met Glu Glu Pro Gln Ser Asp Pro Ser Val Glu Pro Pro Leu Ser Gln
1               5                   10                  15

Glu Thr Phe Ser Asp Leu Trp Lys Leu Leu Pro Glu Asn Asn Val Leu
            20                  25                  30

Ser Pro Leu Pro Ser Gln Ala Met Asp Asp Leu Met Leu Ser Pro Asp
        35                  40                  45

Asp Ile Glu Gln Trp Phe Thr Glu Asp Pro Gly Pro Asp Glu Ala Pro
    50                  55                  60

Arg Met Pro Glu Ala Ala Pro Arg Val Ala Pro Ala Pro Ala Ala Pro
65                  70                  75                  80

Thr Pro Ala Ala Pro Ala Pro Ala Pro Ser Trp Pro Leu Ser Ser Ser
                85                  90                  95

Val Pro Ser Gln Lys Thr Tyr Gln Gly Ser Tyr Gly Phe Arg Leu Gly
            100                 105                 110

Phe Leu His Ser Gly Thr Ala Lys Ser Val Thr Cys Thr Tyr Ser Pro
        115                 120                 125

Ala Leu Asn Lys Met Phe Cys Gln Leu Ala Lys Thr Cys Pro Val Gln
    130                 135                 140

Leu Trp Val Asp Ser Thr Pro Pro Pro Gly Thr Arg Val Arg Ala Met
145                 150                 155                 160

Ala Ile Tyr Lys Gln Ser Gln His Met Thr Glu Val Val Arg Arg Cys
```

```
                165                 170                 175

Pro His His Glu Arg Cys Ser Asp Ser Asp Gly Leu Ala Pro Pro Gln
            180                 185                 190

His Leu Ile Arg Val Glu Gly Asn Leu Arg Val Glu Tyr Leu Asp Asp
        195                 200                 205

Arg Asn Thr Phe Arg His Ser Val Val Val Pro Tyr Glu Pro Pro Glu
    210                 215                 220

Val Gly Ser Asp Cys Thr Thr Ile His Tyr Asn Tyr Met Cys Asn Ser
225                 230                 235                 240

Ser Cys Met Gly Gly Met Asn Arg Arg Pro Ile Leu Thr Ile Ile Thr
                245                 250                 255

Leu Glu Asp Ser Ser Gly Asn Leu Leu Gly Arg Asn Ser Phe Glu Val
            260                 265                 270

Arg Val Cys Ala Cys Ala Gly Arg Asp Arg Arg Thr Glu Glu Glu Asn
        275                 280                 285

Leu Arg Lys Lys Gly Glu Pro His His Glu Leu Pro Pro Gly Ser Thr
    290                 295                 300

Lys Arg Ala Leu Pro Asn Asn Thr Ser Ser Ser Pro Gln Pro Lys Lys
305                 310                 315                 320

Lys Pro Leu Asp Gly Glu Tyr Phe Thr Leu Gln Ile Arg Gly Arg Glu
                325                 330                 335

Arg Phe Glu Met Phe Arg Glu Leu Asn Glu Ala Leu Glu Leu Lys Asp
            340                 345                 350

Ala Gln Ala Gly Lys Glu Pro Gly Gly Ser Arg Ala His Ser Ser His
        355                 360                 365

Leu Lys Ser Lys Lys Gly Gln Ser Thr Ser Arg His Lys Lys Leu Met
    370                 375                 380

Phe Lys Thr Glu Gly Pro Asp Ser Asp
385                 390
```